# EUROPEAN PATENT APPLICATION

(11) **EP 4 644 378 A1**
(43) Date of publication of application: **05.11.2025**
(21) Application number: 23912861.4
(22) Date of filing: 26.12.2023
(51) Int. Cl.: C07D 311/96, C07D 409/14, C07D 335/04, C07D 409/12, C07D 405/12, C07D 407/14, C07D 407/12, H10K 85/60

(54) **ORGANIC COMPOUND AND ORGANIC ELECTROLUMINESCENT DIODE USING SAME**

(30) Priority: 26.12.2022 KR 20220185027
(71) Applicant: Solus Advanced Materials Co., Ltd., Iksan-si, Jeollabuk-do 54584 (KR)
(72) Inventor: LEE, Yonghwan, Seongnam-si, Gyeonggi-do 13636 (KR); SIM, Jaeyi, Seongnam-si, Gyeonggi-do 13636 (KR); BAE, Hyungchan, Gyeonggi-do 13636 (KR)
(74) Representative: Office Freylinger
(86) International application number: PCT/KR2023/021550
(87) International publication number: WO 2024/144186

(57) **Abstract**

The present invention relates to a novel organic light-emitting compound and an organic electroluminescent diode using same and, more specifically, to: a compound having excellent thermal stability, electrochemical stability, light-emitting ability, and hole/electron transport ability; and an organic electroluminescent diode which contains the compound in at least one organic layer and thus has improved characteristics such as luminous efficiency, driving voltage, service life, and the like.

## Description

### TECHNICAL FIELD

The present disclosure relates to a novel organic light-emitting compound and an organic electroluminescent device using the same, and more specifically, to a compound having excellent characteristics such as chemical structural stability, thermal stability, electrochemical stability, and hole transport ability, and an organic electroluminescent device having improved characteristics such as luminous efficiency, driving voltage, service life, and the like by including the compound in one or more organic layers.

### BACKGROUND ART

In organic electroluminescent ("EL") devices, upon application of current or voltage across two electrodes, holes are injected from an anode to an organic layer and electrons are injected from a cathode into the organic layer. Injected holes and electrons recombine to form excitons, and light is emitted when these excitons decay to the ground state. In such cases, materials used for the organic layer may be classified, for example, as luminescent (e.g., light emitting) materials, hole injection materials, hole transport materials, electron transport materials and electron injection materials, depending on their function.

Light emitting materials in an organic EL device may be classified into blue-, green- and red-light emitting materials depending on their emission colors. Additionally, yellow and orange light emitting materials may be employed as a light emitting material to achieve more natural color reproduction. In addition, a host/dopant system may also be used in the light emitting material to enhance color purity and luminescence efficiency through energy transfer. Dopant materials may be classified into fluorescent dopants, which use organic materials, and phosphorescent dopants, which use metal complex compounds containing heavy atoms such as iridium (Ir) and platinum (Pt). The phosphorescent materials may theoretically achieve up to four times higher luminescence efficiency than the fluorescent materials, so attention has been directed toward phosphorescent dopants as well as phosphorescent host materials.

To date, NPB, BCP and Alq₃, whose chemical formulas are shown below, have been widely recognized as materials used in the hole injection layer, hole transport layer, hole blocking layer and electron transport layer, and anthracene derivatives have also been reported as fluorescent dopant/host materials for light emitting materials. In particular, Ir-based metal complexes such as FIrpic, Ir(ppy)₃, and Ir(btp)₂(acac) are known as phosphorescent dopant materials for improving efficiency among light emitting materials, and are used as blue, green and red dopant materials. Among phosphorescent host materials, CBP has demonstrated excellent performance to date.

However, despite exhibiting good luminescence properties, conventional materials have shown low glass transition temperatures and poor thermal stability, and are therefore inadequate in terms of service life characteristics for organic EL devices. Accordingly, there is a growing demand for the development of organic layer materials with enhanced performance.

### DETAILED DESCRIPTION OF THE INVENTION

### TECHNICAL OBJECTIVES

The present disclosure is applicable to organic EL devices, and is directed to a novel organic compound that has excellent characteristics such as thermal stability, electrochemical stability, and hole transport ability to be used as a hole transport layer material.

In addition, the present disclosure is further directed to an organic EL device including the novel organic compound, thereby exhibiting low operating voltage, high luminous efficiency, and improved service life.

### TECHNICAL MEANS

To achieve the above objectives, the present disclosure provides a compound represented by the following Chemical Formula 1: 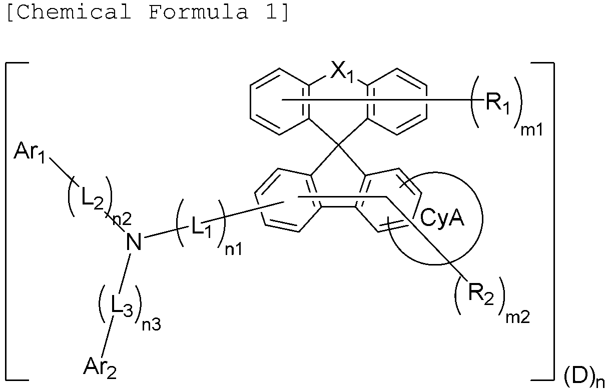 (wherein in Chemical Formula 1,
n≥1,
X₁ is O or S,
a CyA ring is a C₆ to C₃₀ aromatic ring,
each of n1 to n3 is an integer in a range of 0 to 3,
L₁ to L₃ are the same as or different from each other, and each independently is a single bond, or is selected from the group consisting of a C₆ to C₃₀ arylene group and a heteroarylene group having 5 to 30 nuclear atoms,
m1 is an integer in a range of 0 to 8,
m2 is an integer in a range of 0 to 23,
R₁, R₂, Ar₁ and Ar₂ are the same as or different from each other, and each independently is selected from the group consisting of: hydrogen, deuterium (D), halogen group, cyano group, nitro group, amino group, C₁ to C₄₀ alkyl group, C₂ to C₄₀ alkenyl group, C₂ to C₄₀ alkynyl group, C₃ to C₄₀ cycloalkyl group, heterocycloalkyl group having 3 to 40 nuclear atoms, C₆ to C₆₀ aryl group, heteroaryl group having 5 to 60 nuclear atoms, C₁ to C₄₀ alkyloxy group, C₆ to C₆₀ aryloxy group, C₁ to C₄₀ alkylsilyl group, C₆ to C₆₀ arylsilyl group, C₁ to C₄₀ alkylboron group, C₆ to C₆₀ arylboron group, C₆ to C₆₀ arylphosphine group, C₆ to C₆₀ arylphosphine oxide group, C₆ to C₆₀ arylamine group, C₆ to C₆₀ (aryl) (heteroaryl)amine group, and heteroarylamine group having 5 to 60 nuclear atoms, or R₁, R₂, Ar₁ and Ar₂ each independently is fused with an adjacent group to form a fused ring, and
the arylene group and the heteroarylene group of L₁ to L₃, and the alkyl group, the alkenyl group, the alkynyl group, the cycloalkyl group, the heterocycloalkyl group, the aryl group, the heteroaryl group, the alkyloxy group, the aryloxy group, the alkylsilyl group, the arylsilyl group, the alkylboron group, the arylboron group, the arylphosphine group, the arylphosphine oxide group, the arylamine group, the (aryl) (heteroaryl)amine group and the heteroarylamine group of R₁, R₂, Ar₁ and Ar₂ each independently is substituted or unsubstituted with one or more substituents selected from the group consisting of deuterium (D), halogen, cyano group, nitro group, amino group, C₁ to C₄₀ alkyl group, C₂ to C₄₀ alkenyl group, C₂ to C₄₀ alkynyl group, C₃ to C₄₀ cycloalkyl group, heterocycloalkyl group having 3 to 40 nuclear atoms, C₆ to C₆₀ aryl group, heteroaryl group having 5 to 60 nuclear atoms, C₁ to C₄₀ alkyloxy group, C₆ to C₆₀ aryloxy group, C₁ to C₄₀ alkylsilyl group, C₆ to C₆₀ arylsilyl group, C₁ to C₄₀ alkylboron group, C₆ to C₆₀ arylboron group, C₆ to C₆₀ arylphosphine group, C₆ to C₆₀ arylphosphine oxide group, C₆ to C₆₀ arylamine group, C₆ to C₆₀ (aryl) (heteroaryl)amine group, and heteroarylamine group having 5 to 60 nuclear atoms, and when the substituents are plural, the substituents are the same as or different from each other).

Furthermore, the present disclosure provides an organic electroluminescent device including: an anode, a cathode, and one or more organic layers disposed between the anode and the cathode,
wherein at least one of the one or more organic layers includes the compound represented by the above Chemical Formula 1.

In an example, the organic layer including the compound may be a hole transport layer.

### EFFECTS OF THE INVENTION

A compound according to an embodiment of the present disclosure may be effectively applied as an organic layer material in an organic EL device due to its excellent thermal stability, electrochemical stability, and hole transport ability. In particular, when used as a hole transport layer material, the compound according to an embodiment of the present disclosure may enable fabrication of an organic EL device exhibiting superior luminescence performance, low driving voltage, high efficiency, and long service life characteristics compared to conventional materials, and may also contribute to fabrication of a full-color display panel with enhanced performance and service life.

The effects of the present disclosure are not limited to the examples described above, and various other advantages and effects are also encompassed within the scope of the present specification.

### BRIEF DESCRIPTION OF THE INVENTION

FIG. 1 is a cross-sectional view schematically illustrating an organic EL device according to an example of the present disclosure.
FIG. 2 is a cross-sectional view schematically illustrating an organic EL device according to another example of the present disclosure.

### <Reference numeral>

100: anode, 200: cathode,
300: organic layer, 310: hole injection layer,
320: hole transport layer, 3₃₀: electroluminescent layer,
340: electron transport layer, 350: electron injection layer

### MODE FOR IMPLEMENTING THE INVENTION

Hereinafter, the present disclosure will be described in detail.

### <Novel compound>

The present disclosure is directed to a compound comprising: an amine group substituted with an aryl group and/or a heteroaryl group; and a spiro ring connected to the amine group directly or via a linker group (e.g., a phenylene group, etc.), wherein at least one deuterium (D) is essentially included in the molecular structure, and is represented by Chemical Formula 1. The compound of Chemical Formula 1 not only has excellent electrochemical stability, thermal stability, and carrier transport ability (particularly, hole transport ability), but also has excellent chemical structural stability due to deuterium (D) substitution, and thus it may be applied to a hole transport layer to collectively implement the characteristics of the organic EL device, such as low voltage, high efficiency, and long life characteristics of the device.

Specifically, the compound represented by Chemical Formula 1 according to the present disclosure includes at least one deuterium (D) in the molecular structure. In terms of potential energy, deuterium (D) has a higher molecular mass and lower zero-point energy than hydrogen (H). For this reason, dissociation of deuterium is relatively more difficult in terms of reaction. However, since the compound substituted with deuterium has a low zero-point energy, the bond dissociation energy increases, which lowers the reactivity, and thus the stability of the molecule increases (Molecules 2014, 19 Chem. Commun., 2014, 50, 14870-14872 J. Org. Chem. 2004, 69, 7212-7219). Accordingly, the compound of the above Chemical Formula 1 has excellent chemical structural stability and thermal stability compared to a compound of the same structure that does not contain deuterium in the molecule, and thus may significantly improve the service life characteristics of the device. In particular, when the compound of the above Chemical Formula 1 is applied as a hole transport layer material of the organic EL device, the device may be driven at a low voltage due to the deuterium (D) substitution in the molecular structure, and thus the service life of the device may be improved.

In addition, in the compound of Chemical Formula 1 according to the present disclosure, the spiro ring has a structure in which a monocyclic or polycyclic aromatic ring, such as a benzene ring or a naphthalene ring, is fused to a benzene portion on one side of fluorene unit in a spiro[fluorene-xanthene] moiety or a spiro[fluorene-thioxanthene] moiety, thereby increasing hole mobility and providing excellent hole transport ability. In addition, the compound of Chemical Formula 1 has a HOMO energy level and a LUMO energy level between HOMO and LUMO energy levels of the hole injection layer and the electroluminescent layer, thereby facilitating hole injection and transfer. Accordingly, when the compound of the present disclosure is included in the organic EL device as a hole transport layer material, the electroluminescent efficiency of the organic EL device may be improved, and the driving voltage may also be lowered and the service life may be increased.

In addition, in the compound of the above Chemical Formula 1, since the amine group substituted with aryl and/or heteroaryl is introduced to a benzene portion on another side of fluorene unit in the spiro ring, luminescence efficiency may be further improved due to the physicochemical characteristics of amorphous characteristics and high refractive index characteristics. In addition, the compound of the above Chemical Formula 1 has a high glass transition temperature (Tg), accordingly, the compound is not only excellent in stability but also excellent in electrochemical stability.

As described above, the compound represented by Chemical Formula 1 according to the present disclosure has excellent chemical structural stability, thermal stability, electrochemical stability, and hole transport properties. Accordingly, the compound represented by Chemical Formula 1 of the present disclosure may be used as an organic layer material, preferably a hole transport layer material or a hole transport auxiliary layer material, more preferably a hole transport layer material, of an organic EL device. The organic EL device including the compound of the present disclosure may have significantly improved performance and service life characteristics, and a full-color organic EL panel to which the organic EL device is applied may also have its performance maximized.

In the compound represented by Chemical Formula 1 according to the present disclosure, (D)n means the number of deuterium (D) included in the compound of Chemical Formula 1, and n≥1. That is, the compound of the present disclosure has a structure in which a spiro ring and an amine group substituted with aryl and/or heteroaryl are connected to each other on both sides of the molecule, directly or via a linker group (e.g., a phenylene group, etc.), and at least one deuterium (D) is essentially included in the molecular structure. For example, 1≤n≤87 may be satisfied, and more specifically, 1≤n≤47 may be satisfied.

In addition, in the compound represented by Chemical Formula 1, X₁ may be O or S. Accordingly, the spiro ring may be a moiety formed by fusing an aromatic ring to a benzene portion on one side of fluorene unit in spiro[fluorene-xanthene], or a moiety formed by fusing an aromatic ring on a benzene portion on one side of fluorene unit in spiro[fluorene-thioxanthene]. The compound of Chemical Formula 1 according to the present disclosure containing such a spiro ring may have excellent amphoteric properties of electrons and holes, and thus have excellent carrier transport ability, compared to a case when X₁ is N or C. Accordingly, when the compound of Chemical Formula 1 according to the present disclosure is used as a hole transport layer material, the efficiency and driving voltage enhancement effects may be improved due to the improved hole transport ability.

In the compound represented by the above Chemical Formula 1, a CyA ring may be a ring fused to a benzene portion on one side of fluorene unit in the spiro ring and may be a C₆ to C₃₀ aromatic ring, and specifically, may be a C₆ to C₃₀ monocyclic aromatic ring or a C₆ to C₃₀ polycyclic aromatic ring. In an example, the CyA ring may be, but is not limited to, a benzene ring, a naphthalene ring, an anthracene ring, a tetracene ring, a pyrene ring, a phenanthrene ring, a phenalene ring, a benzoanthracene ring, a benzopyrene ring, a triphenylene ring, a chrysene ring, a pentaphene ring, a pentacene ring, and a fused ring between the two types of rings described above. Here, each ring in the polycyclic aromatic ring may be the same as or different from each other.

Depending on the type of the CyA ring, the 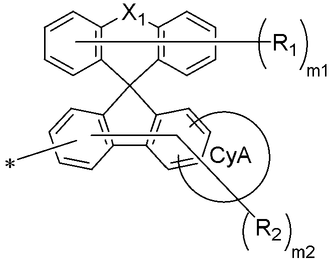 moiety in the compound represented by Chemical Formula 1 may be any one of the following moieties Mo1-1 to Mo1-3. However, the present disclosure is not limited thereto.

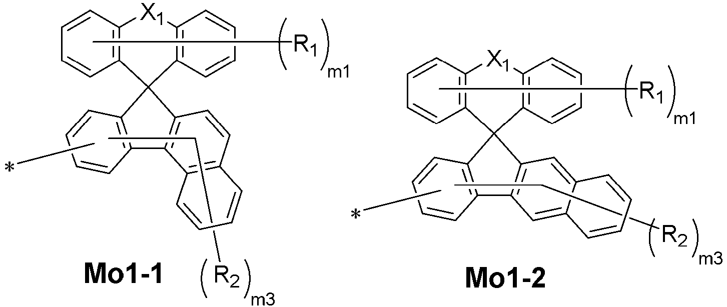

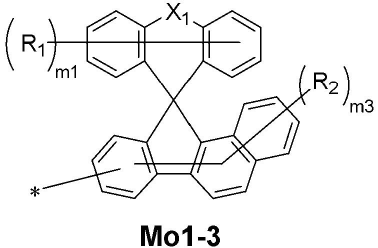

In the above moieties Mo1-1 to Mo1-3,
* may represent a site bonded to Chemical Formula 1,
X₁, m1, R₁, and R₂ may each be as defined in Chemical Formula 1, and
m3 may be an integer in a range of 0 to 9.

The compound represented by Chemical Formula 1 may be a compound represented by any one of the following Chemical Formulas 2 to 22, depending on the bonding position of deuterium included in the compound and the type of the CyA ring. However, the present disclosure is not limited thereto.

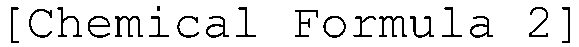

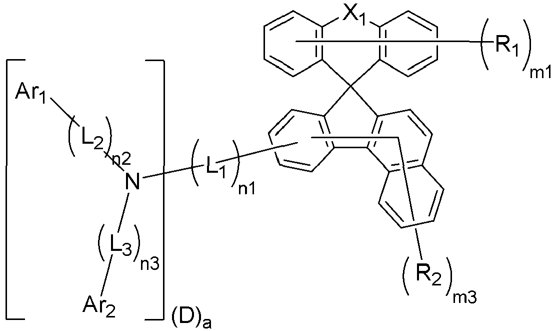

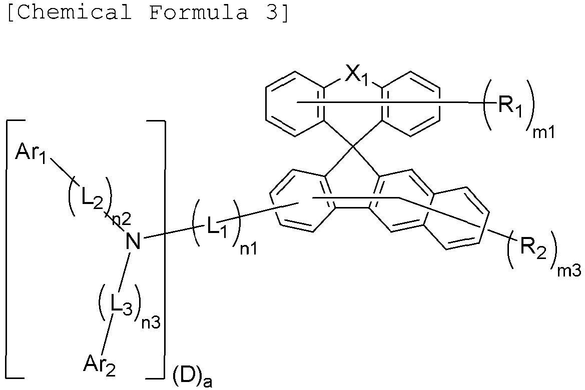

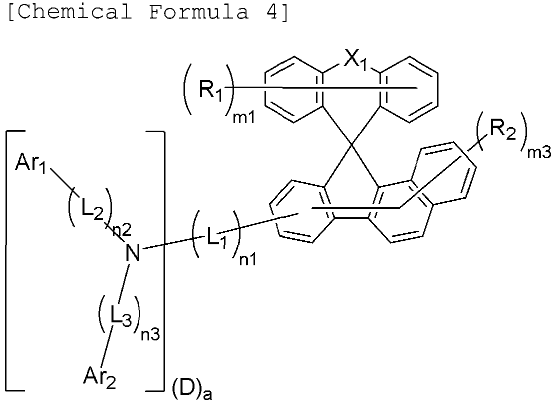

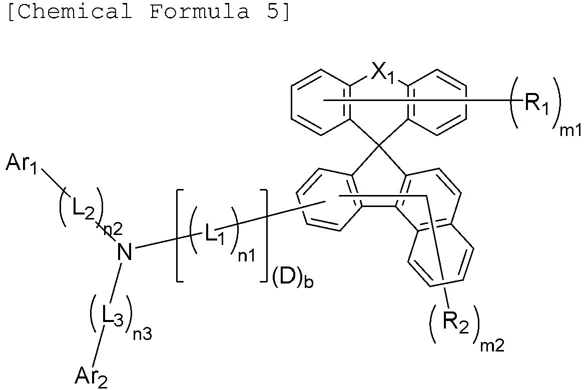

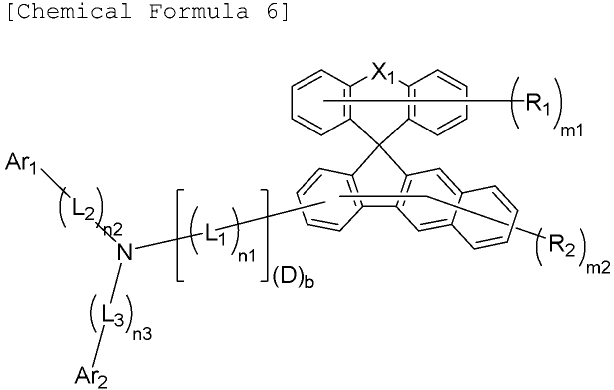

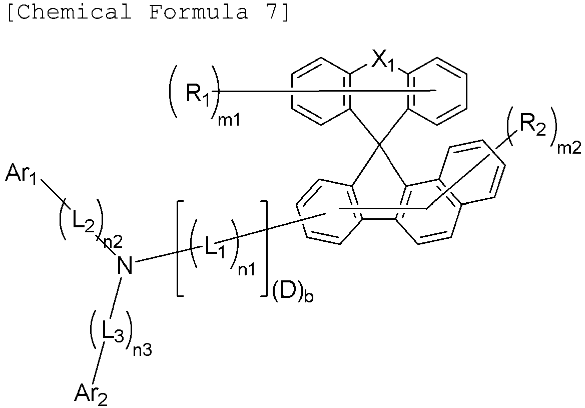

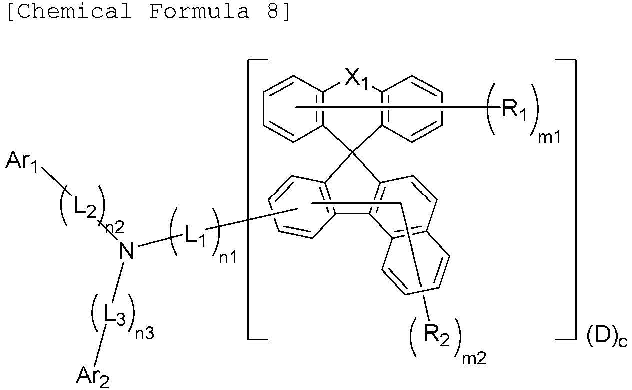

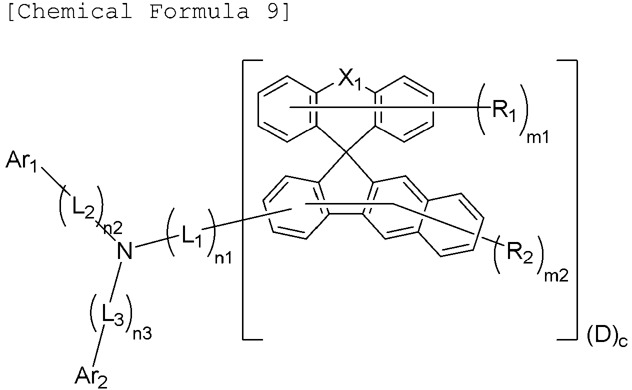

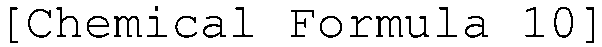

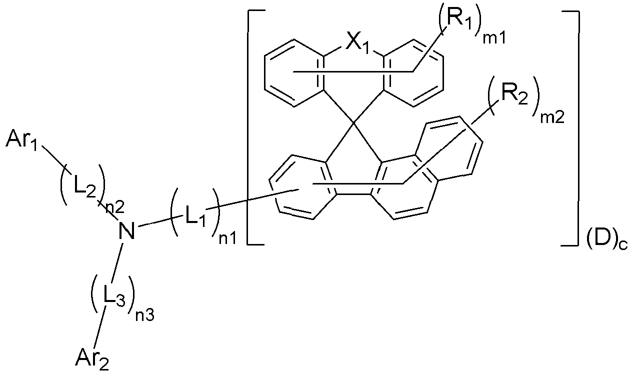

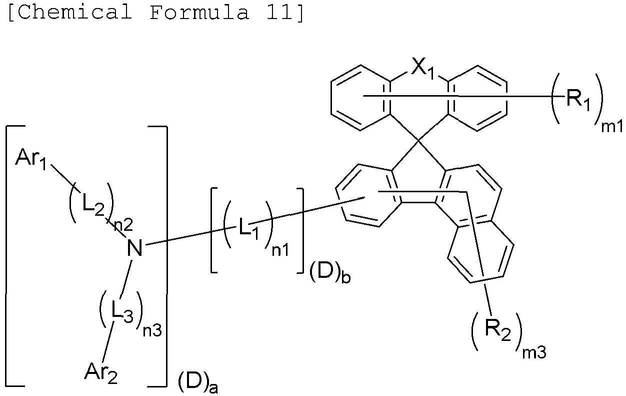

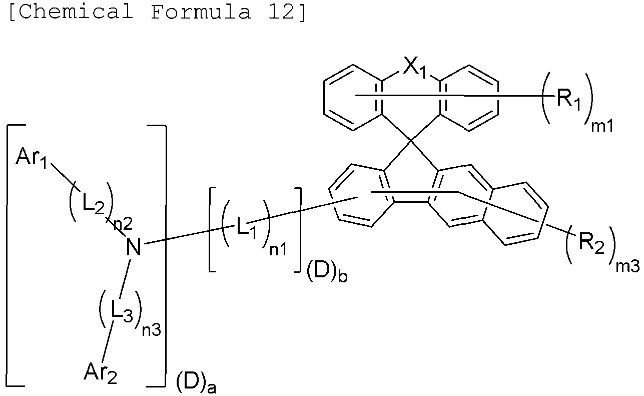

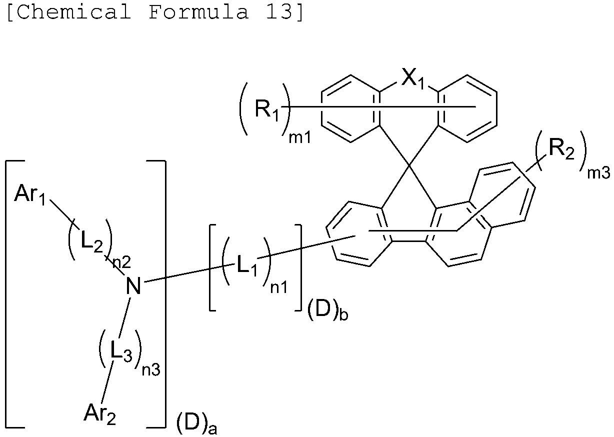

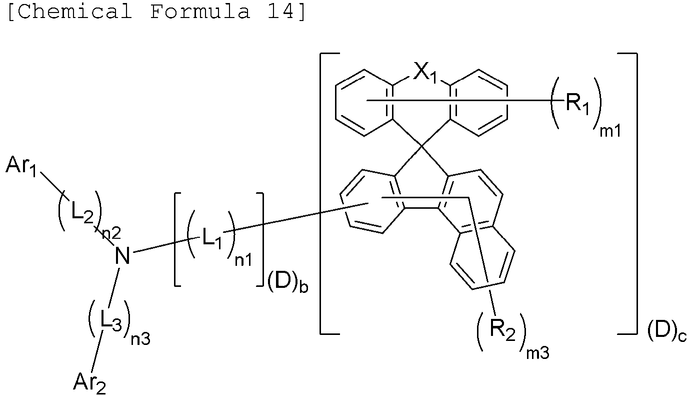

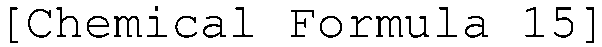

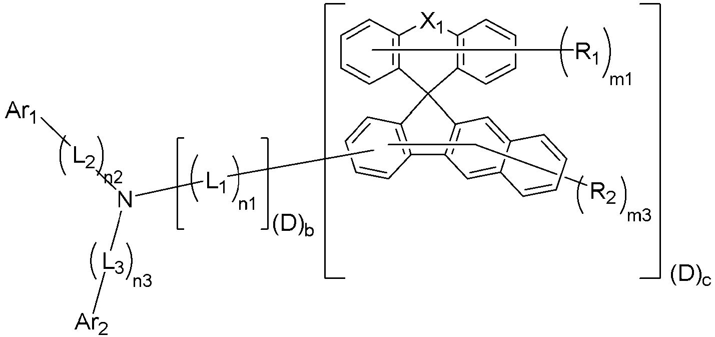

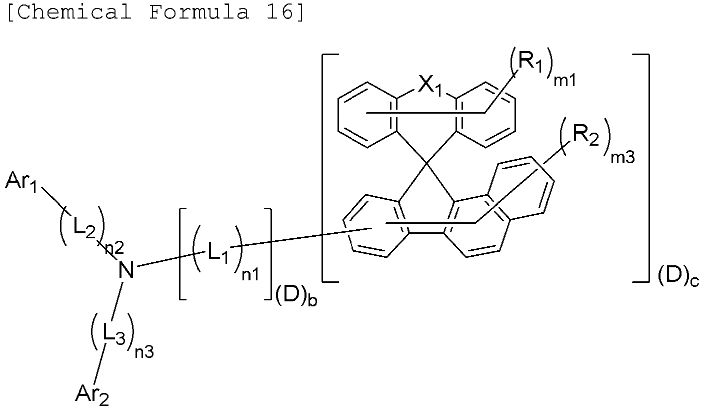

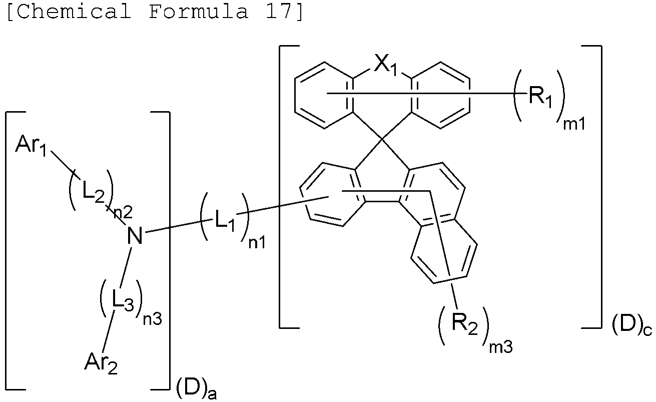

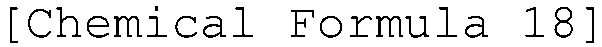

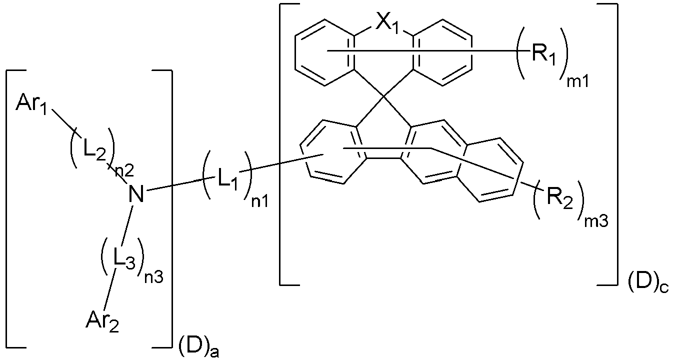

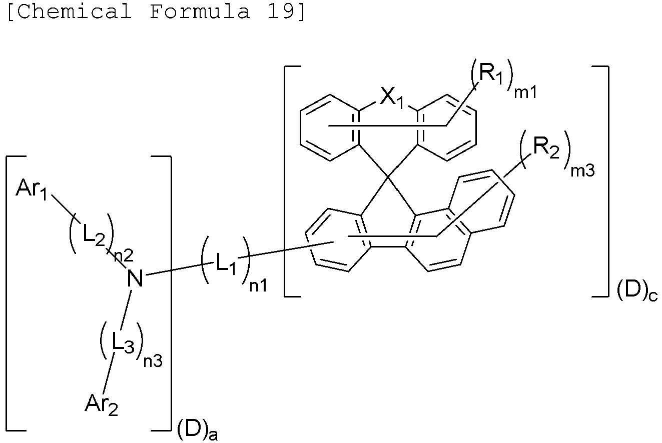

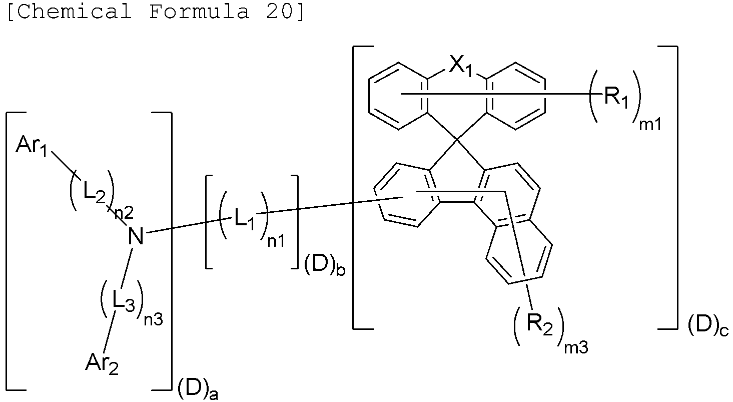

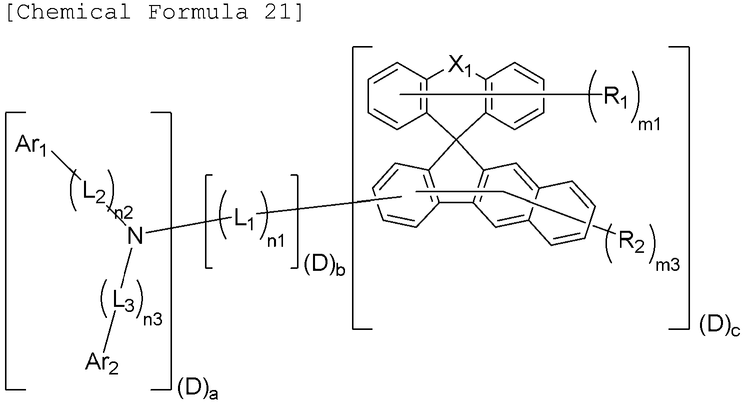

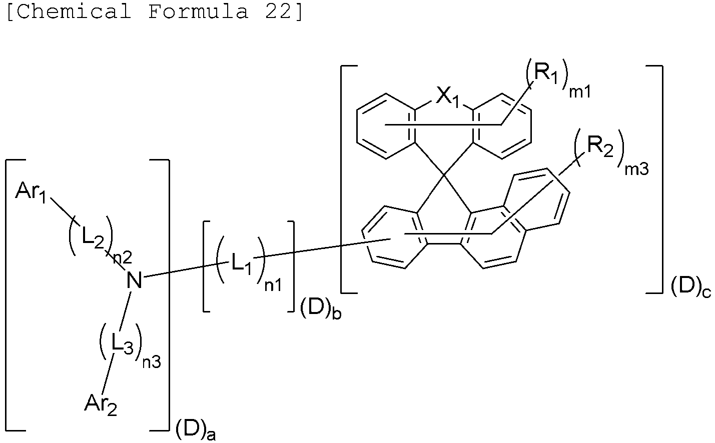

In Chemical Formulas 2 to 22 above,
X₁, m1, R₁, R₂, n1 to n3, L₁ to L₃, Ar₁, and Ar₂ may each be as defined in Chemical Formula 1 above,
m3 may be an integer in a range of 0 to 9,
(D)ₐ may represent the number of deuterium (D) included in the 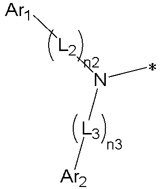 moiety, a may be an integer in a range of 0 to 58, and specifically, a may be an integer in a range of 0 to 26,
(D)_{b} may represent the number of deuterium included in the 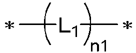 moiety, b may be an integer in a range of 0 to 12, and specifically, b may be an integer in a range of 0 to 4, and
(D)_{c} may represent the number of deuterium included in the 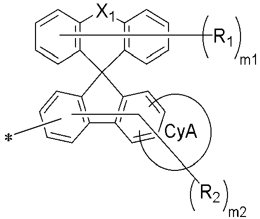 moiety, c may be an integer in a range of 0 to 17, and specifically, c may be an integer in a range of 0 to 17,
where a+b+c≥1, and specifically, 1≤a+b+c≤39.

In the compound represented by Chemical Formula 1 according to the present disclosure, n1 to n3 may each be an integer in a range of 0 to 3, and specifically, may be 0 or 1.

Here, when n1 to n3 are each 0, it means that L₁ to L₃ may each be a single bond (direct bond). Meanwhile, when n1 to n3 each is an integer in a range of 1 to 3, L₁ to L₃ may be divalent linkers, which may be the same as or different from each other, and may each independently be selected from the group consisting of: a C₆ to C₃₀ arylene group and a heteroarylene group having 5 to 30 nuclear atoms, and specifically, may be selected from the group consisting of a C₆ to C₁₈ arylene group and a heteroarylene group having 5 to 18 nuclear atoms. Here, one or more L₁, one or more L₂, and one or more L₃ may be the same as or different from each other.

In such a case, the arylene group and the heteroarylene group of L₁ to L₃ may each independently be substituted or unsubstituted with one or more substituents selected from the group consisting of: deuterium (D), halogen (e.g., -F, -Cl, -Br, -I, etc.), cyano group (-CN), nitro group (-NO₂), amino group (-NH₂), C₁ to C₄₀ alkyl group, C₂ to C₄₀ alkenyl group, C₂ to C₄₀ alkynyl group, C₃ to C₄₀ cycloalkyl group, heterocycloalkyl group having 3 to 40 nuclear atoms, C₆ to C₆₀ aryl group, heteroaryl group having 5 to 60 nuclear atoms, C₁ to C₄₀ alkyloxy group, C₆ to C₆₀ aryloxy group, C₁ to C₄₀ alkylsilyl group, C₆ to C₆₀ arylsilyl group, C₁ to C₄₀ alkylboron group, C₆ to C₆₀ arylboron group, C₆ to C₆₀ arylphosphine group, C₆ to C₆₀ arylphosphine oxide group, C₆ to C₆₀ arylamine group, C₆ to C₆₀ (aryl) (heteroaryl)amine group and heteroarylamine group having 5 to 60 nuclear atoms, and specifically, it may be substituted or unsubstituted with one or more substituents selected from the group consisting of deuterium (D), cyano group (-CN), C₁ to C₂₀ alkyl group, C₃ to C₂₀ cycloalkyl group, heterocycloalkyl group having 3 to 20 nuclear atoms, C₆ to C₃₀ aryl group, heteroaryl group having 5 to 30 nuclear atoms, and C₆ to C₃₀ arylamine group, and more specifically, it may be substituted with at least one deuterium (D). In such a case, when the substituents are plural, they may be the same as or different from each other.

In an example, L₁ to L₃ may be the same as or different from each other, and may each independently be a single bond, or may be selected from the group consisting of phenylene group, biphenylene group, terphenylene group, naphthalene group, phenanthrene group, triphenylene group, carbazole group, dibenzofuran group, dibenzothiophene group, fluorene group, and combinations thereof. Here, hydrogens of the phenylene group, the biphenylene group, the terphenylene group, the naphthalene group, the phenanthrene group, the triphenylene group, the carbazole group, the dibenzofuran group, the dibenzothiophene group and the fluorene group may be substituted or unsubstituted with one or more substituents selected from the group consisting of deuterium (D), halogen (e.g., -F, -Cl, -Br, -I, etc.), cyano group (-CN), nitro group (-NO₂), amino group (-NH₂), C₁ to C1₂ alkyl group, C₆ to C₁₀ aryl group, and heteroaryl group having 5 to 10 nuclear atoms, and specifically, may be substituted with at least one deuterium (D).

In another example, L₁ to L₃ may be the same as or different from each other, and may each independently be the following linker group L1-1. However, the present disclosure is not limited thereto.

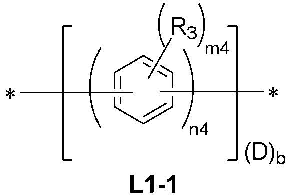

In the above linker group L1-1,
* may represent a site bonded to Chemical Formula 1,
(D)_{b} may represent the number of deuterium included in the linker group L1-1, 0≤b≤12 may be satisfied, and specifically 0≤b≤4 may be satisfied,
n4 may be an integer in a range of 0 to 3,
m4 may be an integer in a range of 0 to 4,
a plurality of R₃ may be the same as or different from each other,
R₃ may be selected from the group consisting of hydrogen, deuterium (D), halogen (e.g., -F, -Cl, -Br, -I, etc.), cyano group (-CN), nitro group (-NO₂), amino group (-NH₂), C₁ to C₄₀ alkyl group, C₂ to C₄₀ alkenyl group, C₂ to C₄₀ alkynyl group, C₃ to C₄₀ cycloalkyl group, heterocycloalkyl group having 3 to 40 nuclear atoms, C₆ to C₆₀ aryl group, heteroaryl group having 5 to 60 nuclear atoms, C₁ to C₄₀ alkyloxy group, C₆ to C₆₀ aryloxy group, C₁ to C₄₀ alkylsilyl group, C₆ to C₆₀ arylsilyl group, C₁ to C₄₀ alkylboron group, C₆ to C₆₀ arylboron group, C₆ to C₆₀ arylphosphine group, C₆ to C₆₀ arylphosphine oxide group, C₆ to C₆₀ arylamine group, C₆ to C₆₀ (aryl) (heteroaryl)amine group and heteroarylamine group having 5 to 60 nuclear atoms, or R₃ may be fused with an adjacent group (e.g., R₃-R₃) to form a fused ring, and specifically, it may be selected from the group consisting of deuterium (D), halogen (e.g., -F, -Cl, -Br, -I, etc.), cyano group (-CN), nitro group (-NO₂), amino group (-NH₂), C₁ to C₂₀ alkyl group, heterocycloalkyl group having 3 to 20 nuclear atoms, C₆ to C₃₀ aryl group, heteroaryl group having 5 to 30 nuclear atoms, and C₆ to C₃₀ arylamine group.

In particular, when L₁ is the linker group L1-1, the 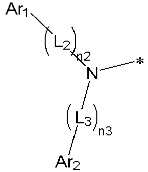 moiety and the 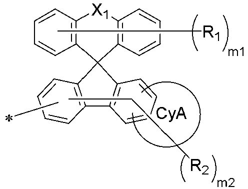 moiety may be introduced to L₁ at a para position with respect to each other. That is, when the 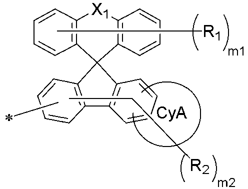 moiety is introduced (substituted) at the carbon position 1 of L₁, the 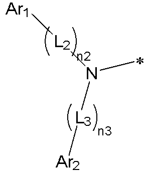 moiety may be introduced at the para-position (i.e., the carbon position 4) with respect to the 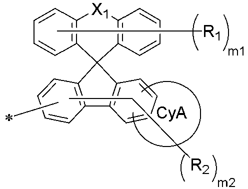 moiety. In such a case, the compound of Chemical Formula 1 not only maximizes the hole transport ability, but also facilitates intermolecular hole transfer, and thus the driving voltage of the organic EL device may be lowered.

In the compound represented by Chemical Formula 1 according to the present disclosure, m1 may be an integer in a range of 0 to 8, m2 may be an integer in a range of 0 to 23, specifically, m2 may be an integer in a range of 0 to 9, and more specifically, may be an integer in a range of 1 to 5.

Herein, when each of m1 and m2 is 0, it means that hydrogen is not substituted with substituents R₁ and R₂, respectively. On the other hand, when m1 is an integer in a range of 1 to 8, it means that hydrogen is substituted with the substituent R₁, and when m2 is an integer in a range of 1 to 23, it means that hydrogen is substituted with the substituent R₂. In such a case, the plurality of R₁ may be the same as or different from each other, and the plurality of R₂s may be the same as or different from each other.

The above R₁ and R₂ may be the same as or different from each other, and may each independently be selected from the group consisting of hydrogen, deuterium (D), halogen (e.g., -F, -Cl, - Br, -I, etc.), cyano group (-CN), nitro group (-NO₂), amino group (-NH₂), C₁ to C₄₀ alkyl group, C₂ to C₄₀ alkenyl group, C₂ to C₄₀ alkynyl group, C₃ to C₄₀ cycloalkyl group, heterocycloalkyl group having 3 to 40 nuclear atoms, C₆ to C₆₀ aryl group, heteroaryl group having 5 to 60 nuclear atoms, C₁ to C₄₀ alkyloxy group, C₆ to C₆₀ aryloxy group, C₁ to C₄₀ alkylsilyl group, C₆ to C₆₀ arylsilyl group, C₁ to C₄₀ alkylboron group, C₆ to C₆₀ arylborone group, C₆ to C₆₀ arylphosphine group, C₆ to C₆₀ arylphosphine oxide group, C₆ to C₆₀ arylamine group, C₆ to C₆₀ (aryl) (heteroaryl)amine group, and heteroarylamine group having 5 to 60 nuclear atoms, or R₁ and R₂ may each independently be fused with an adjacent group thereof (e.g., R₁-R₁, R₂-R₂, etc.) to form a fused ring, and specifically, R₁ and R₂ may be the same as or different from each other, and may each independently be selected from the group consisting of hydrogen, deuterium (D), halogen group, cyano group, nitro group, amino group, C₁ to C₂₀ alkyl group, C₃ to C₂₀ cycloalkyl group, heterocycloalkyl group having 3 to 20 nuclear atoms, C₆ to C₃₀ aryl group, heteroaryl group having 5 to 30 nuclear atoms, and C₆ to C₃₀ arylamine group, and more specifically, R₁ and R₂ may be deuterium, or C₆ to C₃₀ aryl group or heteroaryl group having 5 to 30 nuclear atoms, wherein the group is substituted with deuterium.

The alkyl group, the alkenyl group, the alkynyl group, the cycloalkyl group, the heterocycloalkyl group, the aryl group, the heteroaryl group, the alkyloxy group, the aryloxy group, the alkylsilyl group, the arylsilyl group, the alkylboron group, the arylboron group, the arylphosphine group, the arylphosphine oxide group, the arylamine group, the (aryl) (heteroaryl)amine group and the heteroarylamine group of the above R₁ and R₂ may each independently be substituted or unsubstituted with one or more substituents selected from the group consisting of deuterium (D), halogen, cyano group, nitro group, amino group, C₁ to C₄₀ alkyl group, C₂ to C₄₀ alkenyl group, C₂ to C₄₀ alkynyl group, C₃ to C₄₀ cycloalkyl group, heterocycloalkyl group having 3 to 40 nuclear atoms, C₆ to C₆₀ aryl group, heteroaryl group having 5 to 60 nuclear atoms, C₁ to C₄₀ alkyloxy group, C₆ to C₆₀ aryloxy group, C₁ to C₄₀ alkylsilyl group, C₆ to C₆₀ arylsilyl group, C₁ to C₄₀ alkylboron group, C₆ to C₆₀ arylboron group, C₆ to C₆₀ arylphosphine group, C₆ to C₆₀ arylphosphine oxide group, C₆ to C₆₀ arylamine group, C₆ to C₆₀ (aryl) (heteroaryl)amine group, and heteroarylamine group having 5 to 60 nuclear atoms, and specifically, may each independently be substituted or unsubstituted with one or more substituents selected from the group consisting of deuterium (D), cyano group (-CN), C₁ to C₂₀ alkyl group, C₃ to C₂₀ cycloalkyl group, heterocycloalkyl group having 3 to 20 nuclear atoms, C₆ to C₃₀ aryl group, heteroaryl group having 5 to 30 nuclear atoms and C₆ to C₃₀ arylamine group, and more specifically, may be substituted with at least one deuterium (D). In such a case, when the substituents are in plural, they may be the same as or different from each other.

In an example, R₁ and R₂ may be the same as or different from each other and may each independently be selected from the group consisting of deuterium (D), methyl group, ethyl group, propyl group, butyl group, phenyl group, biphenyl group, terphenyl group, and naphthyl group, and specifically, R₁ and R₂ may be deuterium. In such a case, the methyl group, the ethyl group, the propyl group, the butyl group, the phenyl group, the biphenyl group, the terphenyl group, and the naphthyl group of R₁ and R₂ may be substituted with at least one deuterium (D).

In the compound represented by Chemical Formula 1 according to the present disclosure, Ar₁ and Ar₂ may be the same as or different from each other, and may each independently be selected from the group consisting of: hydrogen, deuterium (D), halogen group, cyano group, nitro group, amino group, C₁ to C₄₀ alkyl group, C₂ to C₄₀ alkenyl group, C₂ to C₄₀ alkynyl group, C₃ to C₄₀ cycloalkyl group, heterocycloalkyl group having 3 to 40 nuclear atoms, C₆ to C₆₀ aryl group, heteroaryl group having 5 to 60 nuclear atoms, C₁ to C₄₀ alkyloxy group, C₆ to C₆₀ aryloxy group, C₁ to C₄₀ alkylsilyl group, C₆ to C₆₀ arylsilyl group, C₁ to C₄₀ alkylboron group, C₆ to C₆₀ arylboron group, C₆ to C₆₀ arylphosphine group, C₆ to C₆₀ arylphosphine oxide group, C₆ to C₆₀ arylamine group, C₆ to C₆₀ (aryl) (heteroaryl)amine group, and heteroarylamine group having 5 to 60 nuclear atoms, or Ar₁ and Ar₂ may each independently be fused with an adjacent group thereof (e.g., Ar₁-L₂, Ar₂-L₃) to form a fused ring.

The alkyl group, the alkenyl group, the alkynyl group, the cycloalkyl group, the heterocycloalkyl group, the aryl group, the heteroaryl group, the alkyloxy group, the aryloxy group, the alkylsilyl group, the arylsilyl group, the alkylboron group, the arylboron group, the arylphosphine group, the arylphosphine oxide group, the arylamine group, the (aryl) (heteroaryl)amine group and the heteroarylamine group of the above Ar₁ and Ar₂ may each independently be substituted or unsubstituted with one or more substituents selected from the group consisting of deuterium (D), halogen, cyano group, nitro group, amino group, C₁ to C₄₀ alkyl group, C₂ to C₄₀ alkenyl group, C₂ to C₄₀ alkynyl group, C₃ to C₄₀ cycloalkyl group, heterocycloalkyl group having 3 to 40 nuclear atoms, C₆ to C₆₀ aryl group, heteroaryl group having 5 to 60 nuclear atoms, C₁ to C₄₀ alkyloxy group, C₆ to C₆₀ aryloxy group, C₁ to C₄₀ alkylsilyl group, C₆ to C₆₀ arylsilyl group, C₁ to C₄₀ alkylboron group, C₆ to C₆₀ arylboron group, C₆ to C₆₀ arylphosphine group, C₆ to C₆₀ arylphosphine oxide group, C₆ to C₆₀ arylamine group, C₆ to C₆₀ (aryl) (heteroaryl)amine group, and heteroarylamine group having 5 to 60 nuclear atoms, and specifically, may each independently be substituted or unsubstituted with one or more substituents selected from the group consisting of deuterium (D), cyano group (-CN), C₁ to C₂₀ alkyl group, C₃ to C₂₀ cycloalkyl group, heterocycloalkyl group having 3 to 20 nuclear atoms, C₆ to C₃₀ aryl group, heteroaryl group having 5 to 30 nuclear atoms, and C₆ to C₃₀ arylamine group, and more specifically, may be substituted with at least one deuterium (D). In such a case, when the substituents are plural, they may be the same as or different from each other.

In an example, Ar₁ and Ar₂ may be the same as or different from each other, and may each independently be selected from the group consisting of C₆ to C₆₀ aryl group and heteroaryl group having 5 to 60 nuclear atoms. In such a case, the aryl group and the heteroaryl group of Ar₁ and Ar₂ may be substituted with one or more deuterium.

In another example, Ar₁ and Ar₂ may be the same as or different from each other, and may each independently be selected from the group consisting of the following substituents S1-1 to S1-3. However, the present disclosure is not limited thereto.

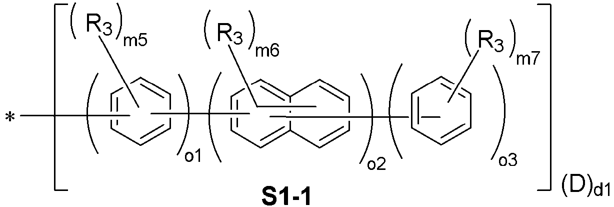

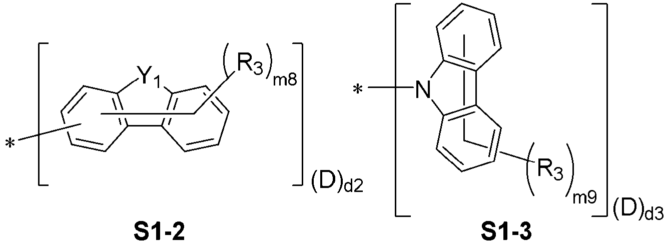

(In the above substituents S1-1 to S1-3,
* may represent a site bonded to Chemical Formula 1,
(D)_{d1} may represent the number of deuterium (D) included in the substituent S1-1, 0≤d1≤15 may be satisfied, and specifically 0≤d1≤15 may be satisfied,
(D)_{d2} may represent the number of deuterium (D) included in the substituent S1-2, 0≤d2≤7 may be satisfied, and specifically 0≤d2≤7 may be satisfied,
(D)_{d3} may represent the number of deuterium (D) included in the substituent S1-3, 0≤d3≤8 may be satisfied, and specifically 0≤d3≤8 may be satisfied,
o1 to o3 may each be 0 or 1, where o1+o2+o3≥1 may be satisfied,
m5 may be an integer in a range of 0 to 4,
m6 may be an integer in a range of 0 to 6,
m7 may be an integer in a range of 0 to 5,
m8 may be an integer in a range of 0 to 7,
m9 may be an integer in a range of 0 to 8,
Y₁ may be selected from the group consisting of O, S, C(Ar₃)(Ar₄), and N(Ar₅),
R₃ and Ar₃ to Ar₅ may be the same as or different from each other, and may each independently be selected from the group consisting of hydrogen, deuterium (D), halogen group, cyano group, nitro group, amino group, C₁ to C₄₀ alkyl group, C₂ to C₄₀ alkenyl group, C₂ to C₄₀ alkynyl group, C₃ to C₄₀ cycloalkyl group, heterocycloalkyl group having 3 to 40 nuclear atoms, C₆ to C₆₀ aryl group, heteroaryl group having 5 to 60 nuclear atoms, C₁ to C₄₀ alkyloxy group, C₆ to C₆₀ aryloxy group, C₁ to C₄₀ alkylsilyl group, C₆ to C₆₀ arylsilyl group, C₁ to C₄₀ alkylboron group, C₆ to C₆₀ arylboron group, C₆ to C₆₀ arylphosphine group, C₆ to C₆₀ arylphosphine oxide group, C₆ to C₆₀ arylamine group, C₆ to C₆₀ (aryl) (heteroaryl)amine group, and heteroarylamine group having 5 to 60 nuclear atoms, or R₃ and Ar₃ to Ar₅ may each independently be fused with an adjacent group thereof (e.g., R₃-R₃, Ar₃-Ar₄, etc.) to form a fused ring,
and the alkyl group, the alkenyl group, the alkynyl group, the cycloalkyl group, the heterocycloalkyl group, the aryl group, the heteroaryl group, the alkyloxy group, the aryloxy group, the alkylsilyl group, the arylsilyl group, the alkylboron group, the arylboron group, the arylphosphine group, the arylphosphine oxide group, the arylamine group, the (aryl) (heteroaryl)amine group and the heteroarylamine group of the above Ar₃ to Ar₅ may each independently be substituted or unsubstituted with one or more substituents selected from the group consisting of deuterium (D), halogen, cyano group, nitro group, amino group, C₁ to C₄₀ alkyl group, C₂ to C₄₀ alkenyl group, C₂ to C₄₀ alkynyl group, C₃ to C₄₀ cycloalkyl group, heterocycloalkyl group having 3 to 40 nuclear atoms, C₆ to C₆₀ aryl group, heteroaryl group having 5 to 60 nuclear atoms, C₁ to C₄₀ alkyloxy group, C₆ to C₆₀ aryloxy group, C₁ to C₄₀ alkylsilyl group, C₆ to C₆₀ arylsilyl group, C₁ to C₄₀ alkylboron group, C₆ to C₆₀ arylboron group, C₆ to C₆₀ arylphosphine group, C₆ to C₆₀ arylphosphine oxide group, C₆ to C₆₀ arylamine group, C₆ to C₆₀ (aryl) (heteroaryl)amine group, and heteroarylamine group having 5 to 60 nuclear atoms, and in such a case, when the substituents are plural, they may be the same as or different from each other.

Hydrogens of the substituents S1-1 to S1-3 may be substituted or unsubstituted with one or more substituents selected from the group consisting of deuterium (D), halogen (e.g., -F, -Cl, -Br, -I, etc.), cyano group (-CN), nitro group (-NO₂), amino group (-NH₂), C₁ to C₁₂ alkyl group, C₆ to C₁₀ aryl group, and heteroaryl group having 5 to 10 nuclear atoms, and specifically, may be substituted with deuterium (D).

In another example, Ar₁ and Ar₂ may be the same as or different from each other, and may each independently be selected from the group consisting of the following substituents S2-1 to S2-12. However, the present disclosure is not limited thereto.

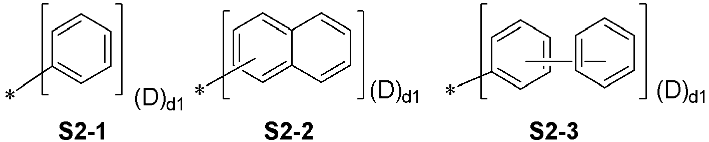

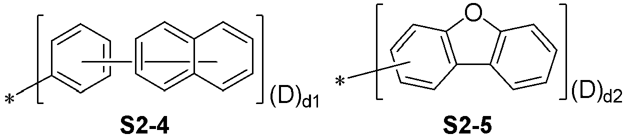

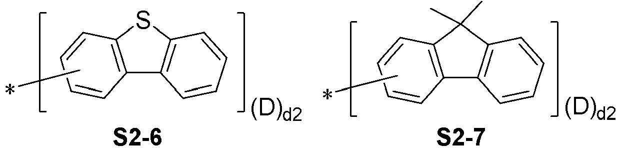

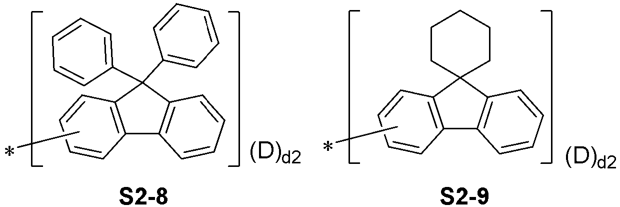

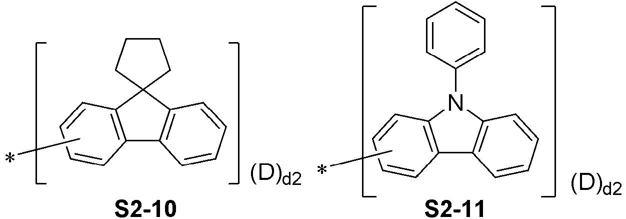

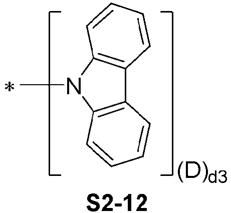

In the substituents S2-1 to S2-12,
* may represent a site bonded to Chemical Formula 1,
(D) _{d1} may represent the number of deuterium (D) included in each of the substituents S2-1 to S2-4, 0≤d1≤15 may be satisfied, and specifically 1≤d1≤15 may be satisfied,
(D)_{d2} may represent the number of deuterium (D) included in each of the substituents S2-5 to S2-11, 0≤d2≤7 may be satisfied, and specifically 1≤d2≤4 may be satisfied,
(D)_{d3} may represent the number of deuterium (D) included in the substituent S2-12, 0≤d3≤8 may be satisfied, and specifically 1≤d3≤4 may be satisfied.

Hydrogens of the above substituents S2-1 to S2-12 may be substituted or unsubstituted with one or more substituents selected from the group consisting of deuterium (D), halogen (e.g., -F, -Cl, -Br, -I, etc.), cyano group (-CN), nitro group (-NO₂), amino group (-NH₂), C₁ to C₁₂ alkyl group, C₆ to C₁₀ aryl group, and heteroaryl group having 5 to 10 nuclear atoms, and specifically may be substituted with deuterium (D).

Depending on the type of the above ring CyA, L₁, and Ar₁, the compound represented by the above Chemical Formula 1 may be a compound represented by any one of the following Chemical Formulas 23 to 31. However, the present disclosure is not limited thereto.

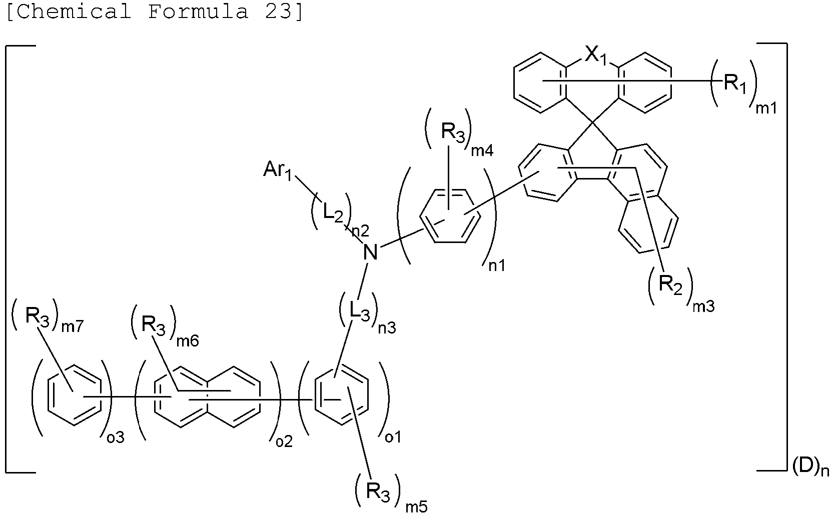

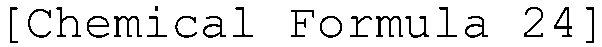

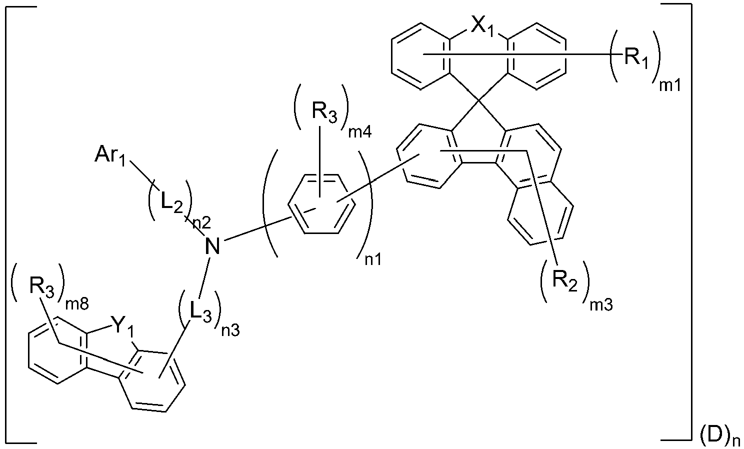

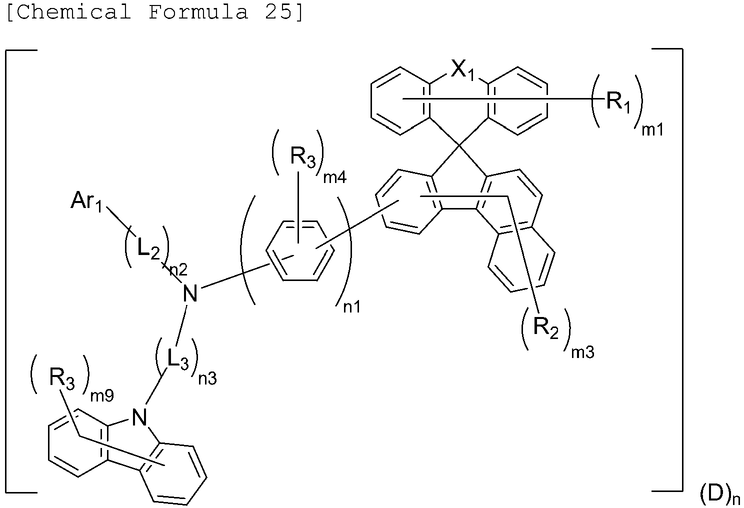

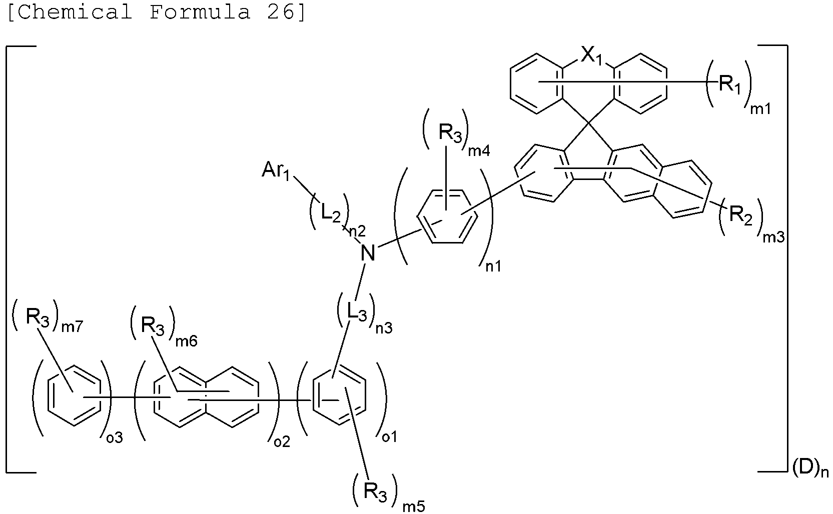

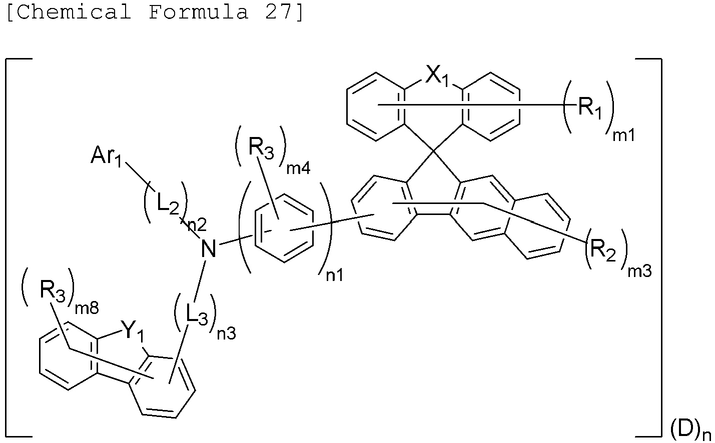

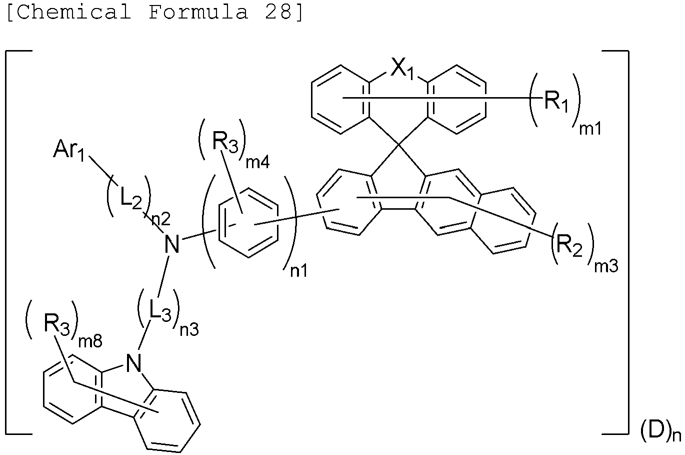

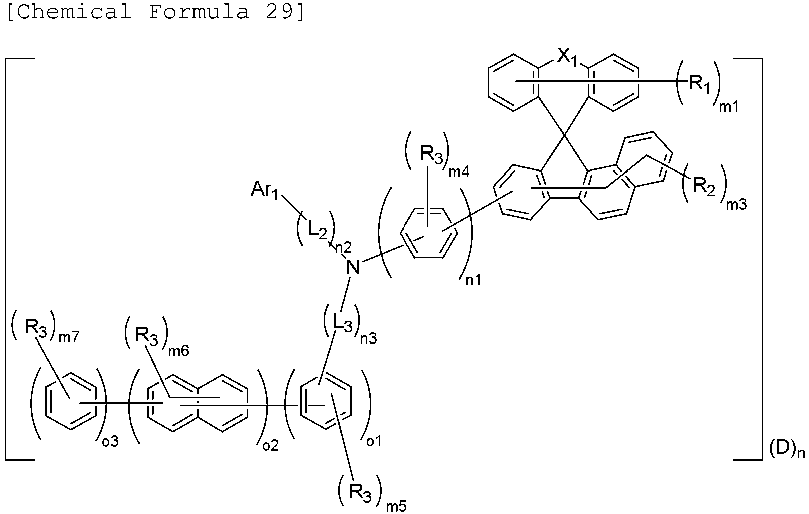

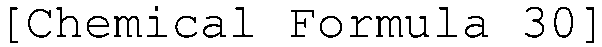

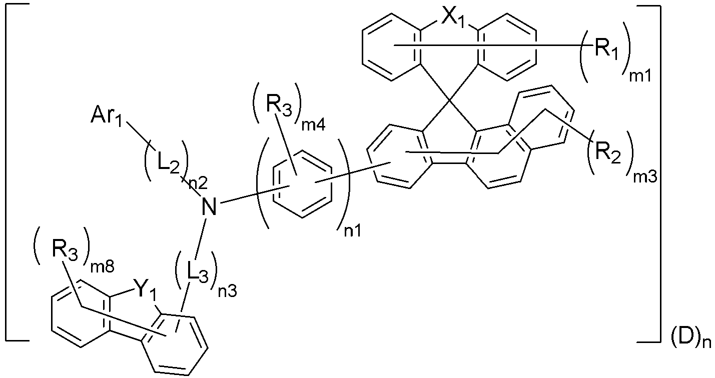

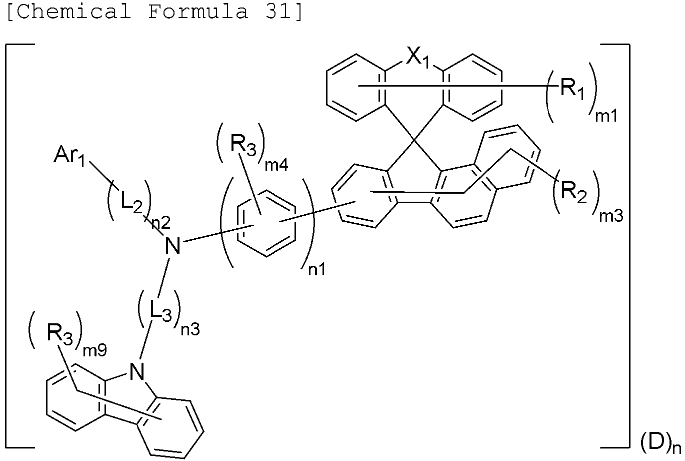

In Chemical Formulas 23 to 31 above,
X₁, n1 to n3, L₂, L₃, m1, R₁, R₂, and Ar₁ may each be as defined in Chemical Formula 1 above,
(D)n may represent the number of deuterium (D) included in the compound, 1≤n≤87 may be satisfied, and specifically, 1≤n≤53 may be satisfied,
m3 may be an integer in a range of 0 to 9,
m4 may be an integer in a range of 0 to 4,
o1 to o3 may each be 0 or 1, where o1+o2+o3≥1 may be satisfied,
m5 may be an integer in a range of 0 to 4,
m6 may be an integer in a range of 0 to 6,
m7 may be an integer in a range of 0 to 5,
m8 may be an integer in a range of 0 to 7,
m9 may be an integer in a range of 0 to 8,
Y₁ may be selected from the group consisting of O, S, C(Ar₃)(Ar₄), and N(Ar₅),
R₃ and Ar₃ to Ar₅ may be the same as or different from each other, and may each independently be selected from the group consisting of hydrogen, deuterium (D), halogen group, cyano group, nitro group, amino group, C₁ to C₄₀ alkyl group, C₂ to C₄₀ alkenyl group, C₂ to C₄₀ alkynyl group, C₃ to C₄₀ cycloalkyl group, heterocycloalkyl group having 3 to 40 nuclear atoms, C₆ to C₆₀ aryl group, heteroaryl group having 5 to 60 nuclear atoms, C₁ to C₄₀ alkyloxy group, C₆ to C₆₀ aryloxy group, C₁ to C₄₀ alkylsilyl group, C₆ to C₆₀ arylsilyl group, C₁ to C₄₀ alkylboron group, C₆ to C₆₀ arylboron group, C₆ to C₆₀ arylphosphine group, C₆ to C₆₀ arylphosphine oxide group, C₆ to C₆₀ arylamine group, C₆ to C₆₀ (aryl) (heteroaryl)amine group, and heteroarylamine group having 5 to 60 nuclear atoms, or R₃ and Ar₃ to Ar₅ may each independently be fused with an adjacent group thereof to form a fused ring, and
the alkyl group, the alkenyl group, the alkynyl group, the cycloalkyl group, the heterocycloalkyl group, the aryl group, the heteroaryl group, the alkyloxy group, the aryloxy group, the alkylsilyl group, the arylsilyl group, the alkylboron group, the arylboron group, the arylphosphine group, the arylphosphine oxide group, the arylamine group, the (aryl) (heteroaryl)amine group and the heteroarylamine group of Ar₃ to Ar₅ may each independently be substituted or unsubstituted with one or more substituents selected from the group consisting of deuterium (D), halogen, cyano group, nitro group, amino group, C₁ to C₄₀ alkyl group, C₂ to C₄₀ alkenyl group, C₂ to C₄₀ alkynyl group, C₃ to C₄₀ cycloalkyl group, heterocycloalkyl group having 3 to 40 nuclear atoms, C₆ to C₆₀ aryl group, heteroaryl group having 5 to 60 nuclear atoms, C₁ to C₄₀ alkyloxy group, C₆ to C₆₀ aryloxy group, C₁ to C₄₀ alkylsilyl group, C₆ to C₆₀ arylsilyl group, C₁ to C₄₀ alkylboron group, C₆ to C₆₀ arylboron group, C₆ to C₆₀ arylphosphine group, C₆ to C₆₀ arylphosphine oxide group, C₆ to C₆₀ arylamine group, C₆ to C₆₀ (aryl) (heteroaryl)amine group, and heteroarylamine group having 5 to 60 nuclear atoms, and when the substituents are plural, they may be the same as or different from each other.

The compound represented by Chemical Formula 1 according to the present disclosure described above may be further specified as the following compounds 1 to 120, but the present disclosure is not limited thereto.

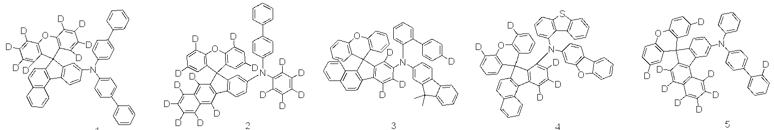

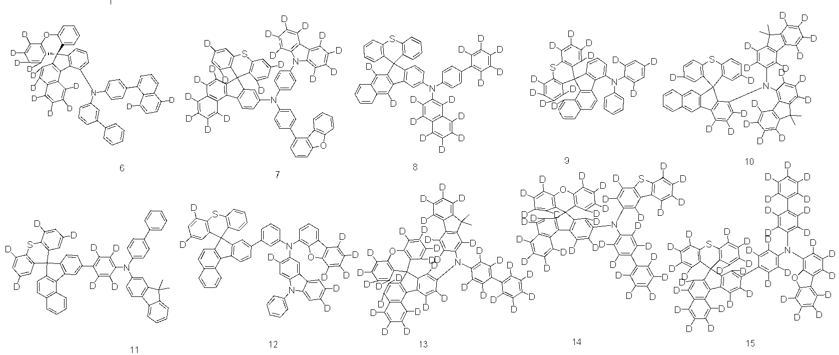

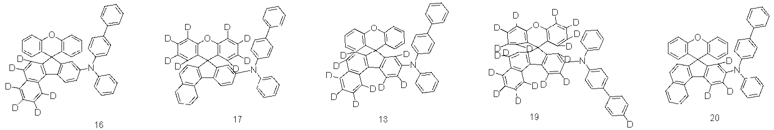

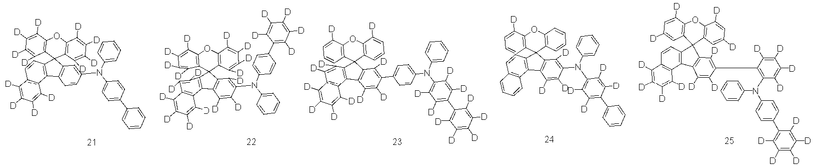

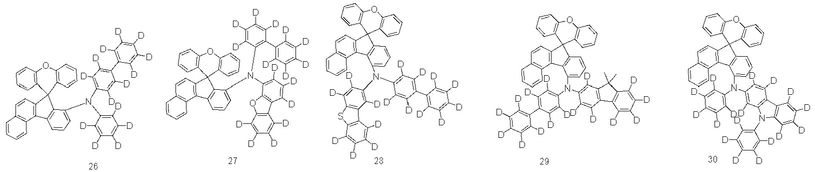

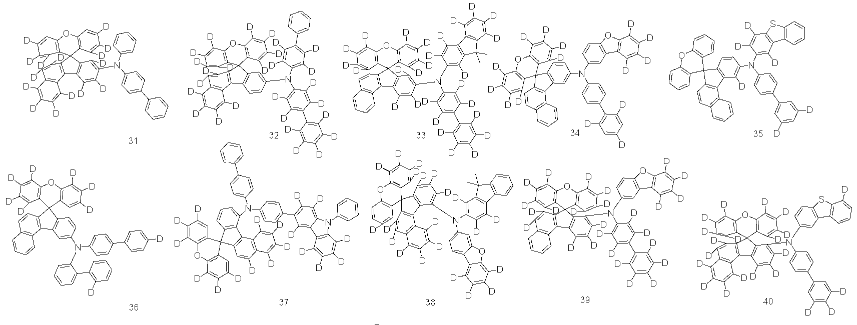

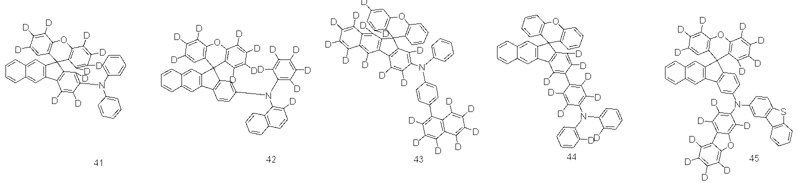

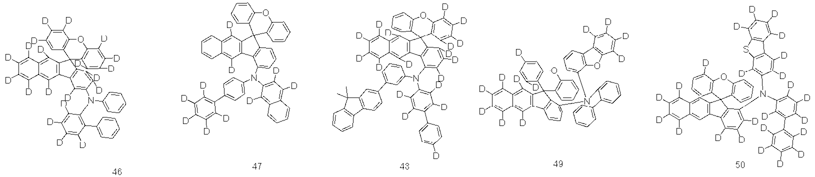

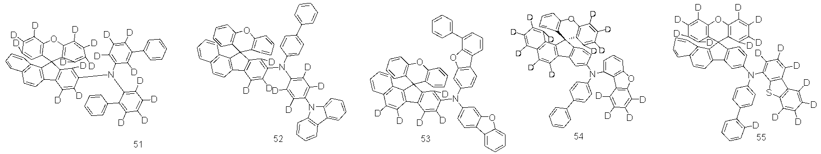

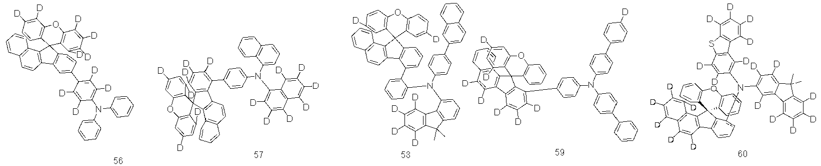

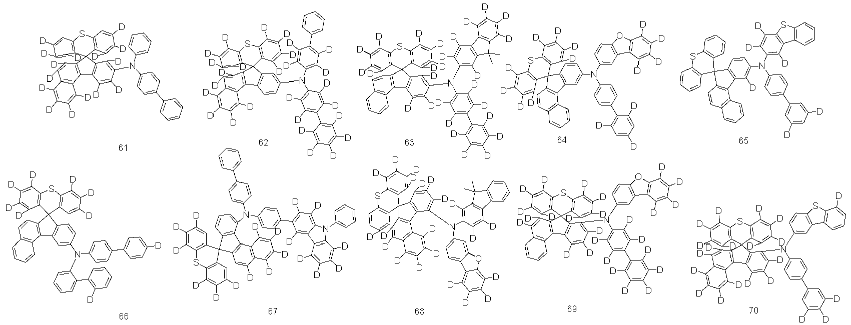

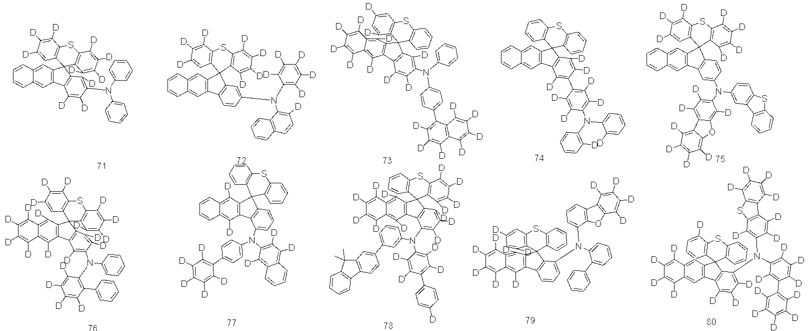

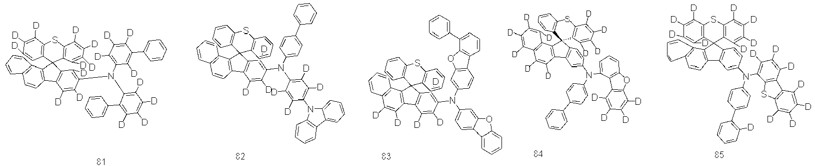

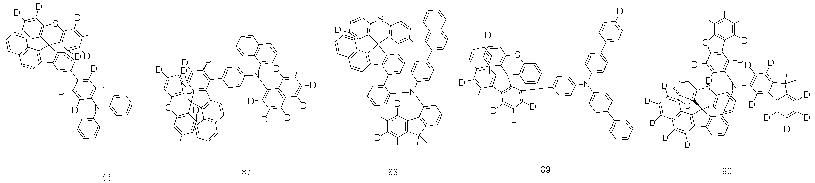

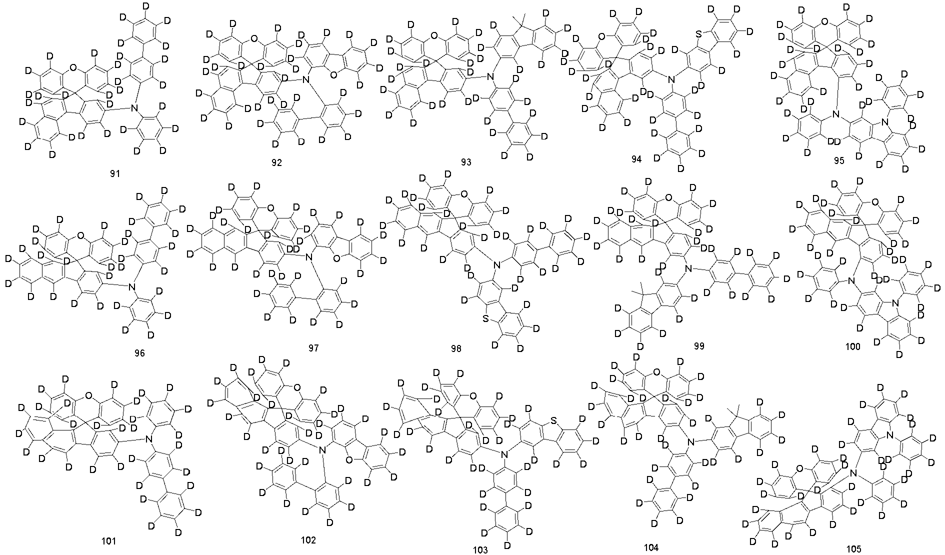

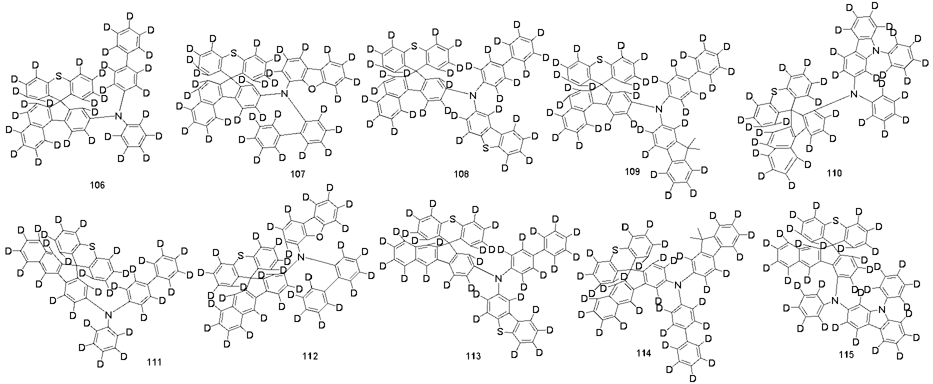

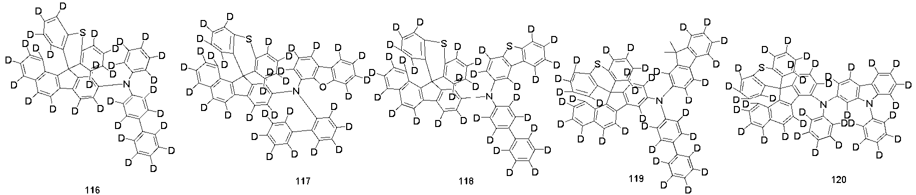

As used herein, "alkyl" may refer to a monovalent substituent derived from a linear or branched chain saturated hydrocarbon having 1 to 40 carbon atoms. Examples of such alkyl may include, but are not limited to, methyl, ethyl, propyl, isobutyl, sec-butyl, pentyl, iso-amyl, hexyl or the like.

As used herein, "alkenyl" may refer to a monovalent substituent derived from a linear or branched chain unsaturated hydrocarbon having 2 to 40 carbon atoms, having at least one carbon-carbon double bond. Examples of such alkenyl may include, but are not limited to, vinyl, allyl, isopropenyl, 2-butenyl or the like.

As used herein, "alkynyl" may refer to a monovalent substituent derived from a linear or branched chain unsaturated hydrocarbon having 2 to 40 carbon atoms, having at least one carbon-carbon triple bond. Examples of such alkynyl may include, but are not limited to, ethynyl, 2-propynyl or the like.

As used herein, "cycloalkyl" may refer to a monovalent substituent derived from a monocyclic or polycyclic non-aromatic hydrocarbon having 3 to 40 carbon atoms. Examples of such cycloalkyl may include, but are not limited to, cyclopropyl, cyclopentyl, cyclohexyl, norbornyl, adamantyl or the like.

As used herein, "heterocycloalkyl" may refer to a monovalent substituent derived from a non-aromatic hydrocarbon having 3 to 40 nuclear atoms, where one or more carbons in the ring, preferably one to three carbons, are substituted with a heteroatom such as N, O, S or Se. Examples of such heterocycloalkyl may include, but are not limited to, morpholine group, piperazine group or the like. Herein, nuclear atomic number means the number of atoms forming a ring (cyclic) structure, i.e., the number of ring atoms.

As used herein, "aryl" may refer to a monovalent substituent derived from an aromatic hydrocarbon having 6 to 60 carbon atoms which is in a structure with a single ring or two or more rings combined with each other. In addition, a form in which two or more rings are pendant to or fused with each other may also be included. Examples of such aryl may include, but are not limited to, phenyl, naphthyl, phenanthryl, anthryl or the like.

As used herein, "heteroaryl" may refer to a monovalent substituent derived from a monoheterocyclic or polyheterocyclic aromatic hydrocarbon having 5 to 60 nuclear atoms. In such a case, one or more carbons in the ring, preferably one to three carbons, are substituted with a heteroatom such as N, O, S or Se. In addition, a form in which two or more rings are pendant to or fused with each other may be included, and a form fused with an aryl group may be included. Examples of such heteroaryl may include, but are not limited to, a 6-membered monocyclic ring including, for example, pyridyl, pyrazinyl, pyrimidinyl, pyridazinyl and triazinyl; a polycyclic ring including, for example, phenoxathienyl, indolinzinyl, indolyl, purinyl, quinolyl, benzothiazole group, and carbazolyl; 2-furanyl; N-imidazolyl; 2-isoxazolyl; 2-pyridinyl; 2-pyrimidinyl or the like. Herein, nuclear atomic number means the number of atoms forming a ring (cyclic) structure, i.e., the number of ring atoms.

As used herein, "alkyloxy" refers to a monovalent substituent represented by R'O-, where R' refers to an C₁ to C₄₀ alkyl, and such alkyloxy may include a linear, branched or cyclic structure. Examples of such alkyloxy may include, but are not limited to, methoxy, ethoxy, n-propoxy, 1-propoxy, t-butoxy, n-butoxy, pentoxy or the like.

As used herein, "aryloxy" refers to a monovalent substituent represented by RO-, where R refers to a C₅ to C₄₀ aryl. Examples of such aryloxy may include, but are not limited to, phenyloxy, naphthyloxy, diphenyloxy or the like.

As used herein, "alkylsilyl" refers to a silyl substituted with a C₁ to C₄₀ alkyl and includes mono-, di- and tri-alkylsilyl. In addition, "arylsilyl" refers to a silyl substituted with a C₅ to C₆₀ aryl, and includes mono-, as well as polyarylsilyl such as di- and tri-arylsilyl.

As used herein, "alkylboron group" refers to a boron group substituted with a C₁ to C₄₀ alkyl, and "arylboron group" refers to a boron group substituted with a C₆ to C₆₀ aryl.

As used herein, "alkylphosphinyl group" refers to a phosphine group substituted with an C₁ to C₄₀ alkyl, and includes mono- and di-alkylphosphinyl group. In addition, as used herein, "arylphosphinyl group" refers to a phosphine group substituted with a C₁ to C₄₀ monoaryl or diaryl, and includes mono- and di-arylphosphinyl group.

As used herein, "arylamine group" refers to an amine substituted with a C₆ to C₄₀ aryl, and includes mono- and di-arylamine.

### <Organic electroluminescent device>

Another aspect of the present disclosure relates to an organic electroluminescent device (hereinafter, "organic EL device") (e.g., the organic EL diode) including the compound represented by Chemical Formula 1.

Specifically, the organic EL device according to the present disclosure may include an anode, a cathode, and one or more organic layers disposed between the anode and the cathode, and at least one of the one or more organic layers may include the compound represented by Chemical Formula 1. In such a case, the compound may be used alone or in combination with two or more types thereof.

The one or more organic layers may include one or more of a hole injection layer, a hole transport layer, a light emitting layer, a hole blocking layer, an electron transport layer, and an electron injection layer, and at least one of the organic layers may include the compound represented by Chemical Formula 1. Preferably, the organic layer including the compound represented by Chemical Formula 1 may be a hole transport layer.

In an example, the one or more organic layer may include a hole injection layer, a hole transport layer, a light emitting layer, an electron transport layer, and an electron injection layer, and the hole transport layer may include the compound represented by Chemical Formula 1.

The compound represented by the above Chemical Formula 1 may be included in the organic EL device as a hole transport layer material. In such a case, the compound of the above Chemical Formula 1 has a high glass transition temperature and high hole mobility, thereby having high hole transport ability, and due to appropriate HOMO and LUMO energy levels between the hole injection layer and the light emitting layer, hole injection and transfer from the hole injection layer to the light emitting layer may be smooth, and it has amorphous crystallinity and high refractive index characteristics. Accordingly, the organic EL device including the compound of the above Chemical Formula 1 may have improved efficiency (luminescence efficiency and power efficiency), service life, brightness, driving voltage, thermal stability, etc.

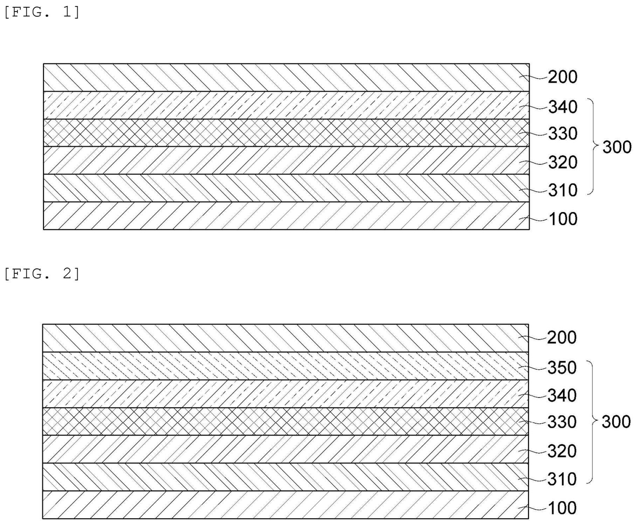

The structure of the organic EL device of the present disclosure is not particularly limited, and for example, may include an anode 100, one or more organic layers 300, and a cathode 200 which may be sequentially stacked, for example, on a substrate (see FIGS. 1 and 2). In addition, it may have a structure in which an insulating layer or an adhesive layer is inserted at an interface between the electrode and the organic layer.

In an example, the organic EL device may have a structure in which the anode 100, a hole injection layer 310, a hole transport layer 320, a light emitting layer 330, an electron transport layer 340, and the cathode 200 are sequentially laminated, for example, on a substrate, as illustrated in FIG. 1. Optionally, as illustrated in FIG. 2, an electron injection layer 350 may be positioned between the electron transport layer 340 and the cathode 200. In addition, a hole blocking layer (not illustrated) may be positioned between the light emitting layer 330 and the electron transport layer 340. The organic EL device of the present disclosure may be fabricated by forming organic layers and electrodes using materials and methods known in the art, except that at least one of the organic layers 300 [e.g., the hole transport layer 320] includes the compound represented by Chemical Formula 1.

The above organic layer may be formed by vacuum deposition or solution coating. Examples of the solution coating may include, but are not limited to, spin coating, dip coating, doctor blading, inkjet printing, or thermal transfer.

The substrate applicable in the present invention is not particularly limited, and non-limiting examples may include silicon wafers, quartz, glass plates, metal plates, plastic films, sheets, and the like.

In addition, examples of the anode material may include, but are not limited to, a metal such as vanadium, chromium, copper, zinc, and gold or an alloy thereof; metal oxides such as zinc oxide, indium oxide, indium tin oxide (ITO), or indium zinc oxide (IZO); combination of oxide with metal such as ZnO:Al or SnO₂:Sb; conductive polymers such as polythiophene, poly (3-methylthiophene), poly [3,4-(ethylene-1,2-dioxy) thiophene] (PEDT), polypyrrole or polyaniline; carbon black or the like.

In addition, examples of the cathode material may include, but are not limited to, a metal such as magnesium, calcium, sodium, potassium, titanium, indium, yttrium, lithium, gadolinium, aluminum, silver, tin, or lead or an alloy thereof; a multi-layered material such as LiF/Al or LiO₂/Al or the like.

In addition, the hole injection layer, the light emitting layer, the electron transport layer, and the electron injection layer are not particularly limited and conventional materials known in the art may be used without limitation.

Hereinafter, the present disclosure will be described in detail with reference to the following examples. However, the following examples are merely to illustrate the disclosure, and the scope of the present disclosure is not limited to the following embodiments.

### [Preparation Example 1] Synthesis of compound J-1

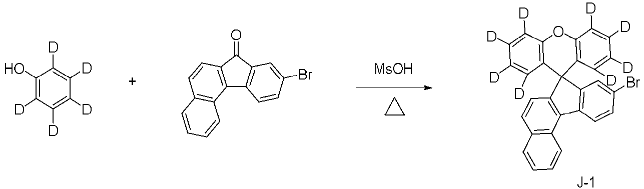

After adding methanesulfonic acid (MsOH) (3.84 g, 40 mmol) to phen-d5-ol (9.91 g, 100 mmol) and 9-bromo-7H-benzo[c]fluoren-7-one (3.09 g, 10 mmol), the mixture was heated to reflux at a temperature of 120°C for 12 hours. Subsequently, the temperature of the reaction solution obtained by the heating and refluxing was cooled to room temperature, and distilled water was added to the reaction solution to terminate the reaction. After completion of the reaction, the resulting mixture was extracted with CH₂Cl₂ to separate an organic layer, and then, the separated organic layer was neutralized with saturated calcium carbonate and washed with distilled water. After that, the washed organic layer was dried over anhydrous MgSO₄, distilled under reduced pressure, and purified by silica gel column chromatography to obtain a target compound J-1 (2.34 g, yield 50%).

### [Preparation Example 2] Synthesis of Compound J-2

### <Step 1> Synthesis of 2-bromo-11H-benzo[b]fluoren-11-one-5,6,7,8,9,10-d6

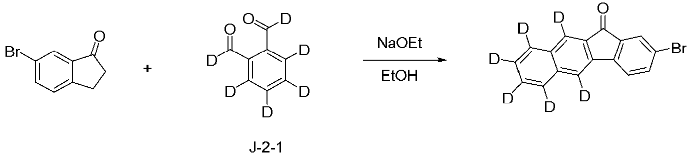

Under a nitrogen atmosphere, 6-bromo-2,3-dihydro-1H-inden-1-one (2.11 g, 10 mmol), compound J-2-1 (1.40 g, 10 mmol), and NaOEt (1.36 g, 20 mmol) were added to 25 mL of ethyl alcohol, and the resulting mixture was refluxed for 6 hours. The reaction solution was cooled to 0°C to precipitate a solid, and the precipitated solid was filtered, washed with methyl alcohol, and filtered under reduced pressure to obtain a compound 2-bromo-11H-benzo[b]fluoren-11-one-5,6,7,8,9,10-d6 (1.54 g, yield 49%).

### <Step 2> Synthesis of compound J-2

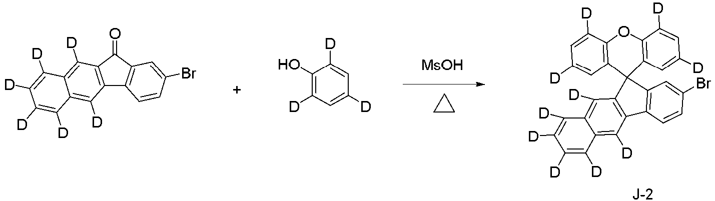

After adding methanesulfonic acid (MsOH) (3.84 g, 40 mmol) to phen-2,4,6-d3-ol (9.71 g, 100 mmol) and 2-bromo-11H-benzo[b]fluoren-11-one-5,6,7,8,9,10-d6 (3.15 g, 10 mmol), the mixture was heated to reflux at a temperature of 120°C for 12 hours. Subsequently, the temperature of the reaction solution obtained by the heating and refluxing was cooled to room temperature, and distilled water was added to the reaction solution to terminate the reaction. After completion of the reaction, the resulting mixture was extracted with CH₂Cl₂ to separate an organic layer, and then, the separated organic layer was neutralized with saturated calcium carbonate and washed with distilled water. After that, the washed organic layer was dried over anhydrous MgSO₄, distilled under reduced pressure, and purified by silica gel column chromatography to obtain a target compound J-2 (2.26 g, yield 48%) .

### [Preparation Example 3] Synthesis of Compound J-3

### <Step 1> Synthesis of 9-bromo-11H-benzo[a]fluoren-11-one-7,8,10-d3

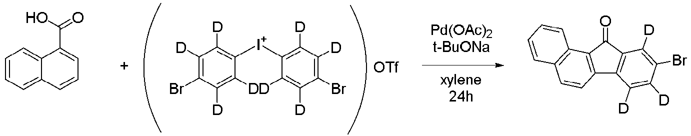

1-naphthoic acid (1.72 g, 10 mmol), diaryliodonium Salt (11.92 g, 20 mmol), Pd(OAc)₂ (0.02 g, 1 mmol), and t-BuONa (0.96 g, 10 mmol) were added to 50 ml of xylene and stirred at 110°C for 24 hours. After completion of the reaction, a solvent was removed by distillation under reduced pressure, and then column chromatography was used to obtain a target compound 9-bromo-11H-benzo[a]fluoren-11-one-7,8,10-d3 (1.46 g, yield 47%).

### <Step 2> Synthesis of compound J-3

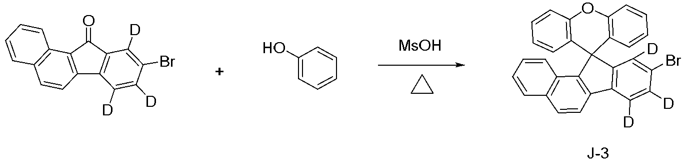

After adding methanesulfonic acid (MsOH) (3.84 g, 40 mmol) to phenol (9.41 g, 100 mmol) and 9-bromo-11H-benzo[a]fluoren-11-one-7,8,10-d3 (3.12 g, 10 mmol), the mixture was heated to reflux at a temperature of 120°C for 12 hours. Subsequently, the temperature of the reaction solution obtained by the heating and refluxing was cooled to room temperature, and distilled water was added to the reaction solution to terminate the reaction. After completion of the reaction, the resulting mixture was extracted with CH₂Cl₂ to separate an organic layer, and then, the separated organic layer was neutralized with saturated calcium carbonate and washed with distilled water. After that, the washed organic layer was dried over anhydrous MgSO₄, distilled under reduced pressure, and purified by silica gel column chromatography to obtain a target compound J-3 (2.13 g, yield 46%).

### [Preparation Example 4] Synthesis of Compound J-4

### <Step 1> Synthesis of methyl 2-bromo-6-(naphthalen-1-yl)benzoate-3,4,5-d3

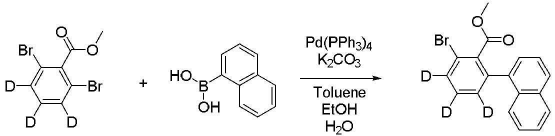

Methyl 2,6-dibromobenzoate-3,4,5-d3 (2.96 g, 10 mmol), naphthalen-1-ylboronic acid (1.71 g, 10 mmol), Pd(PPh₃)₄ (0.34 g, 0.3 mmol), and K₂CO₃ (2.76 g, 20 mmol) were added to 100 ml of toluene/25 ml of ethanol/25 ml of H₂O and stirred at 100°C for 8 hours. After completion of the reaction, extraction was performed with methylene chloride, MgSO₄ was added thereto, and filtering was performed. After a solvent was removed from the filtered organic layer, column chromatography was used to obtain a target compound methyl 2-bromo-6-(naphthalen-1-yl)benzoate-3,4,5-d3 (1.54 g, Yield 45 %).

### <Step 2> Synthesis of 2-bromo-6-(naphthalen-1-yl)benzoic-3,4,5-d3 acid

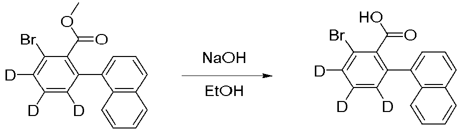

Methyl 2-bromo-6-(naphthalen-1-yl)benzoate-3,4,5-d3 (3.44 g, 10 mmol) and sodium hydroxide (0.40 g, 10 mmol) were added to 50 mL of ethanol and refluxed for 6 hours. After lowering the temperature to room temperature and acidifying with 2 M hydrochloric acid, a precipitate was filtered and recrystallized with ethanol to obtain a target compound 2-bromo-6-(naphthalen-1-yl)benzoic-3,4,5-d3 acid (1.45 g, 44%).

### <Step 3> Synthesis of 8-bromo-7H-benzo[c]fluoren-7-one-9,10,11-d3

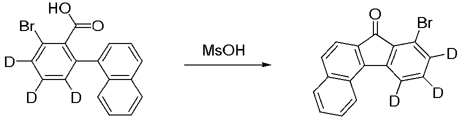

2-bromo-6- (naphthalen-1-yl)benzoic-3,4,5-d3 acid (3.₃₀ g, 10 mmol) was dissolved in 100 mL of methanesulfonic acid and stirred for 24 hours, and then ice water was poured over it to precipitate a solid. The precipitated solid was filtered, washed with distilled water, and then sodium bicarbonate aqueous solution was added thereto and stirred for 3 hours. After filtration, followed by neutralization with distilled water and recrystallization with acetic acid, a target compound 8-bromo-7H-benzo[c]fluoren-7-one-9,10,11-d3 (1.34 g, 43%) was obtained.

### <Step 4> Synthesis of compound J-4

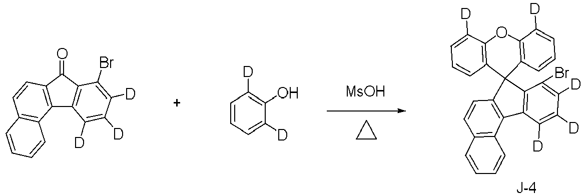

After adding methanesulfonic acid (MsOH) (3.84 g, 40 mmol) to phen-2,6-d2-ol (9.61 g, 100 mmol) and 8-bromo-7H-benzo[c]fluoren-7-one-9,10,11-d3 (3.12 g, 10 mmol), the mixture was heated to reflux at 120°C for 12 hours. Subsequently, the temperature of the reaction solution obtained by the heating and refluxing was cooled to room temperature, and distilled water was added to the reaction solution to terminate the reaction. After completion of the reaction, the resulting mixture was extracted with CH₂Cl₂ to separate an organic layer, and then, the separated organic layer was neutralized with saturated calcium carbonate and washed with distilled water. After that, the washed organic layer was dried over anhydrous MgSO₄, distilled under reduced pressure, and purified by silica gel column chromatography to obtain a target compound J-4 (1.95 g, Yield 42 %).

### [Preparation Example 5] Synthesis of Compound J-5

### <Step 1> Synthesis of methyl 4-chloro-2-(naphthalen-1-yl-d7)benzoate

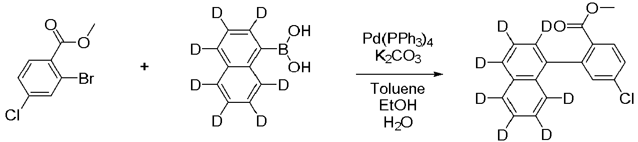

Methyl 2-bromo-4-chlorobenzoate (2.49 g, 10 mmol), (naphthalen-1-yl-d7)boronic acid (1.79 g, 10 mmol), Pd(PPh₃)₄ (0.34 g, 0.3 mmol), and K₂CO₃ (2.76 g, 20 mmol) were added to 100 ml of toluene/25 ml of ethanol/25 ml of H₂O and stirred at 100°C for 8 hours. After completion of the reaction, extraction was performed with methylene chloride, MgSO₄ was added thereto, and filtering was performed. After removing a solvent from the filtered organic layer, column chromatography was used to obtain a target compound methyl 4-chloro-2-(naphthalen-1-yl-d7)benzoate (1.24 g, Yield 41 %).

### <Step 2> Synthesis of 4-chloro-2-(naphthalen-1-yl-d7)benzoic acid

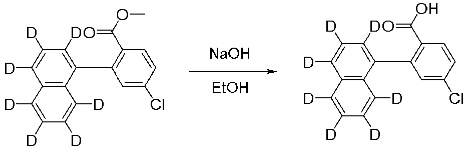

Methyl 4-chloro-2-(naphthalen-1-yl-d7)benzoate (3.03 g, 10 mmol) and sodium hydroxide (0.40 g, 10 mmol) were added to 50 mL of ethanol and refluxed for 6 hours. The temperature was lowered to room temperature, acidified with 2 M hydrochloric acid, and a precipitate was filtered and recrystallized with ethanol to obtain a compound 4-chloro-2-(naphthalen-1-yl-d7)benzoic acid (1.15 g, 40%).

### <Step 3> Synthesis of 10-chloro-7H-benzo[c]fluoren-7-one-1,2,3,4,5,6-d6

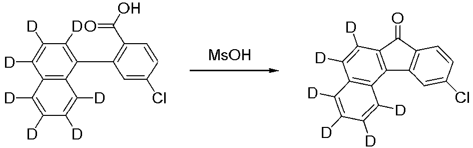

4-chloro-2-(naphthalen-1-yl-d7)benzoic acid (2.89 g, 10 mmol) was dissolved in 100 mL of methanesulfonic acid and stirred for 24 hours, and then ice water was poured over it to precipitate it. The precipitated solid was filtered, washed with distilled water, and then an aqueous sodium bicarbonate solution was added thereto and stirred for 3 hours. After filtration, followed by neutralization with distilled water and recrystallization with acetic acid, a target compound 10-chloro-7H-benzo[c]fluoren-7-one-1,2,3,4,5,6-d6 (1.11 g, 41%) was obtained.

### <Step 4> Synthesis of compound J-5

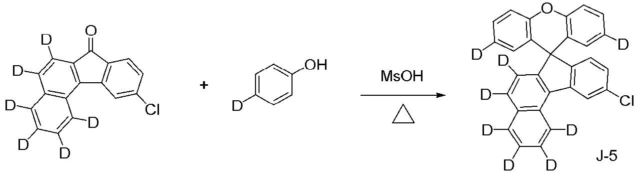

After adding methanesulfonic acid (MsOH) (3.84 g, 40 mmol) to phen-4-d-ol (9.51 g, 100 mmol) and 10-chloro-7H-benzo[c]fluoren-7-one-1,2,3,4,5,6-d6 (2.70 g, 10 mmol), the mixture was heated to reflux at 120°C for 12 hours. Subsequently, the temperature of the reaction solution obtained by the heating and refluxing was cooled to room temperature, and distilled water was added to the reaction solution to terminate the reaction. After completion of the reaction, the resulting mixture was extracted with CH₂Cl₂ to separate an organic layer, and then, the separated organic layer was neutralized with saturated calcium carbonate and washed with distilled water. After that, the washed organic layer was dried over anhydrous MgSO₄, distilled under reduced pressure, and purified by silica gel column chromatography to obtain a target compound J-5 (1.78 g, Yield 42 %).

### [Preparation Example 6] Synthesis of compound J-6

### <Step 1> Synthesis of methyl 3-bromo-2-(naphthalen-1-yl-d7)benzoate

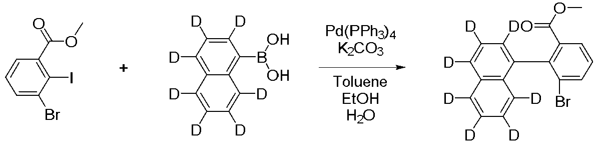

Methyl 3-bromo-2-iodobenzoate (3.40 g, 10 mmol), (naphthalen-1-yl-d7)boronic acid (1.79 g, 10 mmol), Pd(PPh₃)₄ (0.34 g, 0.3 mmol), and K₂CO₃ (2.76 g, 20 mmol) were added to 100 ml of toluene/25 ml of ethanol/25 ml of H₂O and stirred at 100°C for 8 hours. After completion of the reaction, extraction was performed with methylene chloride, MgSO₄ was added thereto, and filtering was performed. After removing a solvent from the filtered organic layer, column chromatography was used to obtain a target compound methyl 3-bromo-2-(naphthalen-1-yl-d7)benzoate (1.49 g, Yield 43 %).

### <Step 2> Synthesis of 3-bromo-2-(naphthalen-1-yl-d7)benzoic acid

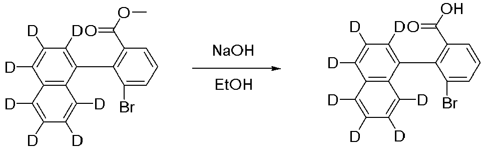

Methyl 3-bromo-2-(naphthalen-1-yl-d7)benzoate (3.48 g, 10 mmol) and sodium hydroxide (0.40 g, 10 mmol) were added to 50 mL of ethanol and refluxed for 6 hours. The temperature was lowered to room temperature, acidified with 2 M hydrochloric acid, and a precipitate was filtered and recrystallized with ethanol to obtain a compound 3-bromo-2-(naphthalen-1-yl-d7)benzoic acid (1.47 g, 44%).

### <Step 3> Synthesis of 11-bromo-7H-benzo[c]fluoren-7-one-1,2,3,4,5,6-d6

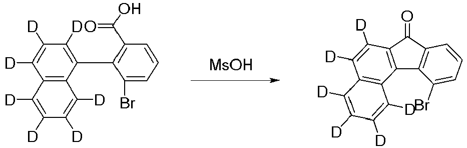

3-bromo-2-(naphthalen-1-yl-d7)benzoic acid (3.34 g, 10 mmol) was dissolved in 100 mL of methanesulfonic acid and stirred for 24 hours, and then ice water was poured over it to precipitate it. The precipitated solid was filtered, washed with distilled water, and then a sodium bicarbonate aqueous solution was added thereto and stirred for 3 hours. After filtration, followed by neutralization with distilled water and recrystallization with acetic acid, a compound 11-bromo-7H-benzo[c]fluoren-7-one-1,2,3,4,5,6-d6 (1.41 g, 45%) was obtained.

### <Step 4> Synthesis of Compound J-6

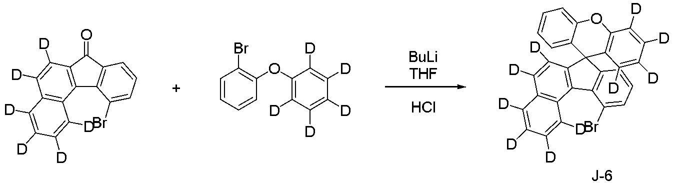

In a baked-out flask, 1-(2-bromophenoxy)benzene-2,3,4,5,6-d5 (2.54 g, 10 mmol) was dissolved in 40 mL of anhydrous THF and the reaction mixture was cooled to -78°C. A solution of n-BuLi (10 mmol) was slowly added dropwise and stirred for 1 hour. Then, 11-bromo-7H-benzo[c]fluoren-7-one-1,2,3,4,5,6-d6 (3.15 g, 10 mmol) was dissolved in 20 mL of THF and added dropwise at -78°C. The temperature of the reaction mixture was slowly raised to room temperature, and after reaction with NH₄Cl was completed, the resultant solution was concentrated under reduced pressure. The concentrated solution was carefully mixed with 30 mL of acetic acid and then 5 mL of fuming HCl was added. After stirring the mixture at 75°C for 6 hours, the mixture was cooled to room temperature, a precipitated solid was filtered under reduced pressure and washed with methanol to obtain a compound J-6 (2.16 g, yield 46%).

### [Preparation Example 7] Synthesis of compound J-7

### <Step 1> Synthesis of methyl 5-bromo-2-(naphthalen-1-yl-d7)benzoate

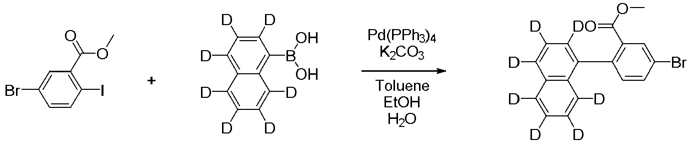

Methyl 5-bromo-2-iodobenzoate (3.40 g, 10 mmol), (naphthalen-1-yl-d7)boronic acid (1.79 g, 10 mmol), Pd(PPh₃)₄ (0.34 g, 0.3 mmol), and K₂CO₃ (2.76 g, 20 mmol) were added to 100 ml of toluene/25 ml of ethanol/25 ml of H₂O and stirred at 100°C for 8 hours. After the reaction was completed, extraction was performed with methylene chloride, MgSO₄ was added, and the mixture was filtered. After removing a solvent from the filtered organic layer, column chromatography was used to obtain a target compound methyl 5-bromo-2-(naphthalen-1-yl-d7)benzoate (1.63 g, Yield 47 %).

### <Step 2> Synthesis of 5-bromo-2-(naphthalen-1-yl-d7)benzoic acid

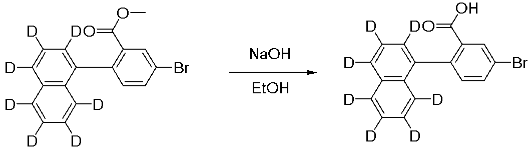

Methyl 5-bromo-2-(naphthalen-1-yl-d7)benzoate (3.48 g, 10 mmol) and sodium hydroxide (0.40 g, 10 mmol) were added to 50 mL of ethanol and refluxed for 6 hours. After lowering the temperature to room temperature and acidifying with 2 M hydrochloric acid, a precipitate was filtered and recrystallized with ethanol to obtain a target compound 5-bromo-2-(naphthalen-1-yl-d7)benzoic acid (1.60 g, 48%).

### <Step 3> Synthesis of 9-bromo-7H-benzo[c]fluoren-7-one-1,2,3,4,5,6-d6

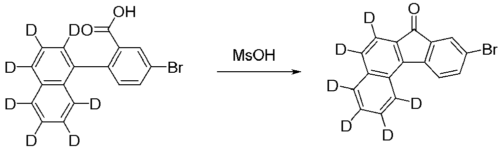

5-bromo-2-(naphthalen-1-yl-d7)benzoic acid (3.34 g, 10 mmol) was dissolved in 100 mL of methanesulfonic acid and stirred for 24 hours, and then ice water was poured over it to precipitate a solid. After the precipitated solid was filtered and washed with distilled water, a sodium bicarbonate aqueous solution was added thereto, and the mixture was stirred for 3 hours. After filtration, followed by neutralization with distilled water and recrystallization with acetic acid, a compound 9-bromo-7H-benzo[c]fluoren-7-one-1,2,3,4,5,6-d6 (1.54 g, 49%) was obtained.

### <Step 4> Synthesis of compound J-7

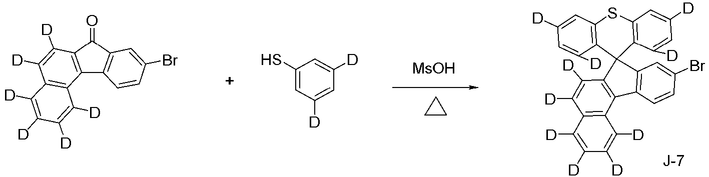

After adding methanesulfonic acid (MsOH) (3.84 g, 40 mmol) to benzene-3,5-d2-thiol (11.21 g, 100 mmol) and 9-bromo-7H-benzo[c]fluoren-7-one-1,2,3,4,5,6-d6 (3.15 g, 10 mmol), the mixture was heated to reflux at 120°C for 12 hours. Subsequently, the temperature of the reaction solution obtained by the heating and refluxing was cooled to room temperature, and distilled water was added to the reaction solution to terminate the reaction. After completion of the reaction, the resulting mixture was extracted with CH₂Cl₂ to separate an organic layer, and then, the separated organic layer was neutralized with saturated calcium carbonate and washed with distilled water. After that, the washed organic layer was dried over anhydrous MgSO₄, distilled under reduced pressure, and purified by silica gel column chromatography to obtain a target compound J-7 (2.43 g, Yield 50 %).

### [Preparation Example 8] Synthesis of compound J-8

### <Step 1> Synthesis of 3-bromo-11H-benzo[b]fluoren-11-one-5,10-d2

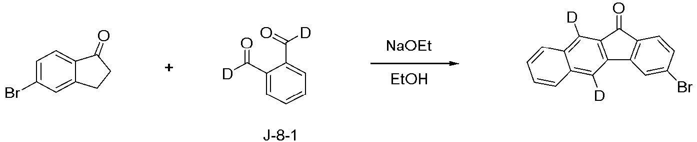

Under a nitrogen atmosphere, 5-bromo-2,3-dihydro-1H-inden-1-one (2.11 g, 10 mmol), J-8-1 (1.36 g, 10 mmol), and NaOEt (1.36 g, 20 mmol) were added to 25 mL of ethyl alcohol, and the resulting mixture was refluxed for 6 hours. The reaction solution was cooled to 0°C to precipitate a solid, and the precipitated solid was filtered, washed with methyl alcohol, and filtered under reduced pressure to obtain a compound 3-bromo-11H-benzo[b]fluoren-11-one-5,10-d2 (1.52 g, Yield 49 %).

### <Step 2> Synthesis of compound J-8

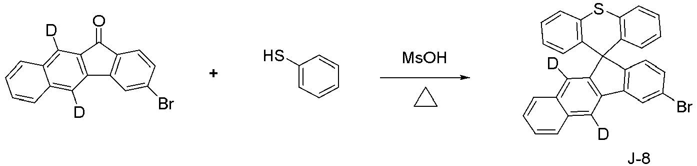

After adding methanesulfonic acid (MsOH) (3.84 g, 40 mmol) to benzenethiol (11.01 g, 100 mmol) and 3-bromo-11H-benzo[b]fluoren-11-one-5,10-d2 (3.11 g, 10 mmol), the mixture was heated to reflux at 120°C for 12 hours. Subsequently, the temperature of the reaction solution obtained by the heating and refluxing was cooled to room temperature, and distilled water was added to the reaction solution to terminate the reaction. After completion of the reaction, the resulting mixture was extracted with CH₂Cl₂ to separate an organic layer, and then, the separated organic layer was neutralized with saturated calcium carbonate and washed with distilled water. After that, the washed organic layer was dried over anhydrous MgSO₄, distilled under reduced pressure, and purified by silica gel column chromatography to obtain a target compound J-8 (2.30 g, Yield 48 %).

### [Preparation Example 9] Synthesis of Compound J-9

### <Step 1> Synthesis of 7-bromo-11H-benzo[a]fluoren-11-one-1-d

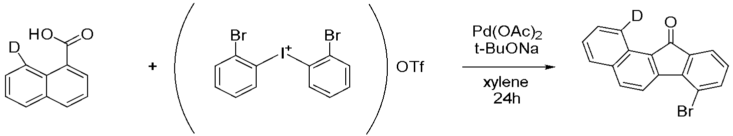

1-naphthoic-8-d acid (1.73 g, 10 mmol), Diaryliodonium Salt (11.75 g, 20 mmol), Pd(OAc)₂ (0.02 g, 1 mmol), and t-BuONa (0.96 g, 10 mmol) were added to 50 ml of xylene and stirred at 110°C for 24 hours. After completion of the reaction, a solvent was removed by distillation under reduced pressure, and then column chromatography was used to obtain a target compound 7-bromo-11H-benzo[a]fluoren-11-one-1-d (1.45 g, Yield 47 %).

### <Step 2> Synthesis of compound J-9

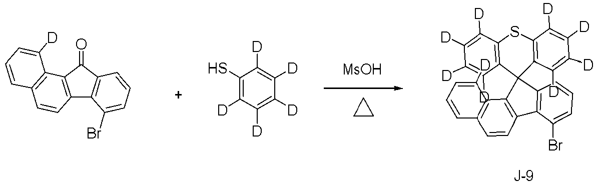

After adding methanesulfonic acid (MsOH) (3.84 g, 40 mmol) to benzene-d5-thiol (11.52 g, 100 mmol) and 7-bromo-11H-benzo[a]fluoren-11-one-1-d (3.10 g, 10 mmol), the mixture was heated to reflux at 120°C for 12 hours. Subsequently, the temperature of the reaction solution obtained by the heating and refluxing was cooled to room temperature, and distilled water was added to the reaction solution to terminate the reaction. After completion of the reaction, the resulting mixture was extracted with CH₂Cl₂ to separate an organic layer, and then, the separated organic layer was neutralized with saturated calcium carbonate and washed with distilled water. After that, the washed organic layer was dried over anhydrous MgSO₄, distilled under reduced pressure, and purified by silica gel column chromatography to obtain a target compound J-9 (2.23 g, Yield 46 %).

### [Preparation Example 10] Synthesis of compound J-10

### <Step 1> Synthesis of methyl 2-bromo-6-(naphthalen-2-yl)benzoate-3,4,5-d3

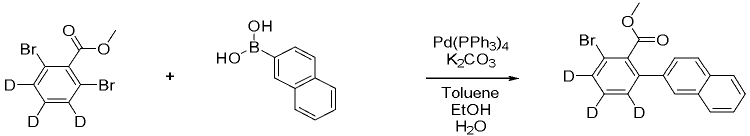

Methyl 2,6-dibromobenzoate-3,4,5-d3 (2.96 g, 10 mmol), naphthalen-2-ylboronic acid (1.71 g, 10 mmol), Pd(PPh₃)₄ (0.34 g, 0.3 mmol) and K₂CO₃ (2.76 g, 20 mmol) were added to 100 ml of toluene/25 ml of ethanol/25 ml of H₂O and stirred at 100°C for 8 hours. After completion of the reaction, extraction was performed with methylene chloride, MgSO₄ was added thereto, and filtering was performed. After removing a solvent from the filtered organic layer, column chromatography was used to obtain a target compound methyl 2-bromo-6-(naphthalen-2-yl)benzoate-3,4,5-d3 (1.54 g, Yield 45 %).

### <Step 2> Synthesis of 2-bromo-6-(naphthalen-2-yl)benzoic-3,4,5-d3 acid

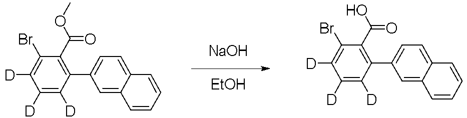

Methyl 2-bromo-6-(naphthalen-2-yl)benzoate-3,4,5-d3 (3.44 g, 10 mmol) and sodium hydroxide (0.40 g, 10 mmol) were added to 50 mL of ethanol, and the mixture was refluxed for 6 hours. After lowering the temperature to room temperature and acidifying with 2 M hydrochloric acid, a precipitate was filtered and recrystallized with ethanol to obtain a target compound 2-bromo-6-(naphthalen-2-yl)benzoic-3,4,5-d3 acid (1.45 g, 44%).

### <Step 3> Synthesis of 1-bromo-11H-benzo[b]fluoren-11-one-2,3,4-d3

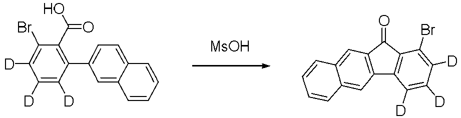

2-bromo-6-(naphthalen-2-yl)benzoic-3,4,5-d3 acid (3.30 g, 10 mmol) was dissolved in 100 mL of methanesulfonic acid and stirred for 24 hours, and then ice water was poured over it to precipitate a solid. The precipitated solid was filtered and washed with distilled water, a sodium bicarbonate aqueous solution was added thereto, and the mixture was stirred for 3 hours. After filtration, followed by neutralization with distilled water and recrystallization with acetic acid, a target compound 1-bromo-11H-benzo[b]fluoren-11-one-2,3,4-d3 (1.34 g, 43%) was obtained.

### <Step 4> Synthesis of compound J-10

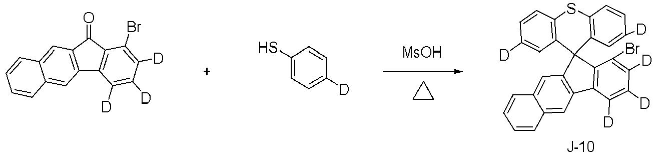

After adding methanesulfonic acid (MsOH) (3.84 g, 40 mmol) to benzene-4-d-thiol (11.11 g, 100 mmol) and 1-bromo-11H-benzo[b]fluoren-11-one-2,3,4-d3 (3.12 g, 10 mmol), the mixture was heated to reflux at 120°C for 12 hours. Subsequently, the temperature of the reaction solution obtained by the heating and refluxing was cooled to room temperature, and distilled water was added to the reaction solution to terminate the reaction. After completion of the reaction, the resulting mixture was extracted with CH₂Cl₂ to separate an organic layer, and then, the separated organic layer was neutralized with saturated calcium carbonate and washed with distilled water. After that, the washed organic layer was dried over anhydrous MgSO₄, distilled under reduced pressure, and purified by silica gel column chromatography to obtain a target compound J-10 (2.02 g, Yield 42 %).

### [Preparation Example 11] Synthesis of compound J-11

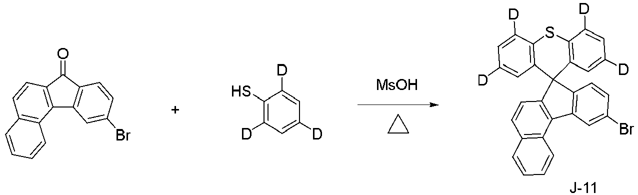

After adding methanesulfonic acid (MsOH) (3.84 g, 40 mmol) to benzene-2,4,6-d3-thiol (11.31 g, 100 mmol) and 10-bromo-7H-benzo[c]fluoren-7-one (3.09 g, 10 mmol), the mixture was heated to reflux at 120°C for 12 hours. Subsequently, the temperature of the reaction solution obtained by the heating and refluxing was cooled to room temperature, and distilled water was added to the reaction solution to terminate the reaction. After completion of the reaction, the resulting mixture was extracted with CH₂Cl₂ to separate an organic layer, and then, the separated organic layer was neutralized with saturated calcium carbonate and washed with distilled water. After that, the washed organic layer was dried over anhydrous MgSO₄, distilled under reduced pressure, and purified by silica gel column chromatography to obtain a target compound J-11 (1.97 g, Yield 41 %).

### [Preparation Example 12] Synthesis of compound J-12

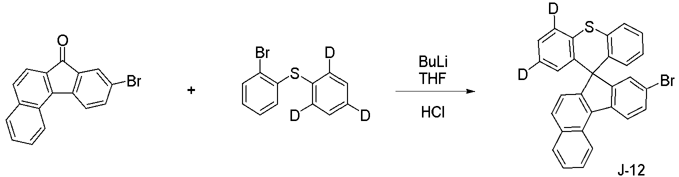

In a baked-out flask, (2-bromophenyl) (phenyl-2,4,6-d3)sulfane (2.68 g, 10 mmol) was dissolved in 40 mL of anhydrous THF and the reaction mixture was cooled to -78°C. A solution of n-BuLi (10 mmol) was slowly added dropwise and stirred for 1 hour. Then, 9-bromo-7H-benzo[c]fluoren-7-one (3.09 g, 10 mmol) was dissolved in 20 mL of THF and added dropwise at -78°C. The temperature of the reaction mixture was slowly raised to room temperature, and after reaction with NH₄Cl was completed, the resultant solution was concentrated under reduced pressure. The concentrated solution was carefully mixed with 30 mL of acetic acid and then 5 mL of fuming HCl was added thereto and stirred at 75°C for 6 hours. Subsequently, the mixture was cooled to room temperature, a precipitated solid was filtered under reduced pressure and washed with methanol to obtain a compound J-12 (1.91 g, Yield 40 %).

### [Preparation Example 13] Synthesis of Compound J-13

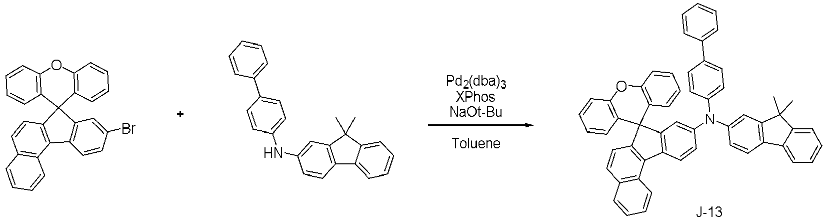

After adding 9-bromospiro[benzo[c]fluorene-7,9'-xanthene] (4.61 g, 10 mmol), N-([1,1'-biphenyl]-4-yl)-9,9-dimethyl-9H-fluoren-2-amine (3.61 g, 10 mmol), Pd2(dba)3 (0.91 g, 1 mmol), X-Phos (0.95 g, 2 mmol) and NaOt-Bu(1.92 g, 20 mmol) to 100 ml of toluene, the mixture was stirred at 120°C for 6 hours. After completion of the reaction, the mixture was extracted with methylene chloride, MgSO₄ was added thereto, and filtering was performed. After removing a solvent from the filtered organic layer, column chromatography was used to obtain a target compound J-13 (3.19 g, Yield 43 %).

### [Preparation Example 14] Synthesis of Compound J-14

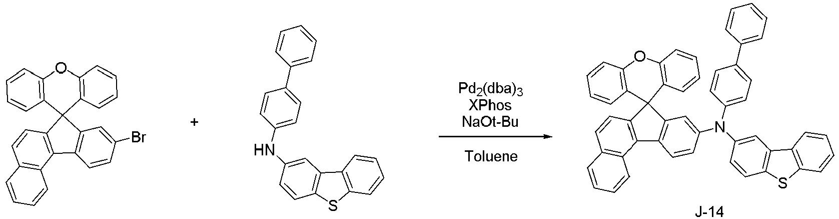

After adding 9-bromospiro[benzo[c]fluorene-7,9'-xanthene] (4.61 g, 10 mmol), N-([1,1'-biphenyl]-4-yl)dibenzo[b,d]thiophen-2-amine (3.51 g, 10 mmol), Pd₂(dba)₃ (0.91 g, 1 mmol), X-Phos (0.95 g, 2 mmol), and NaOt-Bu(1.92 g, 20 mmol) to 100 ml of toluene, the mixture was stirred at 120°C for 6 hours. After completion of the reaction, the mixture was extracted with methylene chloride, MgSO₄ was added thereto, and filtering was performed. After removing a solvent from the filtered organic layer, column chromatography was used to obtain a target compound J-14 (3.22 g, Yield 44 %).

### [Preparation Example 15] Synthesis of Compound J-15

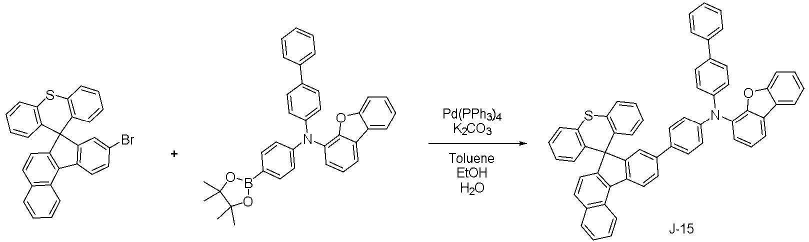

After adding 9-bromospiro[benzo[c]fluorene-7,9'-thioxanthene] (4.77 g, 10 mmol), N-([1,1'-biphenyl]-4-yl)-N-(4-(4,4,5,5-tetramethyl-1,3,2-dioxaborolan-2-yl)phenyl)dibenzo[b,d] furan-4-amine (5.37 g, 10 mmol), Pd(PPh₃)₄ (0.34 g, 0.3 mmol), and K₂CO₃ (2.76 g, 20 mmol) to 100 ml of toluene/25 ml of ethanol/25 ml of H₂, the mixture was stirred at 100°C for 8 hours. After completion of the reaction, the mixture was extracted with methylene chloride, MgSO₄ was added thereto, and filtering was performed. After removing a solvent from the filtered organic layer, column chromatography was used to obtain a target compound J-15 (3.63 g, Yield 45 %).

### [Synthetic Example 1] Synthesis of Compound 1

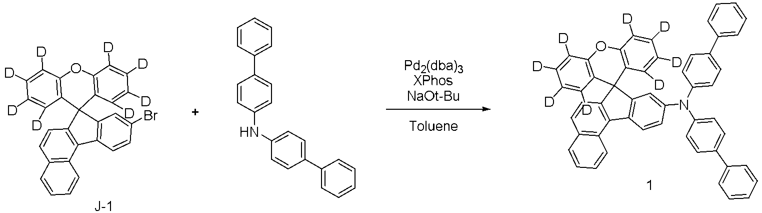

Compound J-1 (4.69 g, 10 mmol) synthesized in Preparation Example 1, di([1,1'-biphenyl]-4-yl)amine (3.21 g, 10 mmol), Pd₂(dba) ₃ (0.91 g, 1 mmol), X-Phos (0.95 g, 2 mmol), and NaOt-Bu(1.92 g, 20 mmol) were added to 100 ml of toluene, and the mixture was stirred at 120°C for 6 hours. After completion of the reaction, the mixture was extracted with methylene chloride, MgSO₄ was added thereto, and filtering was performed. After removing a solvent from the filtered organic layer, column chromatography was used to obtain a target compound J-15 (2.91 g, Yield 41 %).
Mass : [(M+H)⁺] : 709

### [Synthesis Example 2] Synthesis of Compound 2

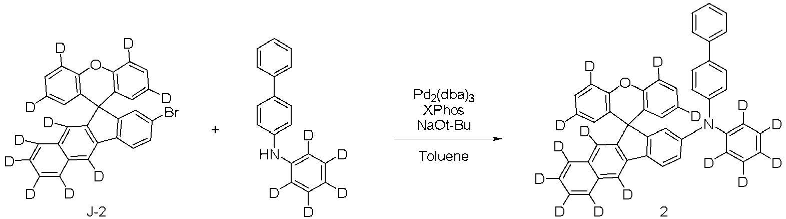

Compound J-2 (4.71 g, 10 mmol) synthesized in Preparation Example 2, N-(phenyl-d5)-[1,1'-biphenyl]-4-amine (2.50 g, 10 mmol), Pd₂(dba)₃ (0.91 g, 1 mmol), X-Phos (0.95 g, 2 mmol), and NaOt-Bu(1.92 g, 20 mmol) were added to 100 ml of toluene, and the mixture was stirred at 120°C for 6 hours. After completion of the reaction, the mixture was extracted with methylene chloride, MgSO₄ was added thereto, and filtering was performed. After removing a solvent from the filtered organic layer, column chromatography was used to obtain a target compound 2 (2.69 g, Yield 42 %).
Mass : [(M+H)⁺] : 640

### [Synthesis Example 3] Synthesis of Compound 3

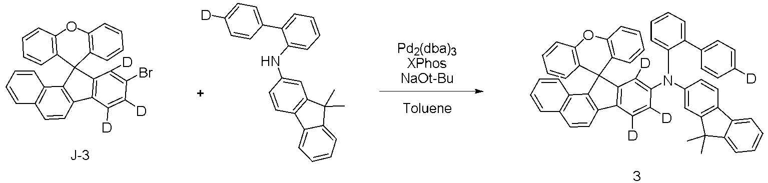

Compound J-3 (4.64 g, 10 mmol) synthesized in Preparation Example 3, N-([1,1'-biphenyl]-2-yl-4'-d)-9,9-dimethyl-9H-fluoren-2-amine (3.62 g, 10 mmol), Pd₂(dba)₃ (0.91 g, 1 mmol), X-Phos (0.95 g, 2 mmol), and NaOt-Bu(1.92 g, 20 mmol) were added to 100 ml of toluene, and the mixture was stirred at 120°C for 6 hours. After completion of the reaction, the mixture was extracted with methylene chloride, MgSO₄ was added thereto, and filtering was performed. After removing a solvent from the filtered organic layer, column chromatography was used to obtain a target compound 3 (3.20 g, Yield 43 %).
Mass : [(M+H)⁺] : 745

### [Synthetic Example 4] Synthesis of Compound 4

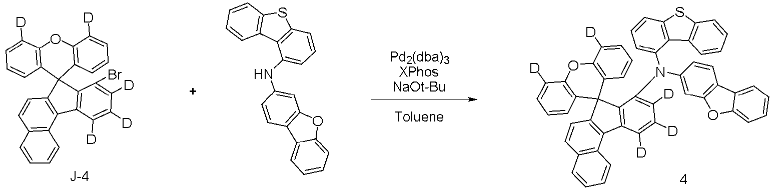

Compound J-4 (4.64 g, 10 mmol) synthesized in Preparation Example 4, N-(dibenzo[b,d]thiophen-1-yl)dibenzo[b,d]furan-3-amine (3.65 g, 10 mmol), Pd₂(dba)₃ (0.91 g, 1 mmol), X-Phos (0.95 g, 2 mmol), and NaOt-Bu(1.92 g, 20 mmol) were added to 100 ml of toluene, and the mixture was stirred at 120°C for 6 hours. After completion of the reaction, the mixture was extracted with methylene chloride, MgSO₄ was added thereto, and filtering was performed. After removing a solvent from the filtered organic layer, column chromatography was used to obtain a target compound 4 (3.30 g, Yield 44 %).
Mass : [(M+H)⁺] : 750

### [Synthesis Example 5] Synthesis of Compound 5

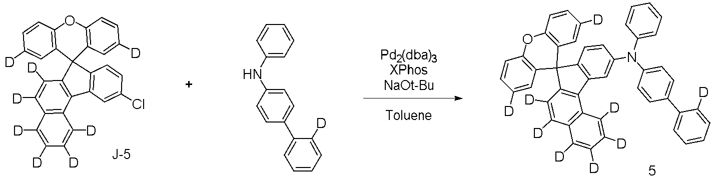

Compound J-5 (4.24 g, 10 mmol) synthesized in Preparation Example 5, N-phenyl-[1,1'-biphenyl]-2'-d-4-amine (2.46 g, 10 mmol), Pd₂(dba)₃ (0.91 g, 1 mmol), X-Phos (0.95 g, 2 mmol), and NaOt-Bu(1.92 g, 20 mmol) were added to 100 ml of toluene, and the mixture was stirred at 120°C for 6 hours. After completion of the reaction, the mixture was extracted with methylene chloride, MgSO₄ was added thereto, and filtering was performed. After removing a solvent from the filtered organic layer, column chromatography was used to obtain a target compound 5 (2.85 g, Yield 45 %).
Mass : [(M+H)⁺] : 634

### [Synthetic Example 6] Synthesis of Compound 6

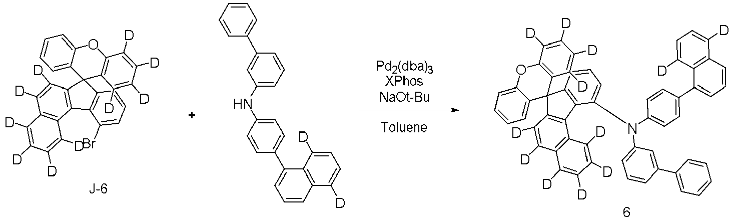

Compound J-6 (4.71 g, 10 mmol) synthesized in Preparation Example 6, N-(4-(naphthalen-1-yl-5,8-d2)phenyl)-[1,1'-biphenyl]-3-amine (3.73 g, 10 mmol), Pd₂(dba)₃ (0.91 g, 1 mmol), X-Phos (0.95 g, 2 mmol), and NaOt-Bu(1.92 g, 20 mmol) were added to 100 ml of toluene, and the mixture was stirred at 120°C for 6 hours. After completion of the reaction, the mixture was extracted with methylene chloride, MgSO₄ was added thereto, and filtering was performed. After removing a solvent from the filtered organic layer, column chromatography was used to obtain a target compound 6 (3.51 g, Yield 46 %).
Mass : [(M+H)⁺] : 764

### [Synthetic Example 7] Synthesis of Compound 7

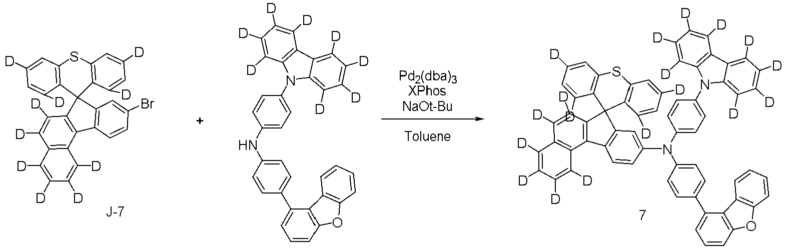

Compound J-7 (4.87 g, 10 mmol) synthesized in Preparation Example 7, N-(4-(9H-carbazol-9-yl-d8)phenyl)-4-(dibenzo[b,d]furan-1-yl)aniline (5.08 g, 10 mmol), Pd₂(dba)₃ (0.91 g, 1 mmol), X-Phos (0.95 g, 2 mmol), and NaOt-Bu(1.92 g, 20 mmol) were added to 100 ml of toluene, and the mixture was stirred at 120°C for 6 hours. After completion of the reaction, extraction was performed with methylene chloride, MgSO₄ was added thereto, and filtering was performed. After removing a solvent from the filtered organic layer, column chromatography was used to obtain a target compound 7 (4.30 g, Yield 47 %).
Mass : [(M+H)⁺] : 915

### [Synthetic Example 8] Synthesis of Compound 8

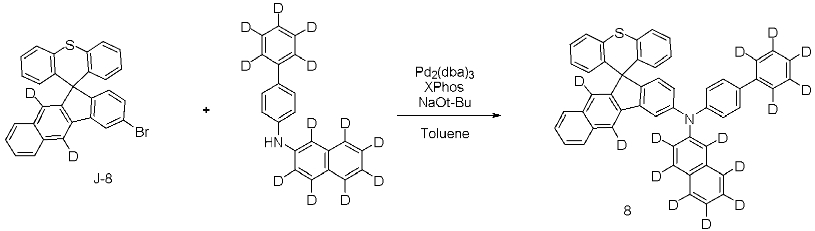

Compound J-8 (4.79 g, 10 mmol) synthesized in Preparation Example 8, N-([1,1'-biphenyl]-4-yl-2',3',4',5',6'-d5)naphthalen-d7-2-amine (3.07 g, 10 mmol), Pd₂(dba)₃ (0.91 g, 1 mmol), X-Phos (0.95 g, 2 mmol), and NaOt-Bu(1.92 g, 20 mmol) were added to 100 ml of toluene, and the mixture was stirred at 120°C for 6 hours. After completion of the reaction, the mixture was extracted with methylene chloride, MgSO₄ was added thereto, and filtering was performed. After removing a solvent from the filtered organic layer, column chromatography was used to obtain a target compound 8 (3.38 g, Yield 48 %).
Mass : [(M+H)⁺] : 705

### [Synthesis Example 9] Synthesis of Compound 9

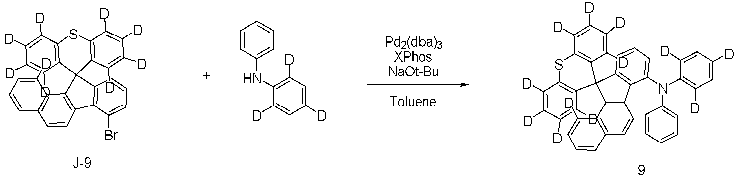

Compound J-9 (4.86 g, 10 mmol) synthesized in Preparation Example 9, N-phenylbenzen-2,4,6-d3-amine (1.72 g, 10 mmol), Pd₂(dba)₃ (0.91 g, 1 mmol), X-Phos (0.95 g, 2 mmol), and NaOt-Bu(1.92 g, 20 mmol) were added to 100 ml of toluene, and the mixture was stirred at 120°C for 6 hours. After completion of the reaction, the mixture was extracted with methylene chloride, MgSO₄ was added thereto, and filtering was performed. After removing a solvent from the filtered organic layer, column chromatography was used to obtain a target compound 9 (2.83 g, Yield 49 %).
Mass : [(M+H)⁺] : 577

### [Synthesis Example 10] Synthesis of Compound 10

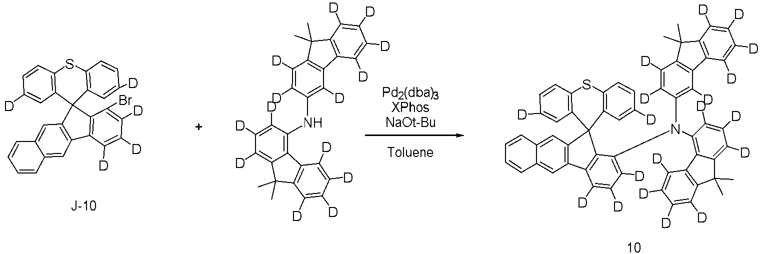

Compound J-10 (4.86 g, 10 mmol) synthesized in Preparation Example 10, N-(9,9-dimethyl-9H-fluoren-4-yl-1,2,3,5,6,7,8-d7)-9,9-dimethyl-9H-fluoren-1,2,4,5,6,7,8-d7-3-amine (4.15 g, 10 mmol), Pd₂(dba)₃ (0.91 g, 1 mmol), X-Phos (0.95 g, 2 mmol), and NaOt-Bu(1.92 g, 20 mmol) were added to 100 ml of toluene, and the mixture was stirred at 120°C for 6 hours. After completion of the reaction, the mixture was extracted with methylene chloride, MgSO₄ was added thereto, and filtering was performed. After removing a solvent from the filtered organic layer, column chromatography was used to obtain a target compound 10 (4.08 g, Yield 50 %).
Mass : [(M+H)⁺] : 817

### [Synthetic Example 11] Synthesis of Compound 11

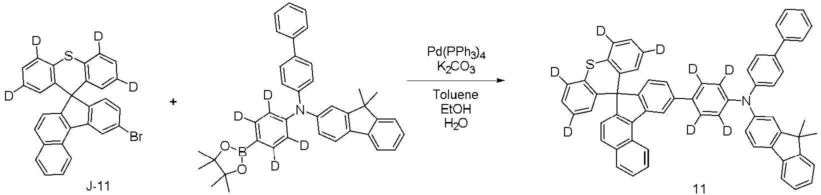

Compound J-11 (4.81 g, 10 mmol) synthesized in Preparation Example 11, N-([1,1'-biphenyl]-4-yl)-9,9-dimethyl-N-(4-(4,4,5,5-tetramethyl-1,3,2-dioxaborolan-2-yl)phenyl-2,3,5,6-d4)-9H-fluoren-2-amine (5.67 g, 10 mmol), Pd(PPh₃)₄ (0.34 g, 0.3 mmol), and K₂CO₃ (2.76 g, 20 mmol) were added to 100 ml of toluene/25 ml of Ethanol/25 ml of H₂O, and the mixture was stirred at 100°C for 8 hours. After completion of the reaction, the mixture was extracted with methylene chloride, MgSO₄ was added thereto, and filtering was performed. After removing a solvent from the filtered organic layer, column chromatography was used to obtain a target compound 11 (4.12 g, Yield 49 %).
Mass : [(M+H)⁺] : 842

### [Synthesis Example 12] Synthesis of Compound 12

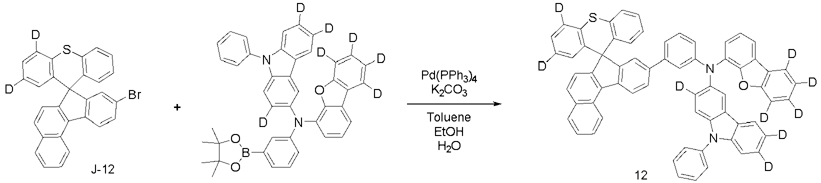

Compound J-12 (4.79 g, 10 mmol) synthesized in Preparation Example 12, N-(dibenzo[b,d]furan-4-yl-6,7,8,9-d4)-9-phenyl-N-(3-(4,4,5,5-tetramethyl-1,3,2-dioxaborolan-2-yl)phenyl)-9H-carbazol-3-amine-2,6,7-d3 (6.33 g, 10 mmol), Pd(PPh₃)₄ (0.34 g, 0.3 mmol), and K₂CO₃ (2.76 g, 20 mmol) were added to 100 ml of toluene/25 ml of Ethanol/25 ml of H₂O, and the mixture was stirred at 100°C for 8 hours. After completion of the reaction, the mixture was extracted with methylene chloride, MgSO₄ was added thereto, and filtering was performed. After removing a solvent from the filtered organic layer, column chromatography was used to obtain a target compound 12 (4.34 g, yield 48%).
Mass : [(M+H)⁺] : 906

### [Synthesis Example 13] Synthesis of Compound 13

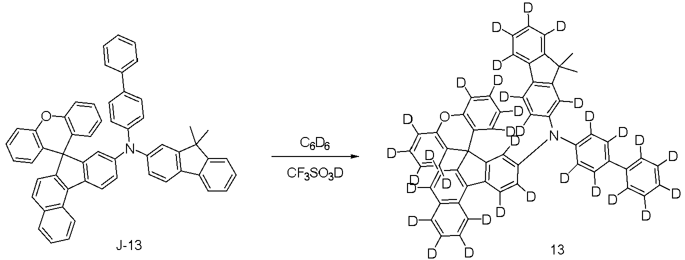

Compound J-13 (7.41 g, 10 mmol) synthesized in Preparation Example 13 was dissolved in perdeuterated benzene (C₆D₆) (84.15 g, 1 mol), CF₃SO₃D (7.55 g, 50 mmol) was added thereto, and the mixture was stirred at 80°C. A sample was taken to measure level of deuteration using LC-MS, and after completion of the exchange reaction, the temperature was lowered to room temperature. It was confirmed from measurements with LC-MS that deuterium substitution may be performed at various rates depending on conditions. The reaction was terminated by adding Na₂CO₃ in D₂O, and an organic solvent was concentrated. Recrystallization was performed using toluene and acetone solvents to obtain a deuterated target compound 13 (3.64 g, yield 47%) .
Mass : [(M+H)⁺] : 775

### [Synthesis Example 14] Synthesis of Compound 14

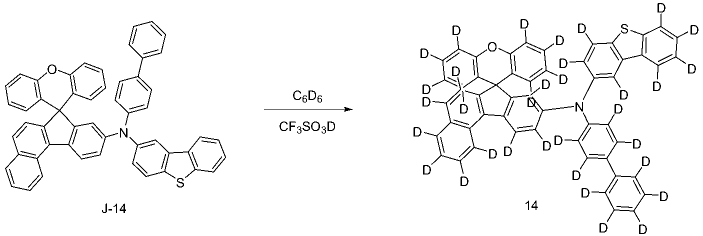

Compound J-14 (7.31 g, 10 mmol) synthesized in Preparation Example 14 was dissolved in perdeuterated benzene (C₆D₆) (84.15 g, 1 mol), CF₃SO₃D (7.55 g, 50 mmol) was added thereto, and the mixture was stirred at 80°C. A sample was taken to measure level of deuteration using LC-MS, and after completion of the exchange reaction, the temperature was lowered to room temperature. It was confirmed from measurements with LC-MS that deuterium substitution may be performed at various rates depending on conditions. The reaction was terminated by adding Na₂CO₃ in D₂O, and an organic solvent was concentrated. Recrystallization was performed using toluene and acetone solvents to obtain a deuterated target compound 14 (3.51 g, Yield 46 %).
Mass : [(M+H)⁺] : 765

### [Synthesis Example 15] Synthesis of Compound 15

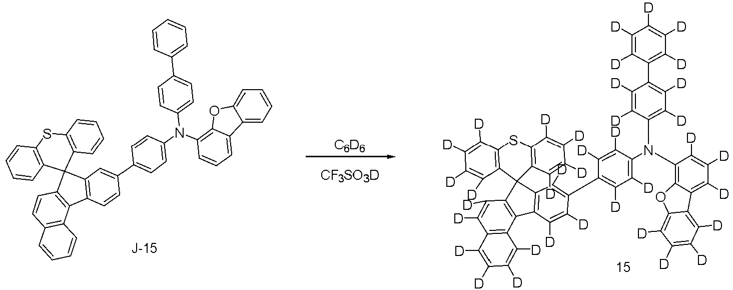

Compound J-15 (8.08 g, 10 mmol) synthesized in Preparation Example 15 was dissolved in perdeuterated benzene (C₆D₆) (84.15 g, 1 mol), CF₃SO₃D (7.55 g, 50 mmol) was added thereto, and the mixture was stirred at 80°C. A sample was taken to measure level of deuteration using LC-MS, and after completion of the exchange reaction, the temperature was lowered to room temperature. It was confirmed from measurements with LC-MS that deuterium substitution may be performed at various rates depending on conditions. The reaction was terminated by adding Na₂CO₃ in D₂O, and an organic solvent was concentrated. Recrystallization was performed using toluene and acetone solvents to obtain a deuterated target compound 15 (3.80 g, Yield 45 %).
Mass : [(M+H)⁺] : 845

### [Embodiment 15] Fabrication of blue organic EL device

Compound 1 synthesized in Synthesis Example 1 was subjected to high-purity sublimation purification in a conventionally known method, and then a blue organic EL device was fabricated as follows.

First, a glass substrate coated with a 1200 Å-thick indium tin oxide (ITO) thin film was ultrasonically cleaned with distilled water. After cleaning with distilled water was completed, the glass substrate was ultrasonically cleaned using a solvent, such as isopropyl alcohol, acetone and methanol, dried, placed in a UV OZONE cleaner (Power sonic 405, Hwasin Tech) to be cleaned with UV light for 5 minutes, and then transferred to a vacuum evaporator.

On the ITO transparent electrode prepared as described above, the following layers were sequentially deposited in the order listed: HI + 2% HAT-CN6 (10 nm) /Compound 1 (140 nm)/EB (5 nm)/BH + 2 % BD (20nm) /HB (5 nm) /ET + Liq (1:1) (30 nm)/LiF (1 nm)/Al (100 nm), such that an organic EL device was prepared. Structures of HI, HAT-CN6, EB, BH, BD, HB, ET, Liq used in such a case were as follows.

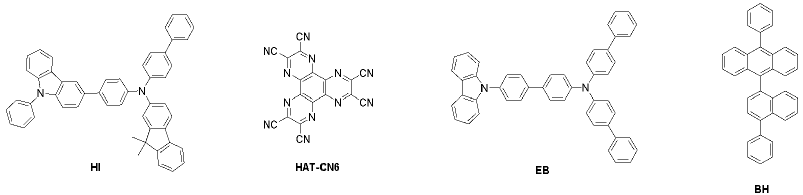

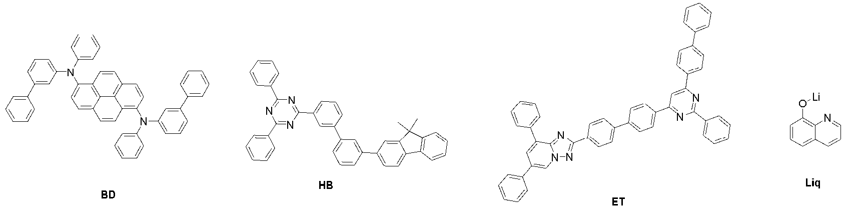

### [Embodiments 2 to 15] Fabrication of blue organic EL devices

Blue organic EL devices were fabricated in the same manner as in Embodiment 1, except that hole transport layer materials shown in Table 1 were respectively used instead of Compound 1 which was used as a hole transport layer material in Embodiment 1.

### [Comparative Examples 1 to 3] Fabrication of Blue Organic EL devices

Blue organic EL devices were fabricated in the same manner as in Embodiment 1, except that Compounds HI, HT1, and HT2 were respectively used instead of Compound 1 which was used as a hole transport layer material in Embodiment 1. The structures of HI, HT1, and HT2 used in such a case were as follows.

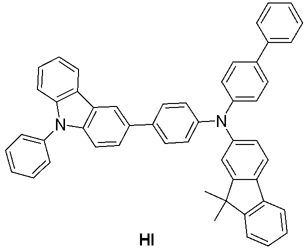

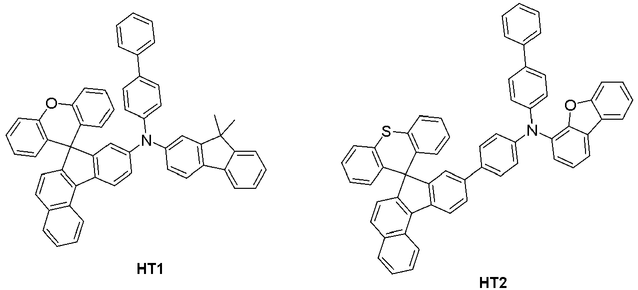

### [Evaluation Example 1]

For each of the blue organic EL devices fabricated in Embodiments 1 to 15 and Comparative Examples 1 to 3, a driving voltage, an emission wavelength, and current efficiency at a current density of 10 mA/cm² were measured and the results are shown in Table 1 below.

**[Table 1]**

| Sample | Hole transport layer material | Driving voltage (V) | Emission peak (nm) | Current efficiency (cd/A) |
|---|---|---|---|---|
| Embodiment 1 | 1 | 4.2 | 457 | 7.7 |
| Embodiment 2 | 2 | 4.4 | 455 | 6.5 |
| Embodiment 3 | 3 | 4.7 | 456 | 6.0 |
| Embodiment 4 | 4 | 4.9 | 456 | 6.1 |
| Embodiment 5 | 5 | 4.1 | 453 | 6.2 |
| Embodiment 6 | 6 | 4.3 | 456 | 6.3 |
| Embodiment 7 | 7 | 4.9 | 455 | 7.0 |
| Embodiment 8 | 8 | 4.0 | 454 | 7.2 |
| Embodiment 9 | 9 | 5.0 | 459 | 7.4 |
| Embodiment 10 | 10 | 5.1 | 455 | 7.1 |
| Embodiment 11 | 11 | 5.0 | 452 | 6.8 |
| Embodiment 12 | 12 | 4.4 | 455 | 6.4 |
| Embodiment 13 | 13 | 4.2 | 454 | 7.2 |
| Embodiment 14 | 14 | 4.5 | 455 | 6.9 |
| Embodiment 15 | 15 | 4.7 | 456 | 7.7 |
| Comp. Ex. 1 | HI | 5.4 | 452 | 5.9 |
| Comp. Ex. 2 | HT1 | 5.2 | 456 | 5.1 |
| Comp. Ex. 3 | HT2 | 5.3 | 454 | 5.2 |

As shown in Table 1, it was appreciated that the organic EL devices of Embodiments 1 to 15, in which the compounds of the present disclosure were used as the hole transport layer, exhibit superior performance in terms of driving voltage, emission peak, and current efficiency, compared to the organic EL devices of Comparative Examples 1 to 3, in which a conventional hole transport material (e.g., HI) or compounds (e.g., HT1, HT2) not containing deuterium (D) were used as the hole transport layer.

## Claims

1. A compound represented by the following Chemical Formula 1: 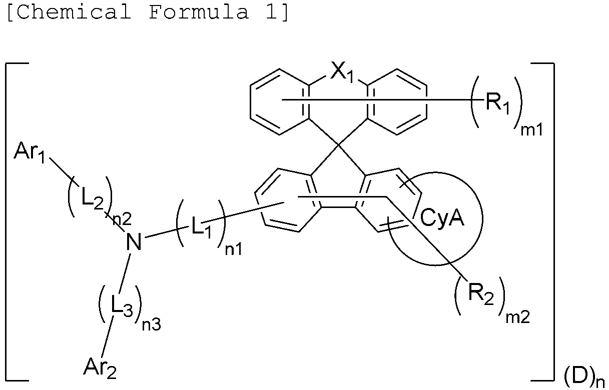 (wherein in Chemical Formula 1,
n≥1,
a CyA ring is a C₆ to C₃₀ aromatic ring,
X₁ is 0 or S,
each of n1 to n3 is an integer in a range of 0 to 3,
L₁ to L₃ are the same as or different from each other, and each independently is a single bond, or is selected from the group consisting of a C₆ to C₃₀ arylene group and a heteroarylene group having 5 to 30 nuclear atoms,
m1 is an integer in a range of 0 to 8,
m2 is an integer in a range of 0 to 23,
R₁, R₂, Ar₁ and Ar₂ are the same as or different from each other, and each independently is selected from the group consisting of: hydrogen, deuterium (D), halogen group, cyano group, nitro group, amino group, C₁ to C₄₀ alkyl group, C₂ to C₄₀ alkenyl group, C₂ to C₄₀ alkynyl group, C₃ to C₄₀ cycloalkyl group, heterocycloalkyl group having 3 to 40 nuclear atoms, C₆ to C₆₀ aryl group, heteroaryl group having 5 to 60 nuclear atoms, C₁ to C₄₀ alkyloxy group, C₆ to C₆₀ aryloxy group, C₁ to C₄₀ alkylsilyl group, C₆ to C₆₀ arylsilyl group, C₁ to C₄₀ alkylboron group, C₆ to C₆₀ arylboron group, C₆ to C₆₀ arylphosphine group, C₆ to C₆₀ arylphosphine oxide group, C₆ to C₆₀ arylamine group, C₆ to C₆₀ (aryl) (heteroaryl)amine group, and heteroarylamine group having 5 to 60 nuclear atoms, or R₁, R₂, Ar₁ and Ar₂ each independently is fused with an adjacent group to form a fused ring, and
the arylene group and the heteroarylene group of L₁ to L₃, and the alkyl group, the alkenyl group, the alkynyl group, the cycloalkyl group, the heterocycloalkyl group, the aryl group, the heteroaryl group, the alkyloxy group, the aryloxy group, the alkylsilyl group, the arylsilyl group, the alkylboron group, the arylboron group, the arylphosphine group, the arylphosphine oxide group, the arylamine group, the (aryl) (heteroaryl)amine group and the heteroarylamine group of R₁, R₂, Ar₁ and Ar₂ each independently is substituted or unsubstituted with one or more substituents selected from the group consisting of deuterium (D), halogen, cyano group, nitro group, amino group, C₁ to C₄₀ alkyl group, C₂ to C₄₀ alkenyl group, C₂ to C₄₀ alkynyl group, C₃ to C₄₀ cycloalkyl group, heterocycloalkyl group having 3 to 40 nuclear atoms, C₆ to C₆₀ aryl group, heteroaryl group having 5 to 60 nuclear atoms, C₁ to C₄₀ alkyloxy group, C₆ to C₆₀ aryloxy group, C₁ to C₄₀ alkylsilyl group, C₆ to C₆₀ arylsilyl group, C₁ to C₄₀ alkylboron group, C₆ to C₆₀ arylboron group, C₆ to C₆₀ arylphosphine group, C₆ to C₆₀ arylphosphine oxide group, C₆ to C₆₀ arylamine group, C₆ to C₆₀ (aryl) (heteroaryl)amine group, and heteroarylamine group having 5 to 60 nuclear atoms, and when the substituents are plural, the substituents are the same as or different from each other).

2. The compound of claim 1,
wherein the 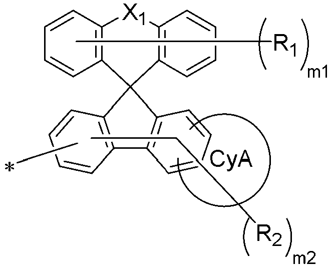
moiety in the compound represented by Chemical Formula 1 is any one of the following moieties Mol-1 to Mol-3: 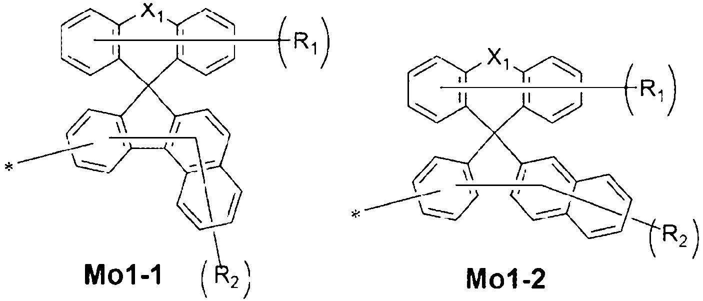 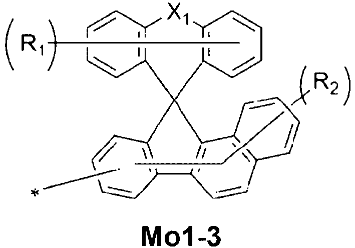
(wherein in the above moieties Mol-1 to Mol-3,
X₁, m1, R₁ and R₂ are each as defined in Chemical Formula 1, and
m3 is an integer in a range of 0 to 9.

3. The compound of claim 1,
wherein the compound represented by Chemical Formula 1 is represented by any one of the following Chemical Formulas 2 to 22, 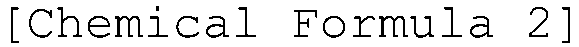 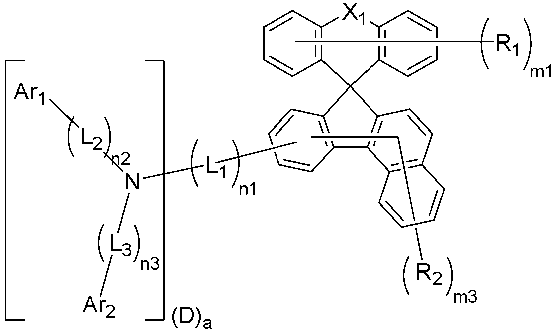 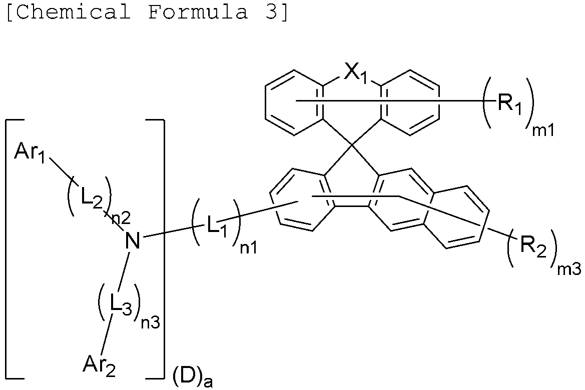 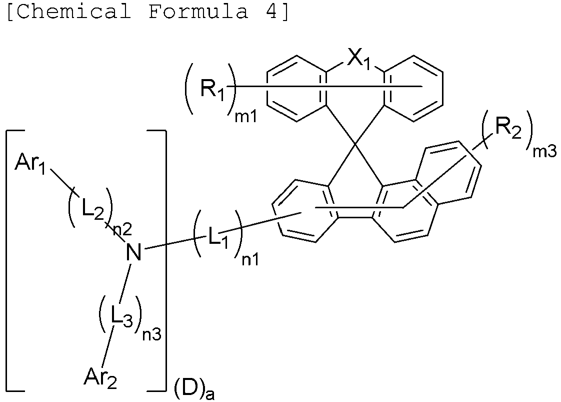 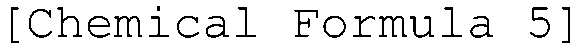 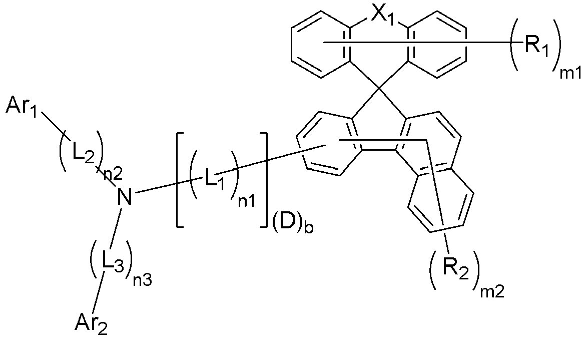 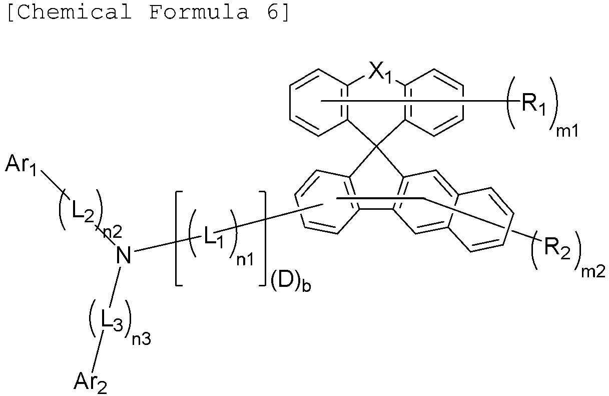 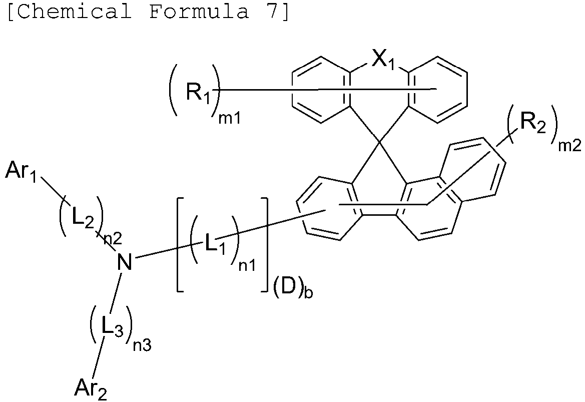 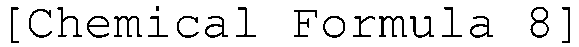 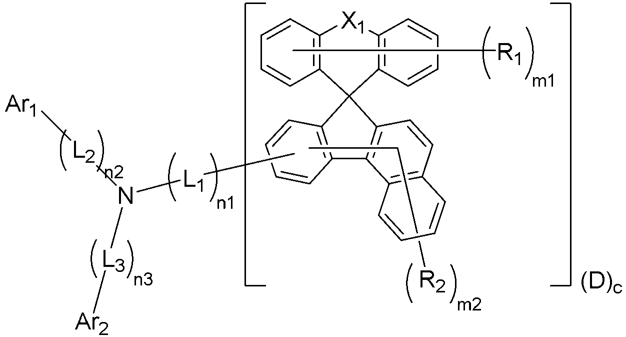 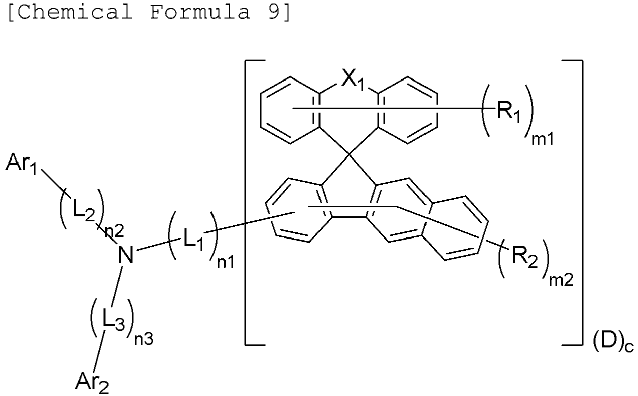 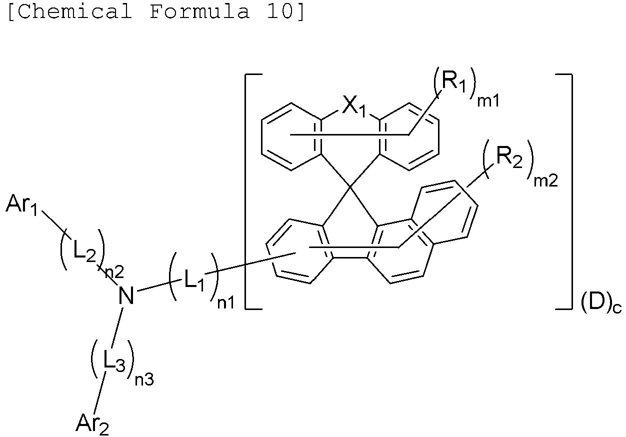 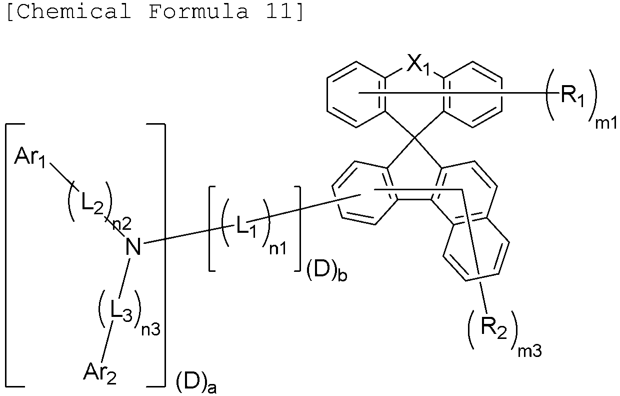 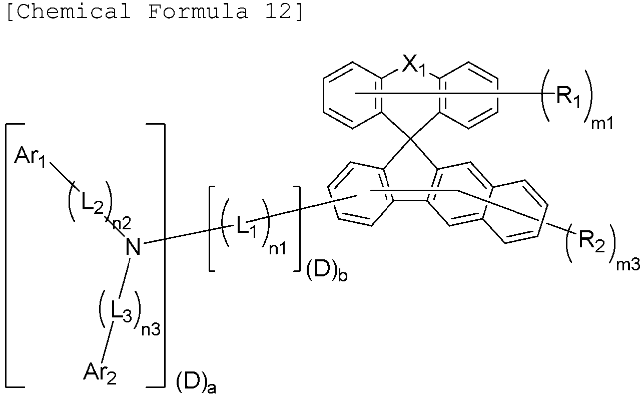 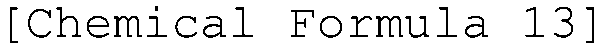 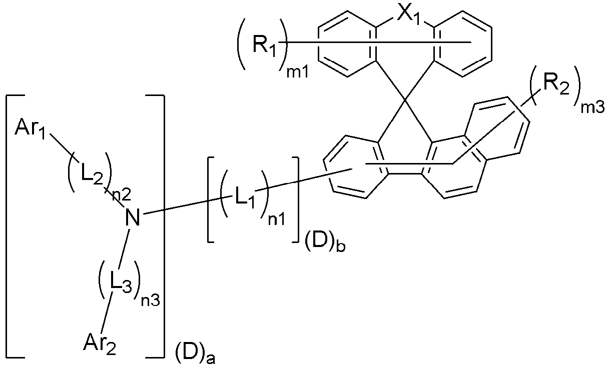 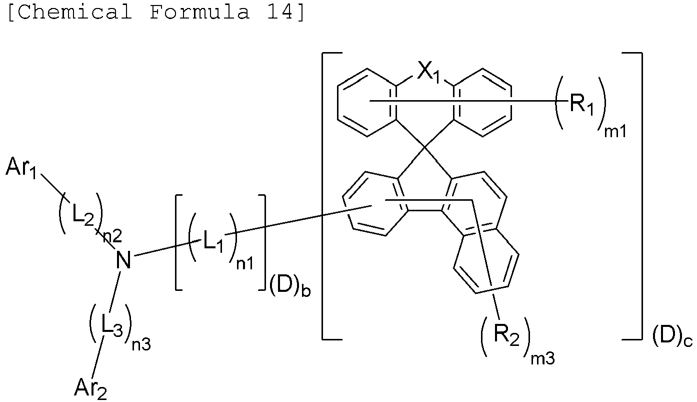 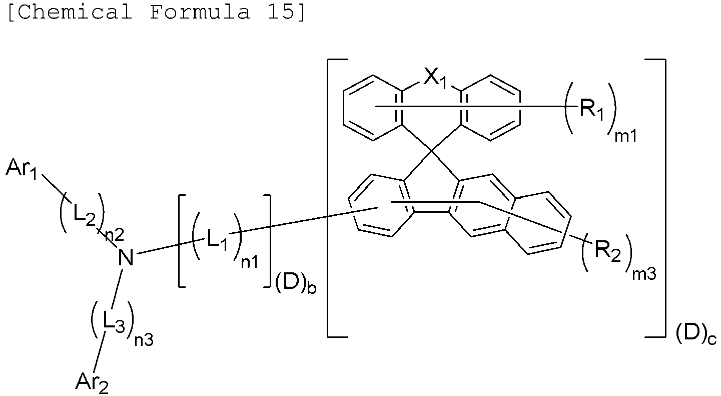 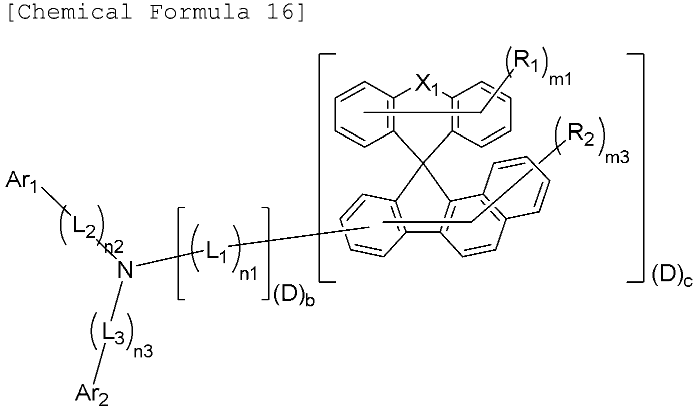 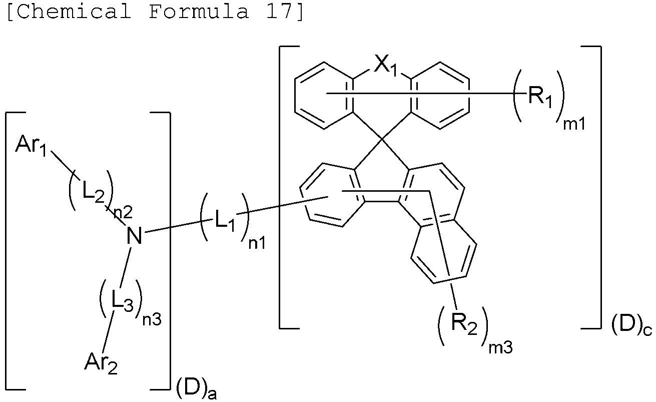 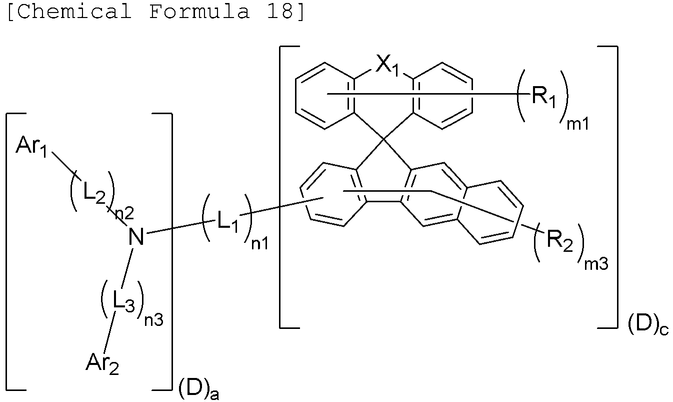 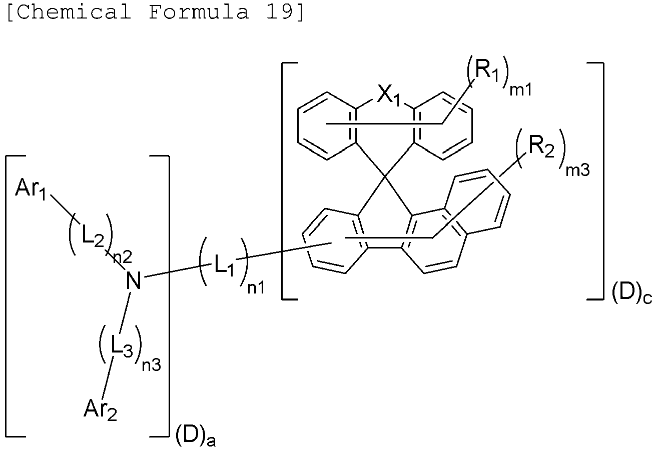 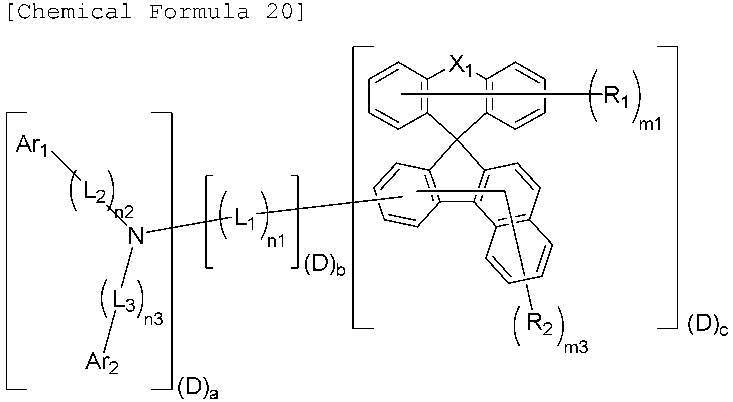 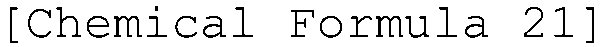 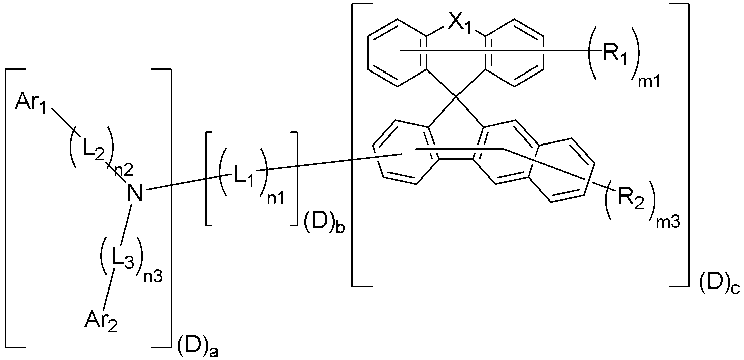 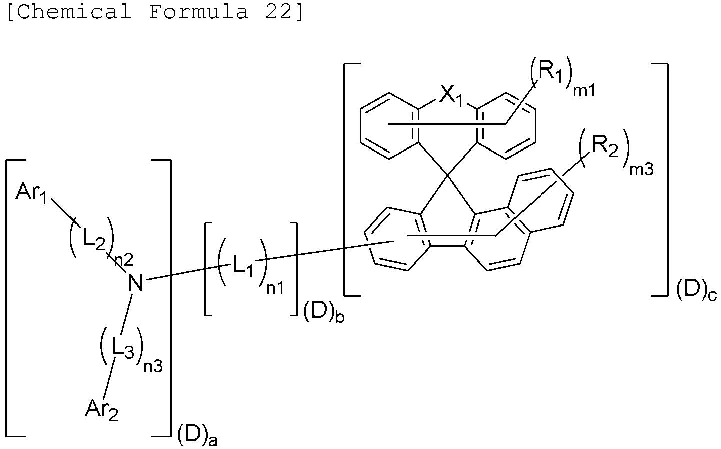
(wherein in Chemical Formulas 2 to 22,
X₁, m1, R₁, R₂, n1 to n3, L₁ to L₃, Ar₁ and Ar₂ are each as defined in claim 1,
m3 is an integer in a range of 0 to 9,
a is an integer in a range of 0 to 58,
b is an integer in a range of 0 to 12,
c is an integer in a range of 0 to 17, and
a+b+c≥1.

4. The compound of claim 1,
wherein L₁ to L₃ are the same as or different from each other, and each independently is the following linker group L1-1: 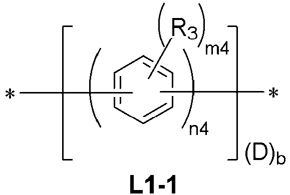
(wherein in the linker group L1-1,
(D)_{b} is the number of deuterium included in the linker group L1-1, 0≤b≤12,
n4 is an integer in a range of 0 to 3,
m4 is an integer in a range of 0 to 4, and
R₃ is selected from the group consisting of: hydrogen, deuterium (D), halogen group, cyano group, nitro group, amino group, C₁ to C₄₀ alkyl group, C₂ to C₄₀ alkenyl group, C₂ to C₄₀ alkynyl group, C₃ to C₄₀ cycloalkyl group, heterocycloalkyl group having 3 to 40 nuclear atoms, C₆ to C₆₀ aryl group, heteroaryl group having 5 to 60 nuclear atoms, C₁ to C₄₀ alkyloxy group, C₆ to C₆₀ aryloxy group, C₁ to C₄₀ alkylsilyl group, C₆ to C₆₀ arylsilyl group, C₁ to C₄₀ alkylboron group, C₆ to C₆₀ arylboron group, C₆ to C₆₀ arylphosphine group, C₆ to C₆₀ arylphosphine oxide group, C₆ to C₆₀ arylamine group, C₆ to C₆₀ (aryl) (heteroaryl)amine group, and heteroarylamine group having 5 to 60 nuclear atoms, or R₃ is fused with an adjacent group to form a fused ring).

5. The compound of claim 1,
wherein Ar₁ and Ar₂ are the same as or different from each other and each independently is selected from the group consisting of the following substituents S1-1 to S1-3: 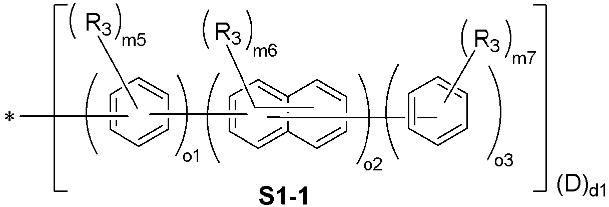 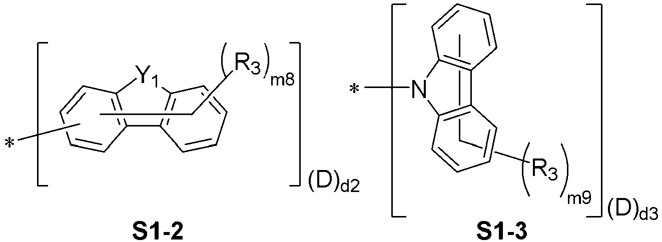
(wherein in the substituents S1-1 to S1-3,
(D)_{d1} is the number of deuterium (D) included in the substituent S1-1, 0≤d1≤15,
(D)_{d2} is the number of deuterium (D) included in the substituent S1-2, 0≤d2≤7,
(D)_{d3} is the number of deuterium (D) included in the substituent S1-3, 0≤d3≤8,
each of o1 to o3 is 0 or 1, wherein o1+o2+o3≥1,
m5 is an integer in a range of 0 to 4,
m6 is an integer in a range of 0 to 6,
m7 is an integer in a range of 0 to 5,
m8 is an integer in a range of 0 to 7,
m9 is an integer in a range of 0 to 8,
Y₁ is selected from the group consisting of: O, S, C(Ar₃)(Ar₄), and N(Ar₅),
R₃ and Ar₃ to Ar₅ are the same as or different from each other, and each independently is selected from the group consisting of: hydrogen, deuterium (D), halogen group, cyano group, nitro group, amino group, C₁ to C₄₀ alkyl group, C₂ to C₄₀ alkenyl group, C₂ to C₄₀ alkynyl group, C₃ to C₄₀ cycloalkyl group, heterocycloalkyl group having 3 to 40 nuclear atoms, C₆ to C₆₀ aryl group, heteroaryl group having 5 to 60 nuclear atoms, C₁ to C₄₀ alkyloxy group, C₆ to C₆₀ aryloxy group, C₁ to C₄₀ alkylsilyl group, C₆ to C₆₀ arylsilyl group, C₁ to C₄₀ alkylboron group, C₆ to C₆₀ arylboron group, C₆ to C₆₀ arylphosphine group, C₆ to C₆₀ arylphosphine oxide group, C₆ to C₆₀ arylamine group, C₆ to C₆₀ (aryl) (heteroaryl)amine group, and heteroarylamine group having 5 to 60 nuclear atoms, or R₃ and Ar₃ to Ar₅ each independently is fused with an adjacent group thereof to form a fused ring, and
the alkyl group, the alkenyl group, the alkynyl group, the cycloalkyl group, the heterocycloalkyl group, the aryl group, the heteroaryl group, the alkyloxy group, the aryloxy group, the alkylsilyl group, the arylsilyl group, the alkylboron group, the arylboron group, the arylphosphine group, the arylphosphine oxide group, the arylamine group, the (aryl) (heteroaryl)amine group and the heteroarylamine group of Ar₃ to Ar₅ each independently is substituted or unsubstituted with one or more substituents selected from the group consisting of: deuterium (D), halogen, cyano group, nitro group, amino group, C₁ to C₄₀ alkyl group, C₂ to C₄₀ alkenyl group, C₂ to C₄₀ alkynyl group, C₃ to C₄₀ cycloalkyl group, heterocycloalkyl group having 3 to 40 nuclear atoms, C₆ to C₆₀ aryl group, heteroaryl group having 5 to 60 nuclear atoms, C₁ to C₄₀ alkyloxy group, C₆ to C₆₀ aryloxy group, C₁ to C₄₀ alkylsilyl group, C₆ to C₆₀ arylsilyl group, C₁ to C₄₀ alkylboron group, C₆ to C₆₀ arylboron group, C₆ to C₆₀ arylphosphine group, C₆ to C₆₀ arylphosphine oxide group, C₆ to C₆₀ arylamine group, C₆ to C₆₀ (aryl) (heteroaryl)amine group, and heteroarylamine group having 5 to 60 nuclear atoms, and when the substituents are plural, the substituents are the same as or different from each other.

6. The compound of claim 1,
wherein the compound represented by Chemical Formula 1 is represented by any one of the following Chemical Formulas 23 to 31: 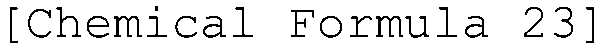 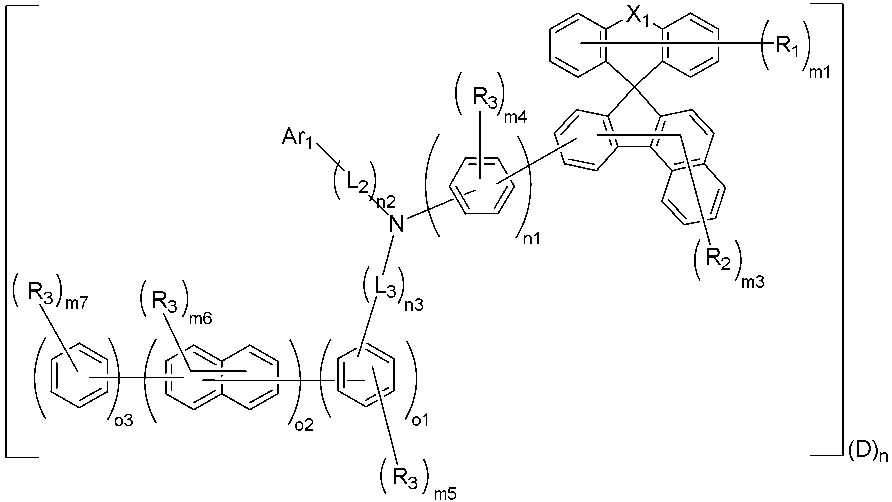 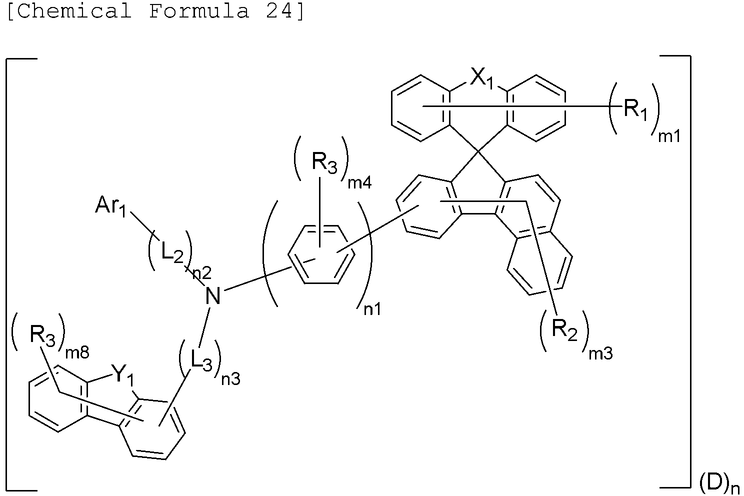 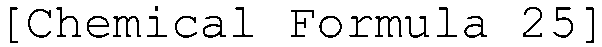 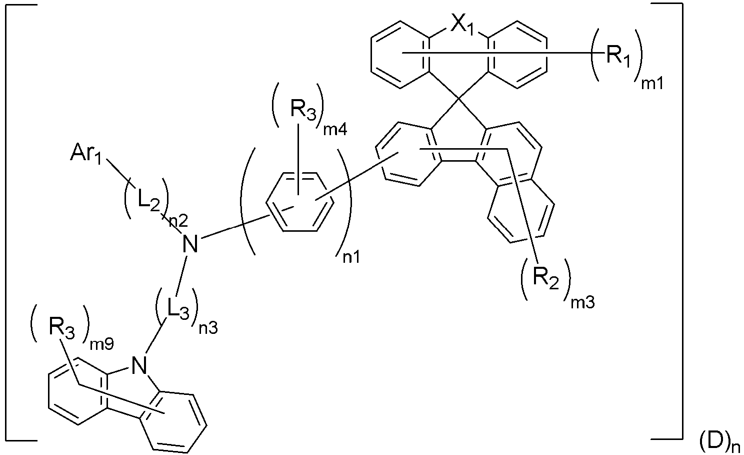 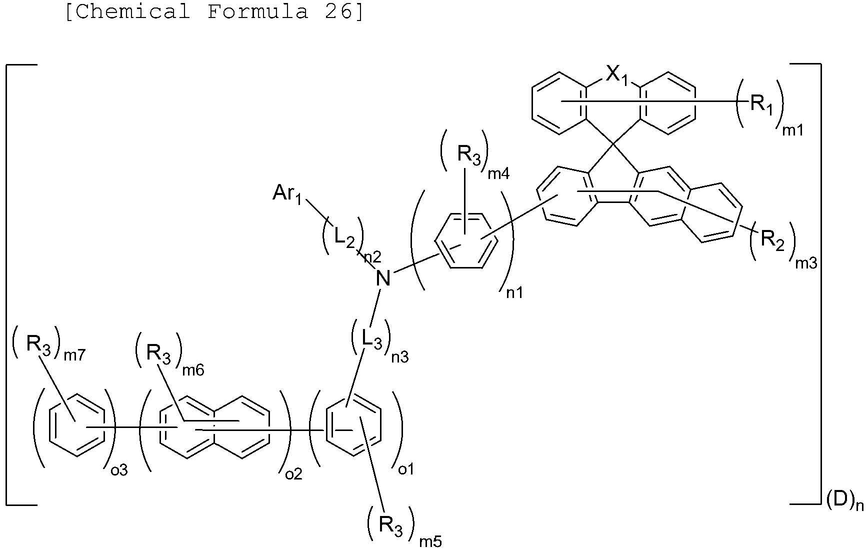 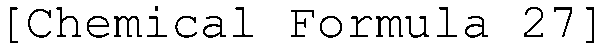 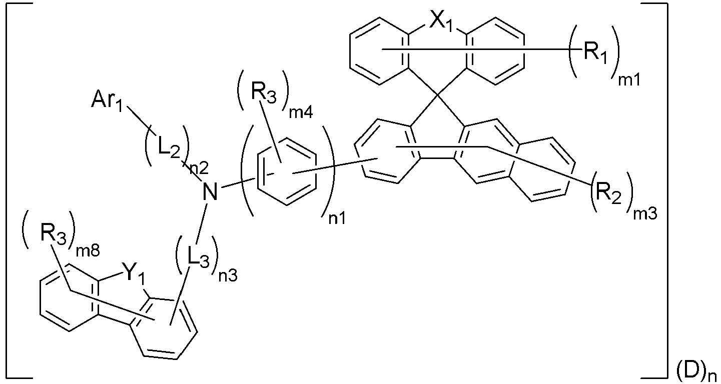 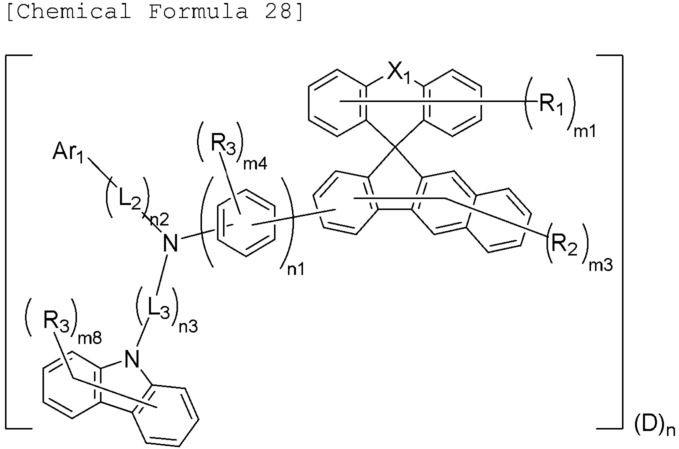 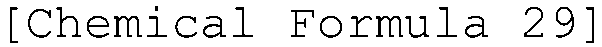 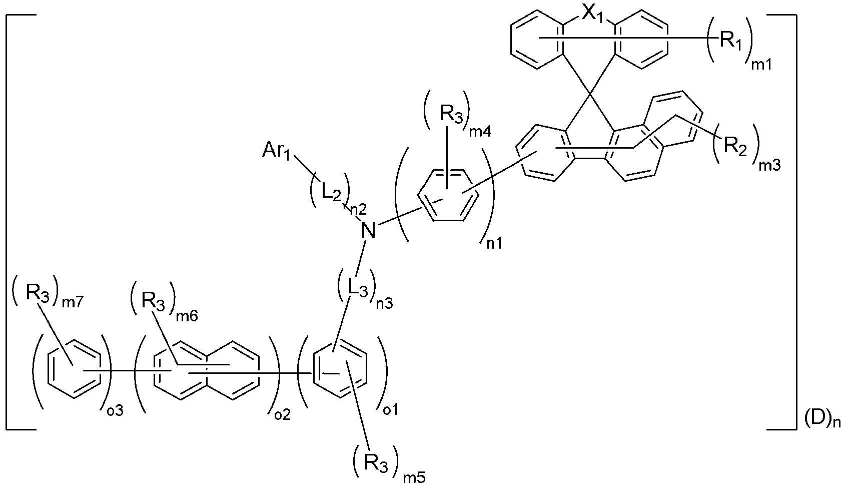 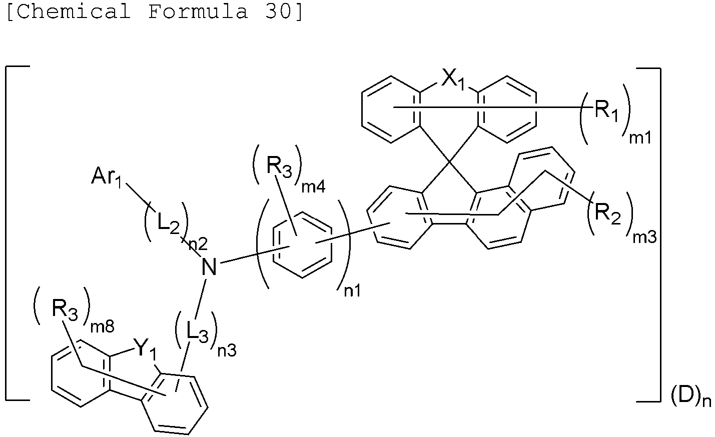 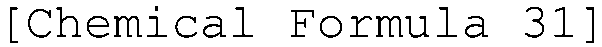 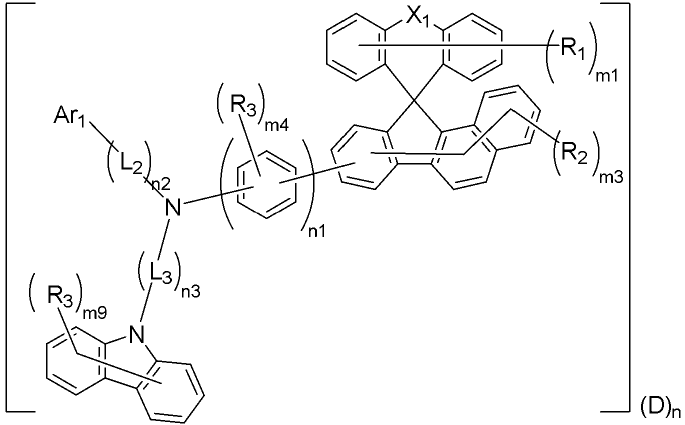
(in Chemical Formulas 23 to 31,
X₁, n1 to n3, L₂, L₃, m1, R₁, R₂, and Ar₁ are each as defined in claim 1,
(D)n is the number of deuterium (D) included in the compound, 1≤n≤87,
m3 is an integer in a range of 0 to 9,
m4 is an integer in a range of 0 to 4,
each of o1 to o3 is 0 or 1, wherein o1+o2+o3≥1,
m5 is an integer in a range of 0 to 4,
m6 is an integer in a range of 0 to 6,
m7 is an integer in a range of 0 to 5,
m8 is an integer in a range of 0 to 7,
m9 is an integer in a range of 0 to 8,
Y₁ is selected from the group consisting of O, S, C(Ar₃)(Ar₄), and N(Ar₅),
R₃ and Ar₃ to Ar₅ are the same as or different from each other, and each independently is selected from the group consisting of: hydrogen, deuterium (D), halogen group, cyano group, nitro group, amino group, C₁ to C₄₀ alkyl group, C₂ to C₄₀ alkenyl group, C₂ to C₄₀ alkynyl group, C₃ to C₄₀ cycloalkyl group, heterocycloalkyl group having 3 to 40 nuclear atoms, C₆ to C₆₀ aryl group, heteroaryl group having 5 to 60 nuclear atoms, C₁ to C₄₀ alkyloxy group, C₆ to C₆₀ aryloxy group, C₁ to C₄₀ alkylsilyl group, C₆ to C₆₀ arylsilyl group, C₁ to C₄₀ alkylboron group, C₆ to C₆₀ arylboron group, C₆ to C₆₀ arylphosphine group, C₆ to C₆₀ arylphosphine oxide group, C₆ to C₆₀ arylamine group, C₆ to C₆₀ (aryl) (heteroaryl)amine group, and heteroarylamine group having 5 to 60 nuclear atoms, or R₃ and Ar₃ to Ar₅ each independently is fused with an adjacent group to form a fused ring, and
the alkyl group, the alkenyl group, the alkynyl group, the cycloalkyl group, the heterocycloalkyl group, the aryl group, the heteroaryl group, the alkyloxy group, the aryloxy group, the alkylsilyl group, the arylsilyl group, the alkylboron group, the arylboron group, the arylphosphine group, the arylphosphine oxide group, the arylamine group, the (aryl) (heteroaryl)amine group and the heteroarylamine group of Ar₃ to Ar₅ each independently is substituted or unsubstituted with one or more substituents selected from the group consisting of deuterium (D), halogen, cyano group, nitro group, amino group, C₁ to C₄₀ alkyl group, C₂ to C₄₀ alkenyl group, C₂ to C₄₀ alkynyl group, C₃ to C₄₀ cycloalkyl group, heterocycloalkyl group having 3 to 40 nuclear atoms, C₆ to C₆₀ aryl group, heteroaryl group having 5 to 60 nuclear atoms, C₁ to C₄₀ alkyloxy group, C₆ to C₆₀ aryloxy group, C₁ to C₄₀ alkylsilyl group, C₆ to C₆₀ arylsilyl group, C₁ to C₄₀ alkylboron group, C₆ to C₆₀ arylboron group, C₆ to C₆₀ arylphosphine group, C₆ to C₆₀ arylphosphine oxide group, C₆ to C₆₀ arylamine group, C₆ to C₆₀ (aryl) (heteroaryl)amine group, and heteroarylamine group having 5 to 60 nuclear atoms, and when the substituents are plural, the substituents are the same as or different from each other).

7. The compound of claim 1,
wherein the compound represented by Chemical Formula 1 is any one of the following compounds 1 to 120:

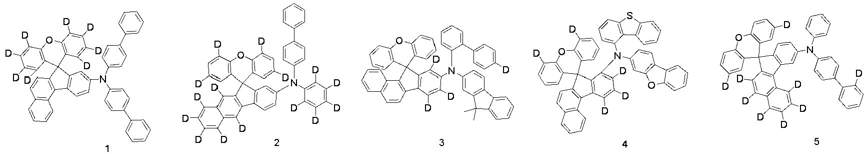

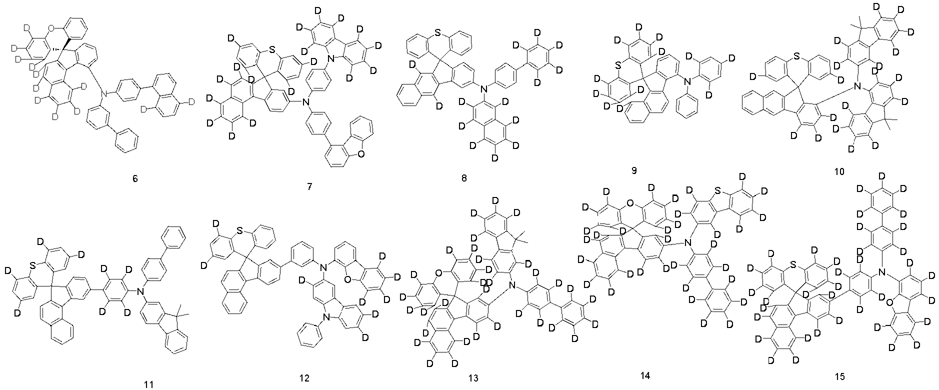

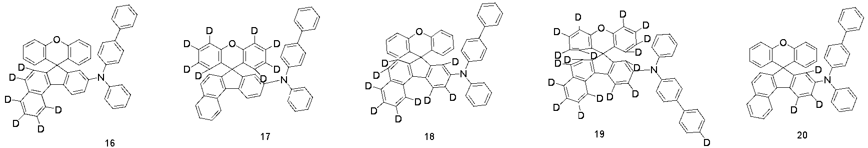

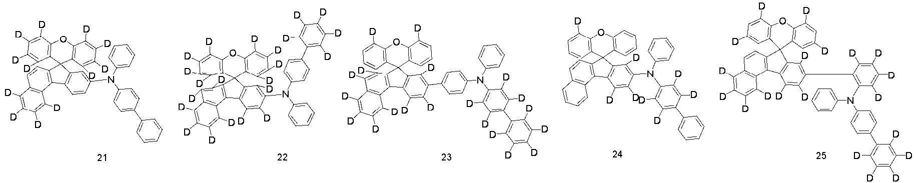

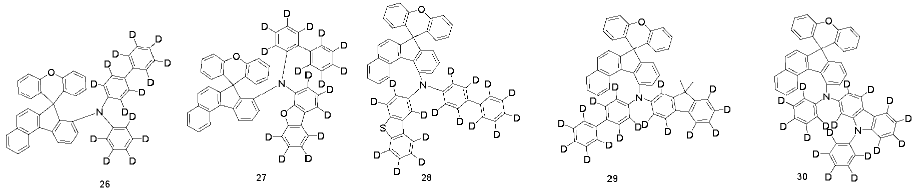

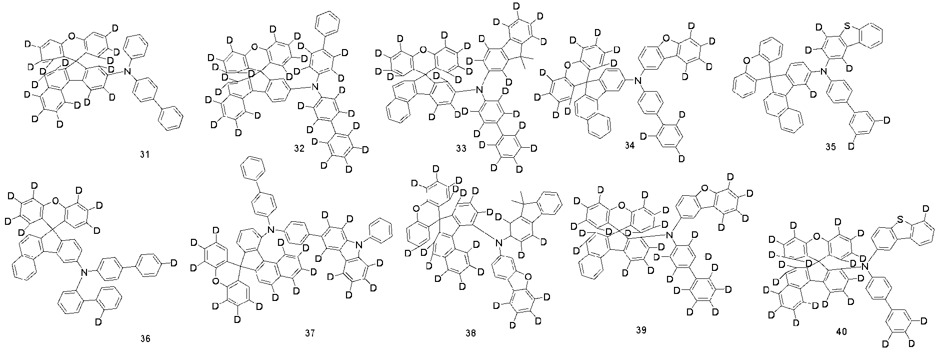

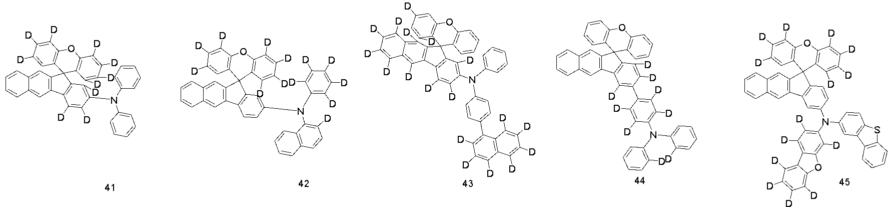

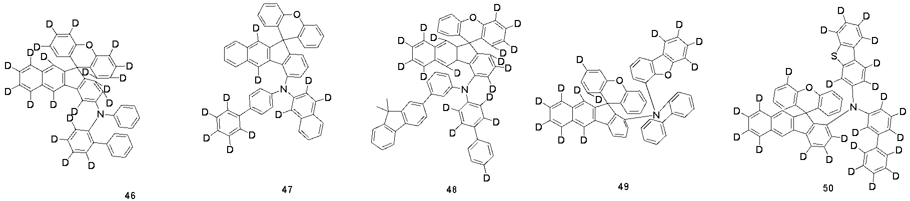

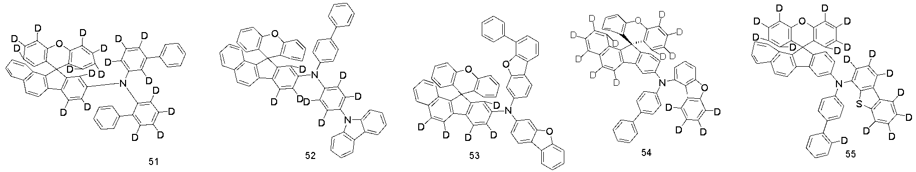

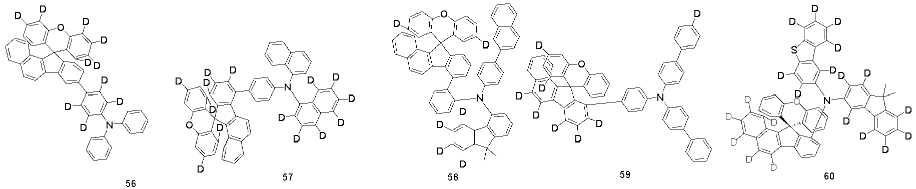

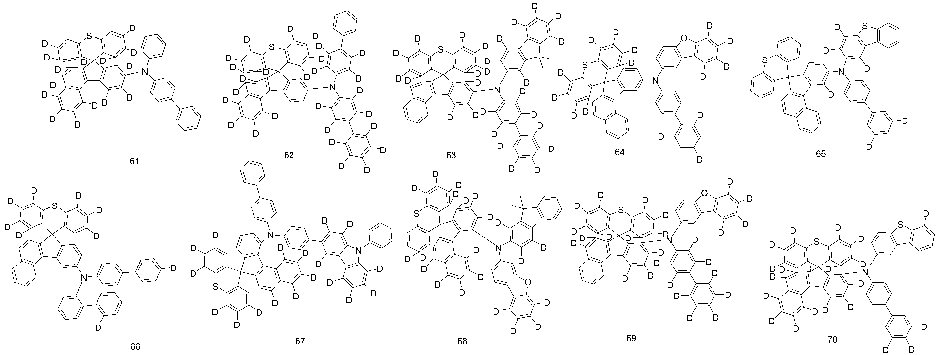

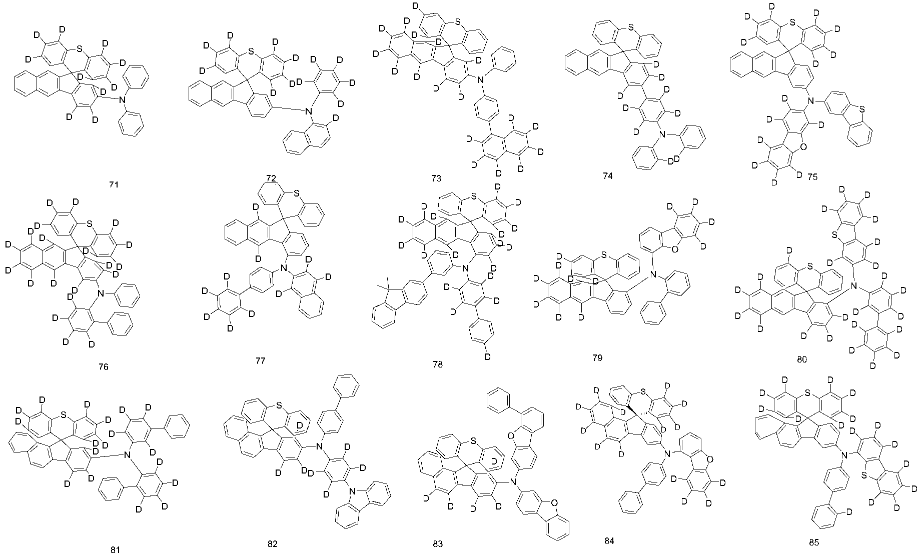

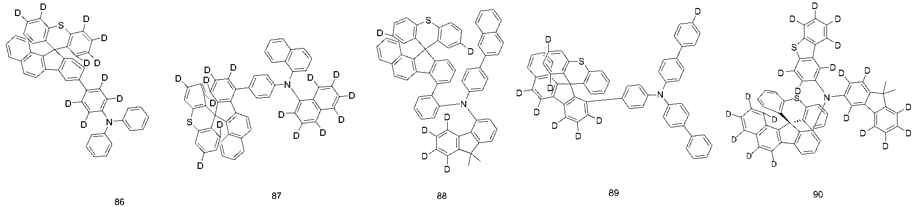

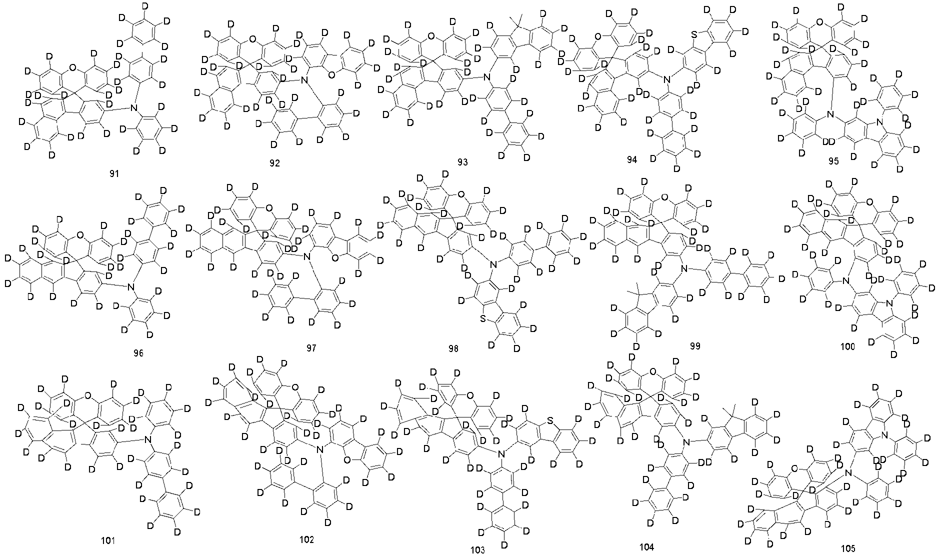

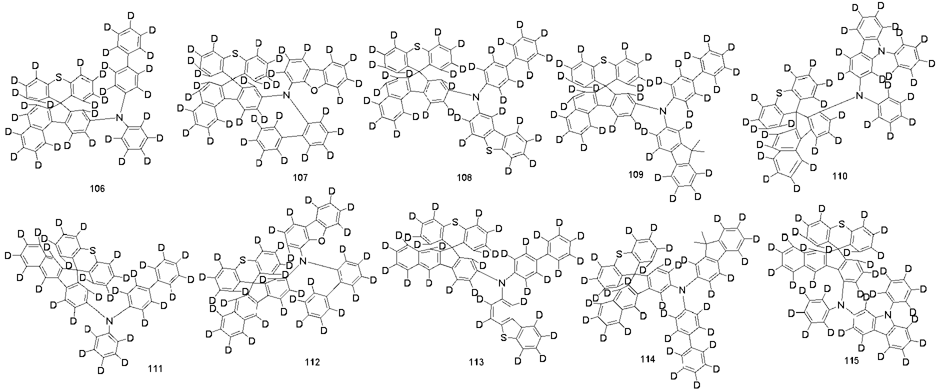

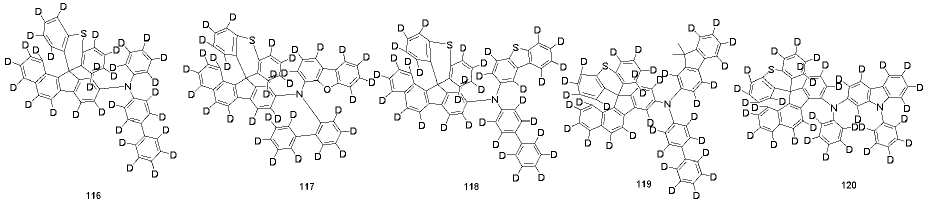

8. An organic electroluminescent device comprising:
an anode, a cathode, and one or more organic layers disposed between the anode and the cathode,
wherein at least one of the one or more organic layers comprises the compound according to any one of claims 1 to 7.

9. The organic electroluminescent device of claim 8,
wherein the organic layer comprising the compound is a hole transport layer.
